# EUROPEAN PATENT APPLICATION

(11) **EP 3 800 247 A1**
(43) Date of publication of application: **07.04.2021**
(21) Application number: 19201003.1
(22) Date of filing: 02.10.2019
(51) Int. Cl.: C12N 9/02, C12N 9/10, C12N 9/12, C12N 9/88, C12N 9/00, C12P 7/22

(54) **BIOTECHNOLOGICAL PRODUCTION OF ALKYLPHENOLS AND THEIR USES**

(71) Applicant: Johann Wolfgang Goethe-Universität Frankfurt, 60323 Frankfurt am Main (DE)
(72) Inventor: Boles, Eckhard, 64289 Darmstadt (DE); Grininger, Martin, 60316 Frankfurt (DE); Hitschler, Julia, 60326 Frankfurt am Main (DE)
(74) Representative: Grahn, Sibylla Maria

(57) **Abstract**

The present invention relates to host cells which contain heterologous nucleic acid molecules coding for a 6-methylsalicylic acid synthase (MSAS), a phosphopantetheinyl transferase (PPTase) for the phosphopantetheinylation of the MSAS, a 6-methylsalicylic acid (6-MSA) decarboxylase, and an increased intracellular provision of propionyl-CoA and/or butyryl-CoA. The present invention further relates to using these host cells, preferably yeast cells, for producing 3-alkylphenols. The present invention further relates to methods of producing 3-alkylphenols, compositions of 3-alkylphenols and to tsetse fly attractant compositions.

## Description

The present invention relates to host cells which contain heterologous nucleic acid molecules coding for a 6-methylsalicylic acid synthase (MSAS), a phosphopantetheinyl transferase (PPTase) for the phosphopantetheinylation of the MSAS, a 6-methylsalicylic acid (6-MSA) decarboxylase, and an increased intracellular provision of propionyl-CoA and/or butyryl-CoA. The present invention further relates to using these host cells, preferably yeast cells, for producing 3-alkylphenols. The present invention further relates to methods of producing 3-alkylphenols, compositions of 3-alkylphenols and to tsetse fly attractant compositions.

### BACKGROUND OF THE INVENTION

Tsetse flies are large biting flies that inhabit much of tropical Africa. Tsetse flies include all the species in the genus *Glossina,* which are placed in their own family, Glossinidae. Tsetse flies are the vector of trypanosomes and are regarded as a major cause of rural poverty in sub-Saharan Africa because they prevent mixed farming. The land infested with tsetse flies is often cultivated by people using hoes rather than more efficient draught animals because nagana, the disease transmitted by tsetse flies, weakens and often kills these animals. The disease nagana or African animal trypanosomiasis (AAT) causes gradual health decline in infected livestock, reduces milk and meat production, increases abortion rates and animals eventually succumb to the disease (annual cattle deaths caused by trypanosomiasis are estimated at 3 million). This has an enormous impact on the livelihood of farmers who live in tsetse-infested areas, as infected animals cannot be used to plough the land, and keeping cattle is only feasible when the animals are kept under constant prophylactic treatment with trypanocidal drugs, often with associated problems of drug resistance, counterfeited drugs, and suboptimal dosage.

Tsetse flies transmit a similar disease to humans, called human African trypanosomiasis (HAT) or sleeping sickness. An estimated 70 million people in 20 countries are at different levels of risk and only 3-4 million people are covered by active surveillance.

3-Alkylphenols (3-methyl, 3-ethyl- and 3-propylphenol) were identified as tsetse flies attractants (Bursell *et al.,* 1988). They are utilized as odour baits to attract tsetse flies to traps impregnated with insecticides to kill the vector of trypanosomes and prevent spreading of HAT and AAT. Currently, 3-alkylphenols are mainly extracted and purified from fossil resources or chemically synthesized for example from cashew nut shell liquids (Baader *et al.,* 2014). Chemical synthesis of these attractants rely on expensive catalysts, additional petrol-based chemicals or elaborate extraction procedures.

Thus, there is a need in the art for improved means and methods for producing 3-alkylphenols.

### SUMMARY OF THE INVENTION

According to the present invention this object is solved by a host cell containing
(i) a heterologous nucleic acid molecule coding for a 6-methylsalicylic acid synthase (MSAS),
(ii) a heterologous nucleic acid molecule coding for a phosphopantetheinyl transferase (PPTase) which is able to transfer a phosphopantethein group to MSAS,
(iii) a heterologous nucleic acid molecule coding for a 6-methylsalicylic acid (6-MSA) decarboxylase, and
(iii) source for increased intracellular synthesis of propionyl-CoA and/or butyryl-CoA, and, optionally,
(v) a block in the degradation pathway of propionyl-CoA and/or butyryl-CoA,
wherein the host cell is a fungus cell.

According to the present invention this object is solved by the use of the host cell according to the present invention for the production of 3-alkylphenols, preferably, 3-ethylphenol (3-EP) and/or 3-propylphenol (3-PP), optionally together with 3-methyl phenol (3-MP).

According to the present invention this object is solved by a method for the production of 3-alkylphenols, preferably of 3-ethylphenol (3-EP), optionally together with 3-methylphenol (3-MP),
comprising the steps of
providing a host cell according to the present invention,
culturing the host cell by adding carbon source(s), preferably sugar(s) to the culture medium,
or culturing the host cell by adding carbon source(s) and propionate to the culture medium,
optionally, obtaining the 3-alkylphenols.

According to the present invention this object is solved by a method for the production of 3-alkyl phenols, preferably 3-propylphenol (3-PP), optionally together with 3-methylphenol (3-MP),
comprising the steps of
providing a host cell according to the present invention,
culturing the host cell by adding carbon source(s), preferably sugar(s) to the culture medium,
or culturing the host cell by adding carbon source(s) and butyrate to the culture medium,
optionally, obtaining the 3-alkylphenols.

According to the present invention this object is solved by a method for the production of 3-alkyl phenols, preferably of 3-ethylphenol (3-EP) and 3-propylphenol (3-PP), optionally together with 3-methylphenol (3-MP)
comprising the steps of
providing a host cell according to the present invention,
culturing the host cell by adding carbon source(s), preferably sugar(s) to the culture medium,
or culturing the host cell by adding carbon source(s) and propionate and, optionally, butyrate to the culture medium
optionally, obtaining the 3-alkylphenols.

According to the present invention this object is solved by a composition comprising 3-alkyl phenols, preferably 3-ethylphenol (3-EP) and/or 3-propylphenol (3-PP), optionally in combination with 3-methylphenol (3-MP),
preferably obtained by a method of the present invention.

According to the present invention this object is solved by the use of at least one of the following:
(a) the host cell according to the present invention,
(b) the host cell extracts and/or the host cell culture and/or the host cell culture supernatant obtained in a method of the present invention,
(c) the composition of the present invention,
as tsetse fly attractant.

According to the present invention this object is solved by a tsetse fly attractant composition, comprising
(a) the host cell according to the present invention,
(b) the composition according to the present invention, and/or
(c) the host cell extracts and/or the host cell culture and/or the host cell culture supernatant obtained in a method of the present invention,
(d) optionally, excipient(s) and/or carrier.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "0.1 to 20" should be interpreted to include not only the explicitly recited values of 0.1 to 20, but also include individual values and sub-ranges within the indicated range. Thus, included in this numerical range are individual values such as 0.1, 0.2, 0.3, 0.4, 0.5, 0.6, 0.7, 0.8, 0.9, 1, 1.1 .... 19.6, 19.7, 19.8, 19.9, 20 and sub-ranges such as from 1 to 10, 0.5 to 5, etc. This same principle applies to ranges reciting only one numerical value, such as "at least 90%". Furthermore, such an interpretation should apply regardless of the breadth of the range or the characteristics being described.

### Host cells

As discussed above, the present invention provides host cells.

A host cell according to the present invention contains
(i) a heterologous nucleic acid molecule coding for a 6-methylsalicylic acid synthase (MSAS),
(ii) a heterologous nucleic acid molecule coding for a phosphopantetheinyl transferase (PPTase) which is able to transfer a phosphopantethein group to MSAS,
(iii) a heterologous nucleic acid molecule coding for a 6-methylsalicylic acid (6-MSA) decarboxylase, and
(iv) source for increased intracellular synthesis of propionyl-CoA and/or butyryl-CoA, and, optionally,
(v) a block in the degradation pathway of propionyl-CoA and/or butyryl-CoA

The host cell is a fungus cell. In a preferred embodiment the fungus cell is a yeast cell, such as *Saccharomyces* species, *Kluyveromyces* sp., *Ogataea* sp., *Pichia* sp. *Rhodotorula* sp. or *Yarrowia* sp., preferably *S. cerevisiae.*

In a preferred embodiment, the nucleic acid molecule coding for MSAS (i) is the *MSAS* gene of *Penicillium patulum.*

In a preferred embodiment, the nucleic acid molecule coding for PPTase (ii) is the *npgA* gene from *Aspergillus nidulans.*

In a preferred embodiment, the nucleic acid molecule coding for 6-MSA decarboxylase (iii) is the 6-MSA decarboxylase *gene patG* of *Aspergillus clavatus.*

In a preferred embodiment, a source for increased intracellular synthesis of propionyl-CoA (iv) is provided by at least one of the following:
(1) culturing the cell in a culture medium containing propionate and/or propionic acid.
   It is to be understood that depending on the pH of the medium propionate can be in its anionic form as propionate or in its acid form as propionic acid. The term "propionate" used in the present invention does not distinguish between both forms.
(2) a heterologous nucleic acid molecule coding for propionyl-CoA synthase, preferably the propionyl-CoA synthase *prpE* gene from *Salmonella typhimurium,* and, optionally, culturing the cell in a culture medium containing propionate and/or propionic acid.
(3) increased degradation of threonine, preferably in combination with increased synthesis of threonine. An intermediate in threonine catabolism is 2-oxobutyrate. Oxidative decarboxylation of 2-oxobutyrate yields propionyl-CoA (Luttik *et al.,* 2000). Conversion of threonine into propionyl-CoA is initiated by its deamination to 2-oxobutyrate, catalyzed by threonine dehydratase or threonine deaminase. The subsequent oxidative decarboxylation of 2-oxobutyrate to propionyl-CoA can be catalyzed by a 2-oxo-acid dehydrogenase complex. Increased synthesis and/or degradation of threonine to propionyl-CoA can be preferably achieved by overexpression, codon-optimization and/or elimination of feed-back inhibition of one or more of the corresponding endogenous or heterologous genes, respectively proteins.
(4) heterologous nucleic acid molecules coding for malonyl-CoA reductase (Mcr) from *Chloroflexus aurantiacus* reducing malonyl-CoA in two steps to 3-hydroxypropionate, the 3-hydroxypropionyl-CoA-synthase (3Hpcs) from *Metallospaera sedula* that esterifies 3-hydroxypropionate to 3-hydroxypropionyl-CoA, the hydroxylpropionyl-CoA dehydratase (3Hpcd) from *Sulfolobus tokadaii* and the acryloyl-CoA reductase (Acr) from *Metallosphaera sedula* that catalyze the subsequent dehydration to acryloyl-CoA and final reduction to propionyl-CoA (Krink-Koutsoubelis *et al.,* 2018).

In a preferred embodiment, a source of increased intracellular synthesis of butyryl-CoA (iv) is provided by at least one of the following:
(1) a heterologous nucleic acid molecule coding for butyryl-CoA synthase and culturing the cell in a culture medium containing butyrate and/or butyric acid.
   Optionally, a gene encoding a transport protein for improved uptake of butyrate and/or butyric acid might be additionally expressed or overexpressed in the host cell.
   It is to be understood that depending on the pH of the medium butyrate can be in its anionic form as butyrate or in its acid form as butyric acid. The term "butyrate" used in the present invention does not distinguish between both forms.
(2) nucleic acid molecules coding for a reverse β-oxidation pathway from acetyl-CoA to butyryl-CoA, comprising thiolase, 3-hydroxybutyryl-CoA dehydrogenase y, crotonase and trans-2-enoyl-CoA reductase such as described by Schadeweg and Boles (2016a, 2016b).
   In this pathway, two molecules of acetyl-CoA are condensed to acetoacetyl-CoA. This reaction is catalyzed by a thiolase, preferably via additional and stronger expression of the endogenous yeast thiolase gene *ERG10.* Then, acetoacetyl-CoA is reduced to 3-hydroxybutyryl-CoA and further converted into crotonyl-CoA via heterologous 3-hydroxybutyryl-CoA dehydrogenase and crotonase, respectively. Crotonyl-CoA is converted into butyryl-CoA by trans-2-enoyl-CoA reductase.

Optionally, the further conversion of butyryl-CoA to butyraldehyde and/or butyric acid and/or its extension to β-ketocaproyl-CoA and/or degradation via peroxisomal β-oxidation is prevented or decreased.

Preferably, the heterologous nucleic acid molecules (i) to (iii), and the nucleic acid molecules used for increased intracellular synthesis of propionyl-CoA and/or butyryl-CoA (iv),
are each linked to a promoter,
such as p*PGK1,* p*HXT7*^{-1- -392}, p*FBA1,* p*PDC1,* p*PYK1,* p*TPI1,* p*ADH1, pTDH3,* p*CCW12* and p*ENO2* promoters, or other promoters.

Preferably, the host cell contains the nucleic acid molecules, preferably linked to promoters, integrated into the genome.

The nucleic acid molecules according to the invention preferably comprise nucleic acid sequences, which are identical with the naturally occurring nucleic acid sequence or are codon-optimized for the use in a host cell.

Every amino acid is encrypted on a gene level by a codon. However, there are several different codons, which code for a single amino acid. Thus, the genetic code is degenerated. The preferred choice of a codon for a corresponding amino acid differs from organism to organism. Therefore, problems can arise in heterologously expressed genes if the host organism or the host cell has a very different codon usage. The gene can be expressed not at all or only slowly.

Even in genes from different metabolic pathways within an organism, a different codon usage can be discovered. It is known that strongly expressed genes from *S. cerevisiae* have a very restrictive codon usage (Coghlan and Wolfe, 2000). It can be assumed that by adapting the codon usage of the *MSAS* gene, the *npgA* gene, the *patG* gene and/or the prpE gene and/or the genes of the reverse β-oxidation pathway to the codon usage of strongly expressed genes from *S. cerevisiae,* an improvement of their respective enzymatic activity in yeast is achieved (Wiedemann and Boles, 2008; Hitschler and Boles, 2019; Schadeweg and Boles, 2016a and 2016b). The genes can be codon-optimized with the JCat tool (Grote *et al.,* 2005) that uses an algorithm proposed by Carbone *et al.* (2003). This algorithm calculates the codon adaptation index (CAI) developed by Sharp and Li (1987) for all genes of an organism and gives it a value compared to highly expressed genes with the highest codon bias in the organism as reference, identifying the codon usage for each amino acid in the process. For codon-optimization of a gene, its amino acid sequence is translated into a nucleic acid sequence utilizing codons with the highest CAI scores (Grote *et al.,* 2005).

Preferably, at least two of the heterologous nucleic acid molecules are codon-optimized for use in a host cell, preferably all three heterologous nucleic acid molecules (i) to (iii), and the nucleic acid molecule(s) used for increased intracellular synthesis of propionyl-CoA and/or butyryl-CoA (iv) are codon-optimized.

Preferably, the nucleic acid molecule coding for MSAS (i) comprises or consists of a nucleic acid sequence coding for an amino acid sequence of SEQ ID NO. 1 or coding for an amino acid sequence which is at least 90% or 95% identical to the amino acid sequence of SEQ ID NO. 1.

Preferably, the nucleic acid molecule coding for PPTase (ii) comprises or consists of a nucleic acid sequence coding for an amino acid sequence of SEQ ID NO. 2 or coding for an amino acid sequence which is at least 90% or 95% identical to the amino acid sequence of SEQ ID NO. 2.

Preferably, the nucleic acid molecule coding for 6-MSA decarboxylase (iii) comprises or consists of a nucleic acid sequence coding for an amino acid sequence of SEQ ID NO. 3 or coding for an amino acid sequence which is at least 90% or 95% identical to the amino acid sequence of SEQ ID NO. 3.

Preferably, the nucleic acid molecule coding for propionyl-CoA synthase comprises or consists of a nucleic acid sequence coding for an amino acid sequence of SEQ ID NO. 4 or coding for an amino acid sequence which is at least 90% or 95% identical to the amino acid sequence of SEQ ID NO. 4.

Preferably, the nucleic acid molecules coding for a reverse β-oxidation pathway comprises nucleic acid sequences coding for amino acid sequences of SEQ ID NOs. 5-8 or amino acid sequences which are at least 90% or 95% identical to the amino acid sequences of SEQ ID NOs. 5-8.

Under certain conditions, preventing the degradation of propionyl-CoA and/or butyryl-CoA might further increase the production of 3-EP and/or 3-PP.
In a preferred embodiment, a block in the degradation pathway of propionyl-CoA (v) is provided by mutation(s) in or, preferably deletion(s) of the gene(s) coding for 2-methylcitrate synthase(s), preferably *CIT3* and more preferably *CIT3* and *CIT2.*
In another preferred embodiment, a block in the degradation pathway of propionyl-CoA (v) is provided by mutation(s) in or, preferably deletion(s) of the gene(s) coding for 2-methylisocitrate lyase(s), preferably *ICL2* and more preferably *ICL2* and *ICL1* (Luttik *et al.,* 2000).

In a preferred embodiment, a block in the degradation pathway of butyryl-CoA (v) is provided by mutation(s) in or, preferably deletion of the *POX1* gene coding for fatty acyl-CoA oxidase.

An example of a host cell of the present invention is a yeast cell *S. cerevisiae,* wherein
- codon-optimized genes *^{Ppopt}MSAS* (from *Penicillium patulum*), *^{opt}npgA* (from *Aspergillus nidulans*) and *^{opt}patG* (from *Aspergillus clavatus*) together under control of the strong constitutive p*PGK1,* p*HXT7*^{-*1*- -*392*} and p*FBA1* promoters, respectively, are genomically integrated,
   such as into the *ura3* locus of *S. cerevisiae* strain CEN.PK2-1C,
   preferably in a culture medium containing propionate.

Another example of a host cell of the present invention is a yeast cell *S. cerevisiae,* wherein
- codon-optimized genes *^{Ppopt}MSAS* (from *Penicillium patulum*), *^{opt}npgA* (from *Aspergillus nidulans*) and *^{opt}patG* (from *Aspergillus clavatus*) together under control of the strong constitutive p*PGK1,* p*HXT7*^{-*1*- -*392*} and p*FBA1* promoters, respectively, are genomically integrated,
   such as into the *ura3* locus of *S. cerevisiae* strain CEN.PK2-1C or CEN.PK2-1C *Δcit2Δcit3,*
   and
- additionally a codon-optimized propionyl-CoA synthase *^{opt}prpE* gene from *Salmonella typhimurium* under control of *pTDH3* promoter is integrated,
   such as in the *SFA1* locus of said *S. cerevisiae* strain,
   preferably in a culture medium containing propionate.

Another example of a host cell of the present invention is a yeast cell *S. cerevisiae,* wherein
- codon-optimized genes *^{Ppopt}MSAS* (from *Penicillium patulum*), *^{opt}npgA* (from *Aspergillus nidulans*) and *^{opt}patG* (from *Aspergillus clavatus*) together under control of the strong constitutive p*PGK1,* p*HXT7*^{-*1*- *-392*} and p*FBA1* promoters, respectively, are genomically integrated,
   such as into the *ura3* locus of *S. cerevisiae* strain CEN.PK2-1C or CEN.PK2-1C *Δpox1,*
   and
- additionally a reverse β-oxidation pathway is provided by overexpression of genes *ERG10, ^{opt}Hbd, ^{opt}Crt* and *^{opt}Ter* under control of the strong promoters p*PGK1,* p*CCW12,* p*ENO2, pTDH3,* respectively, integrated e.g. into the *leu2* locus of said *S*. *cerevisiae* strain.

In one embodiment, the host cell of the present invention can comprise further gene deletion(s) and/or contain further heterologous nucleic acid molecule(s).

For example,
- the host cell of the present invention can comprise in addition to or instead of the nucleic acid molecule coding for MSAS (i) a mutant version of this nucleic acid molecule coding for an MSAS enzyme with increased affinity for propionyl-CoA and/or butyryl-CoA and/or increased catalytic activity for propionyl-CoA and/or butyryl-CoA conversion.
- the host cell of the present invention can comprise in addition to or instead of the nucleic acid molecule coding for 6-MSA decarboxylase (iii) a mutant version of this nucleic acid molecule coding for a 6-MSA decarboxylase enzyme with increased affinity for 6-ethylsalicylic acid and/or 6-propylsalicylic acid and/or increased catalytic activity for 6-ethylsalicylic acid and/or 6-propylsalicylic acid conversion.

### Uses of the host cells for the production of 3-alkylphenols

As discussed above, the present invention provides the use of the host cells according to the present invention for the production of 3-alkylphenols,
preferably 3-ethylphenol (3-EP) and/or 3-propylphenol (3-PP), optionally together with 3-methyl phenol (3-MP).

The host cells of the present invention express a metabolic pathway for 3-alkyl production.

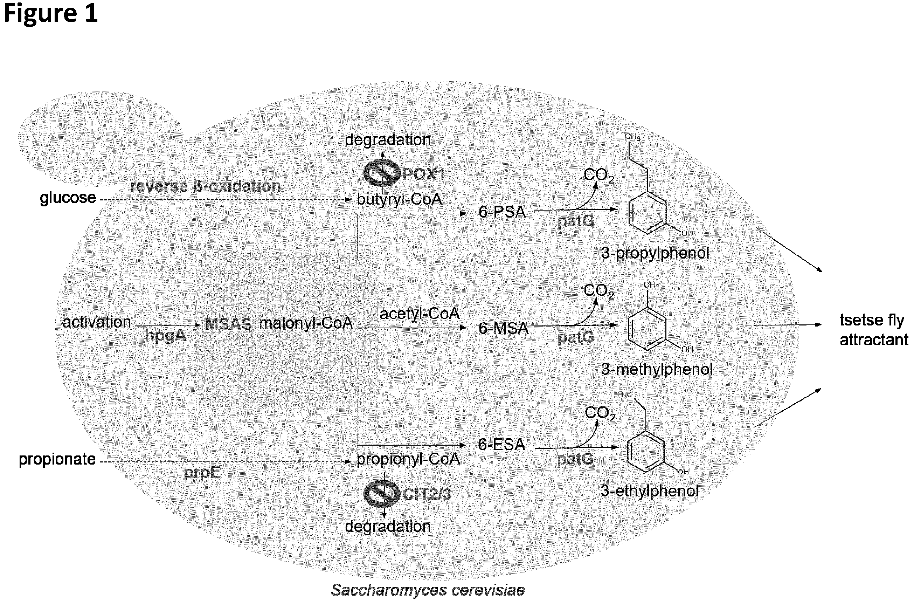

In a preferred embodiment, where the host cell of the present invention is an *S. cerevisiae* cell, 3-ethylphenol (3-EP) is produced from propionate and glucose via 6-ethylsalicylic acid (6-ESA). The bacterial propionyl-CoA synthase (prpE) is providing propionyl-CoA from propionate. Optionally, the propionyl-CoA degrading (2-methyl) citrate synthase genes *CIT2*/*3* are deleted. The endogenous enzyme acetyl-CoA carboxylase provides malonyl-CoA from acetyl-CoA that is synthesized from glucose. The 6-methylsalicylic acid synthase (MSAS), which is activated by phosphopantetheinylation (due to npgA), utilizes malonyl-CoA as extender unit and catalyzes the synthesis of 6-ESA and/or 6-MSA depending on the starter unit propionyl-CoA or acetyl-CoA, respectively. The 6-MSA decarboxylase (patG) converts the 6-alkylsalicylic acids to their respective 3-alkylphenols, see e.g. Figure 1.

In another preferred embodiment, where the host cell of the present invention is an *S*. *cerevisiae* cell, 3-ethylphenol (3-EP) is produced from propionate and glucose via 6-ethylsalicylic acid (6-ESA). Optionally, the propionyl-CoA degrading (2-methyl) citrate synthase genes *CIT2*/*3* are deleted. Propionyl-CoA is produced from propionate, such as via the endogenous acetyl-CoA synthase of the yeast cell. The endogenous enzyme acetyl-CoA carboxylase provides malonyl-CoA from acetyl-CoA that is synthesized from glucose. The 6-methylsalicylic acid synthase (MSAS), which is activated by phosphopantetheinylation (due to npgA), utilizes malonyl-CoA as extender unit and catalyzes the synthesis of 6-ESA and/or 6-MSA depending on the starter unit propionyl-CoA or acetyl-CoA, respectively. The 6-MSA decarboxylase (patG) converts the 6-alkylsalicylic acids to their respective 3-alkylphenols, see e.g. Figure 1.

In another preferred embodiment, where the host cell of the present invention is an *S*. *cerevisiae* cell, 3-ethylphenol (3-EP) is produced from glucose via 6-ethylsalicylic acid (6-ESA). Optionally, the propionyl-CoA degrading (2-methyl) citrate synthase genes *CIT2*/*3* are deleted. Propionyl-CoA synthesis from glucose is enhanced via increased threonine synthesis and degradation. The endogenous enzyme acetyl-CoA carboxylase provides malonyl-CoA from acetyl-CoA that is synthesized from glucose. The 6-methylsalicylic acid synthase (MSAS), which is activated by phosphopantetheinylation (due to npgA), utilizes malonyl-CoA as extender unit and catalyzes the synthesis of 6-ESA and/or 6-MSA depending on the starter unit propionyl-CoA or acetyl-CoA, respectively. The 6-MSA decarboxylase (patG) converts the 6-alkylsalicylic acids to their respective 3-alkylphenols, see e.g. Figure 1.

In another preferred embodiment, where the host cell of the present invention is an *S*. *cerevisiae* cell, 3-propylphenol (3-PP) is produced from glucose via 6-propylsalicylic acid (6-PSA). Butyryl-CoA synthesis is enhanced via a reverse β-oxidation pathway, comprising thiolase, 3-hydroxybutyryl-CoA dehydrogenase, crotonase and trans-2-enoyl-CoA reductase. Preferably, the *POX1* gene is deleted to block degradation of butyryl-CoA. The endogenous enzyme acetyl-CoA carboxylase provides malonyl-CoA from acetyl-CoA that is synthesized from glucose. The 6-methylsalicylic acid synthase (MSAS), which is activated by phosphopantetheinylation (due to npgA), utilizes malonyl-CoA as extender unit and catalyzes the synthesis of 6-PSA and/or 6-MSA depending on the starter unit butyryl-CoA or acetyl-CoA, respectively. The 6-MSA decarboxylase (patG) converts the 6-alkylsalicylic acids to their respective 3-alkylphenols, see e.g. Figure 1.

In a preferred embodiment, further increasing the intracellular ratio of propionyl-CoA and/or butyryl-CoA to acetyl-CoA, or the ratio of propinyl-CoA synthesis activity and/or butyryl-CoA synthesis activity to acetyl-CoA synthesis activity provides a higher ratio of 6-ESA and/or 6-PSA to 6-MSA, resulting in a higher ratio of 3-EP and/or 3-PP to 3-methylphenol (3-MP).

### Methods for the production of 3-alkylphenols

As discussed above, the present invention provides methods for the production of 3-alkylphenols.

A method for the production of 3-alkylphenols according to the present invention,
preferably of 3-ethylphenol (3-EP), optionally together with 3-methylphenol (3-MP), comprising the steps of
providing a host cell according to the present invention,
culturing the host cell by adding carbon source(s), preferably sugar(s) to the culture medium,
or culturing the host cell by adding carbon source(s) and propionate to the culture medium,
optionally, obtaining the 3-alkylphenols.

Another method for the production of 3-alkylphenols according to the present invention, preferably 3-propylphenol (3-PP), optionally together with 3-methylphenol (3-MP), comprises the steps of
providing a host cell according to the present invention,
culturing the host cell by adding carbon source(s), preferably sugar(s) to the culture medium,
or culturing the host cell by adding carbon source(s) and butyrate to the culture medium,
optionally, obtaining the 3-alkylphenols.

Another method for the production of 3-alkylphenols according to the present invention, preferably of 3-ethylphenol (3-EP) and 3-propylphenol (3-PP), optionally together with 3-methylphenol (3-MP)
comprises the steps of
providing a host cell according to the present invention,
culturing the host cell by adding carbon source(s), preferably sugar(s) to the culture medium,
or culturing the host cell by adding carbon source(s) and propionate and, optionally, butyrate to the culture medium,
optionally, obtaining the 3-alkylphenols.

In one embodiment, the host cells are cultured for a time period of up to 200 hours, such as about 150 hours.

As discussed above, the present invention provides a composition comprising 3-alkylphenols, preferably obtained by a method of the present invention.

Preferably, the composition comprises 3-ethylphenol (3-EP) and/or 3-propylphenol (3-PP), optionally in combination with 3-methylphenol (3-MP).

### Tsetse fly attractants

As discussed above, the present invention provides the use of a host cell according to the present invention as tsetse fly attractant.

As discussed above, the present invention provides the use of a composition according to the present invention as tsetse fly attractant.

As discussed above, the present invention provides the use of the host cell extracts and/or the host cell culture and/or the host cell culture supernatant obtained in a method of the present invention as tsetse fly attractant.

A tsetse fly attractant can for example be used in a flytrap.

As discussed above, the present invention provides a tsetse fly attractant composition, comprising
(a) the host cell according to the present invention,
(b) the composition according to the present invention, and/or
(c) the host cell extracts and/or the host cell culture and/or the host cell culture supernatant obtained in a method of the present invention,
(d) optionally, excipient(s) and/or carrier.

### Preferred embodiments

Here we present a simple and inexpensive method to produce alkylphenols in recombinant yeast from sugars and/or other carbon sources. The yeast cultures or extracts or supernatant can be easily incorporated in tsetse fly traps.

### - 3-methylphenol production

For synthesis of 3-methylphenol (m-cresol) we used the polyketide synthase, 6-methylsalicylic acid synthase (MSAS), and 6-methylsalicylic acid (6-MSA) decarboxylase (Hitschler and Boles, 2019) that were already reported to catalyze the formation of the intermediate m-cresol in patulin biosynthesis in many *Penicillium* and *Aspergillus species.* MSAS utilizes one acetyl-CoA and three malonyl-CoA in three decarboxylative claisen thioester condensations to form 6-methylsalicylic acid (6-MSA) (Parascandolo *et al.,* 2016). In the second step of patulin biosynthesis 6-MSA is decarboxylated by the 6-MSA decarboxylase to 3-methylphenol. Due to difficult cultivations and genetical limitations of their native fungal hosts, yeast as a heterologous production system is a promising alternative. The *MSAS* gene of *Penicillium patulum* together with a phosphopantetheinyl transferase gene (*npgA*) from *Aspergillus nidulans,* required for activation of MSAS, were already heterologously expressed in S. cerevisiae to produce the 3-methylphenol precursor 6-MSA (Wattanachaisaereekul *et al.,* 2008). Separately, the 6-MSA decarboxylase gene *patG* of *Aspergillus clavatus* was heterologously expressed to show 6-MSA conversion to 3-methylphenol in *S. cerevisiae* (Snini *et al.,* 2014).

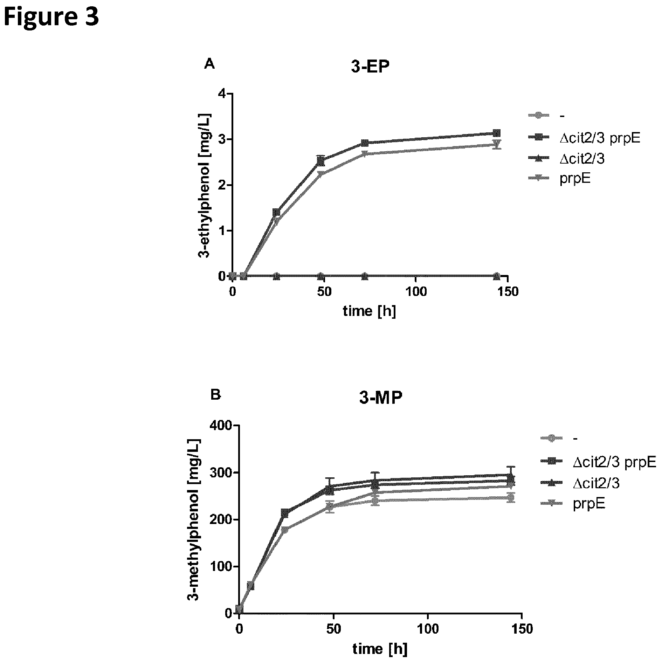

Recently, we established the pathway for *de novo* production of m-cresol from glucose in *S. cerevisiae* (Hitschler and Boles, 2019). In summary, the codon-optimized genes *^{Ppopt}MSAS, ^{opt}npgA* and *^{opt}patG* together under control of the strong constitutive p*PGK1,* p*HXT7^{-1- -392}* and p*FBA1* promoters, respectively, were genomically integrated into the *ura3* locus of *S. cerevisiae* strain CEN.PK2-1C. In this recombinant yeast endogenous pathways provide acetyl-CoA and malonyl-CoA from glucose, the NpgA-activated MSAS utilizes these starter and extender units to synthesize 6-MSA that is decarboxylated by PatG to 3-methylphenol (Figure 1). A high-OD fermentation of the yeast cells (starting OD₆₀₀ = 5) resulted in the production of up to 247 mg/L 3-methylphenol from 20 g/L glucose after 144 h in potassium phosphate (KPᵢ) buffered YPD (yeast extract peptone medium supplemented with 2 % glucose) at pH 6.5 (Figure 3B).

### - 3-ethylphenol production

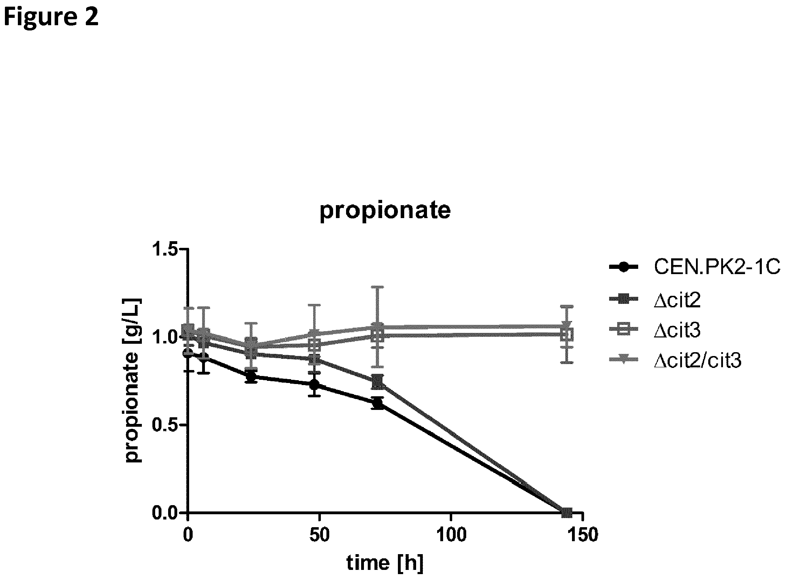

Dimroth *et al.* (1976) reported that *in vitro* MSAS catalyzed the formation of 6-ethylsalicylic acid (6-ESA) utilizing propionyl-CoA as a starter unit but at only 13 % of the 6-MSA formation rate from acetyl-CoA and Light and Vogel (1975) stated that PatG accepted 6-ethylsalicylic acid as a substrate to decarboxylate it to 3-ethylphenol *in vitro.* However, Dimroth *et al.* (1976) raised the concern that the different formation rates of 6-MSA from acetyl-CoA and 6-ESA from propionyl-CoA and the different concentrations of the starter units acetyl-CoA and propionyl-CoA in the cell could result in only 3-methylphenol formation via 6-MSA from acetyl-CoA *in vivo.* Indeed, in the fermentation of CEN.PK2-1C expressing *^{Ppopt}MSAS, ^{opt}npgA* and *^{opt}patG* we did not detect any 3-ethylphenol formation (Figure 3A). Though propionyl-CoA is for example mentioned as an intermediate in isoleucine and threonine catabolism (Pronk *et al.,* 1994; Luttik *et al.,* 2000), in *S. cerevisiae* the low amounts of propionyl-CoA in the cell are probably directly degraded by methylcitrate synthases CIT2 and CIT3 in the 2-methylcitrate cycle (Luttik *et al.,* 2000). Graybill *et al.* (2007) reported abolishment of propionate degradation when *CIT3* and *CIT2* were deleted. We could also show in a fermentation with a starting OD of 4 that in CEN.PK2-1C *Δcit3* or *Δcit2Δcit3* strains degradation of externally added propionate was blocked, compared to the CEN.PK2-1C wild-type strain or a *Δcit2* strain which completely consumed propionate over 144 h in KPᵢ buffered YPD pH 6.5 supplemented with 10 mM propionate (Figure 2), although the *Δcit2* strain seemed to consume it a little bit slower.

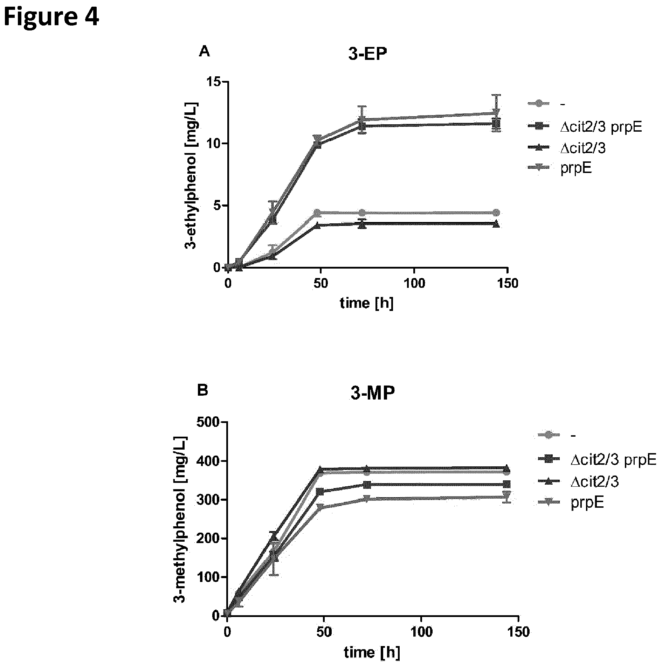

The previous experiment demonstrated that in a *Δcit3* strain propionyl-CoA should no longer be degraded. By deletion of *CIT3,* optionally together with *CIT2,* propionate degradation was blocked and propionyl-CoA should be available for intracellular 3-ethylphenol production. Now, we integrated *^{Ppopt}MSAS, ^{opt}npgA* and *^{opt}patG* together under control of the strong constitutive p*PGK1, pHXT7^{-1- -392}* and p*FBA1* promoters, respectively, in the *ura3*-locus of CEN.PK2-1C (resulting in strain A) and CEN.PK2-1c *Δcit2Δcit3* (resulting in strain B). In the latter strain and strain A we additionally integrated a codon-optimized propionyl-CoA synthase *^{opt}prpE* from *Salmonella typhimurium* (Mutka *et al.,* 2006) under control of p*TDH3* promoter in the *SFA1* locus (resulting in strain C and strain D, respectively). Fermentations were performed at 30°C in KPᵢ buffered YPD pH 6.5 over 144 h (starting OD= 5), with or without the addition of 10 mM propionate. No 3-EP was produced in strain A in the absence of propionate in the medium (Figure 3A). Without propionate addition to the medium, just the deletion of *CIT2*/*3* did not result in 3-ethylphenol production in strain B (Figure 3A), suggesting that degradation of endogenously provided propionyl-CoA is not the reason for lack of 3-ethylphenol production without propionate, more likely endogenously provided propionyl-CoA is not freely available in the cell without propionate. However, the expression of the heterologous ^{opt}*prpE* gene already resulted in low 3-ethylphenol production (2.9 mg/L) in strain D, even without propionate addition to the medium (Figure 3A), indicating that endogenously provided propionyl-CoA normally is limiting 3-ethylphenol production and the heterologous PrpE is supplying intracellular propionyl-CoA as a MSAS starter unit through an unknown mechanism. When the PrpE was expressed, additional deletion of *CIT3*/*2* only slightly increased 3-ethylphenol production further in strain C (Figure 3A), suggesting that degradation of propionyl-CoA might limit 3-ethylphenol production only slightly or only under specific conditions. On the other hand, addition of 10 mM propionate to cultures of strain A resulted in up to 4.4 mg/L 3-ethylphenol (Figure 4A), even without expression of the heterologous ^{opt}*prpE* gene and without a block in the degradation pathway of propionyl-CoA. This is probably due to the fact that the endogenous acetyl-CoA synthase of *S. cerevisiae* is able to accept propionate as a substrate in a minor side reaction to synthesize propionyl-CoA (van den Berg *et al.,* 1996) and this propionyl-CoA was converted by activated ^{Ppopt}MSAS and ^{opt}PatG to 3-ethylphenol. When propionate was supplemented in the medium, in this case blockage of the degradation of propionyl-CoA in strain B did not further increase 3-ethylphenol production (Figure 4A).

The highest 3-ethylphenol titers with up to 11.6 mg/L and 12.5 mg/L were achieved when propionate was supplemented and additionally *^{opt}prpE* was expressed in CEN.PK2-1C *Δcit2Δcit3 sfa1::^{opt}prpE ura3:: ^{Ppopt}MSAS-^{opt}npgA-^{opt}patG* cultures (strain C) and CEN.PK2-1C *sfa1::^{opt}prpE ura3:: ^{Ppopt}MSAS-^{opt}npgA-^{opt}patG* cultures (strain D), respectively (Figure 4B). All strains still produced 3-methylphenol (Figure 3B and Figure 4B). This is probably due to the fact that the MSAS starter unit acetyl-CoA is still provided by endogenous pathways, e.g. via acetyl-CoA synthases (ACS) from glucose (van den Berg *et al.,* 1996).

To conclude,
(i) just expressing a heterologous propionyl-CoA synthase gene leads to an increase in intracellular propionyl-CoA concentrations through an unknown mechanism without extracellularly provided propionate, and it can be converted to 3-EP by activated MSAS and 6-MSA decarboxylase;
(ii) although yeast can already convert extracellularly provided propionate into propionyl-CoA via endogenous enzymes, expression of a heterologous propionyl-CoA synthase gene in a strain (optionally with impaired propionyl-CoA degradation) together with addition of propionate to the medium led to the highest 3-EP production by activated MSAS and 6-MSA decarboxylase.

### - 3-propylphenol production

Reports revealed that *in vitro* MSAS accepted propionyl-CoA and butyryl-CoA besides the natural precursor acetyl-CoA forming 6-ethylsalicylic acid (6-ESA) and 6-propylsalicylic acid (6-PSA) besides 6-methylsalicylic acid (6-MSA) (Dimroth *et al.,* 1976; Richardson and Pohl, 1999). 3-propylphenol (3-PP) would arise from decarboxylation of 6-PSA if the 6-MSA decarboxylase PatG would also accept 6-PSA as substrate but only 6-ESA and 6-MSA were reported substrates (Light and Vogel, 1975).

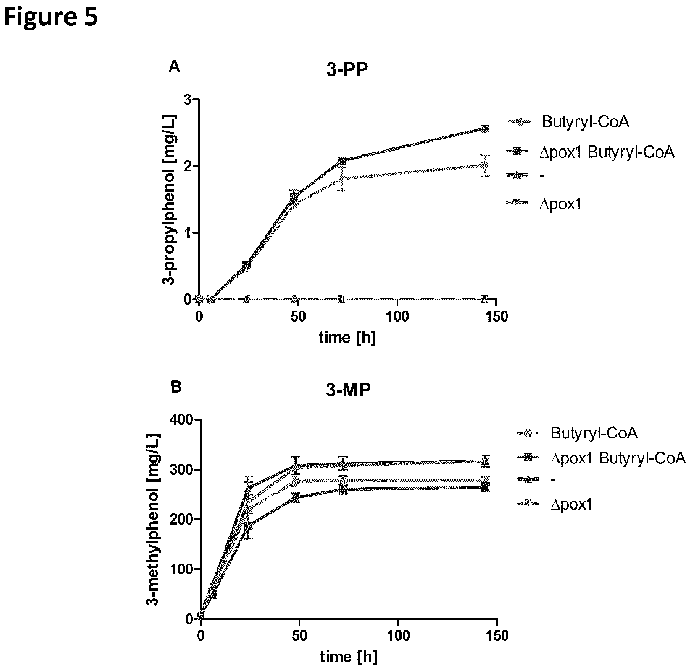

When we expressed *^{Ppopt}MSAS, ^{opt}npgA* and *^{opt}patG* under control of the strong constitutive p*PGK1, pHXT7^{-1- -392}* and p*FBA1* promoters in CEN.PK2-1C (resulting in strain A) and performed a high OD-fermentation (intial OD = 5), we did not detect any 3-PP (Figure 5A) but 3-methylphenol (3-MP; Figure 5B) probably due to the fact that the MSAS starter unit acetyl-CoA is still provided by endogenous pathways, e.g. via acetyl-CoA synthases (ACS) from glucose (van den Berg *et al.,* 1996) and via fatty acid catabolism, whereby butyryl-CoA is also an intermediate but propably directly further degraded to acetyl-CoA by fatty acyl-CoA oxidase POX1 and β-oxidation (Dmochowska *et al.,* 1990).

To provide butyryl-CoA for 3-propylphenol production in yeast cells a heterologous butyryl-CoA synthase, for example AAE7 from *Arabidopsis thaliana* could be employed but there were controversial reports about the substrate preferences between acetate and butyrate (Turner *et al.,* 2005; Shockey *et al.,* 2003). Recently, we established a reverse β-oxidation pathway for *n*-butanol production with butyryl-CoA as intermediate (Schadeweg and Boles, 2016a; 2016b). In this pathway, two acetyl-CoA are condensed to acetoacetyl-CoA catalyzed by thiolase ERG10, the next reduction to 3-hydroxybutyryl-CoA and then dehydration to crotonyl-CoA are catalyzed by Hbd and Crt from *Clostridium acetobutylicum* and the final reduction to butyryl-CoA is catalyzed by trans-2-enoyl-CoA reductase Ter from *Treponema denticola.* For intracellular butyryl-CoA provision, we integrated the reverse β-oxidation pathway genes *ERG10, ^{opt}Hbd, ^{opt}Crt* and *^{opt}Ter* required for butyryl-CoA formation under control of the strong constitutive promoters pPGK1p, pCCW12, pENO2, pTDH3, respectively, together with a clonat resistance cassette for selection into the *leu2* locus of strain A (resulting in strain E).
It should be mentioned here that integration and/or expression of an additional copy of the endogenous yeast *ERG10* coding sequence, or optionally a codon-optimized version, linked to a strong promoter might not be necessary to establish the reverse β-oxidation pathway but that the native endogenous *ERG10* gene already will be enough to establish the reverse β-oxidation pathway; alternatively the promoter of the native endogenous *ERG10* gene might be replaced by a stronger promoter.
We additionally deleted fatty acyl-CoA oxidase *POX1* (Dmochowska *et al.,* 1990) in the latter strain E and strain A to prevent degradation of butyryl-CoA via β-oxidation (resulting in strains F and strain G, respectively).

Fermentations were performed at 30°C in KPᵢ buffered YPD pH 6.5 over 144 h (starting OD = 5). Strain A and strain G did not produce any 3-PP (Figure 5A), suggesting that endogenously provided intracellular butyryl-CoA levels are too low or not freely available in the cell and just deletion of *POX1* is not sufficient for 3-propylphenol production. On the other hand, expression of the reverse β-oxidation pathway resulted in 3-propylphenol production (2 mg/L) in strain E even without a block in the degradation pathway of butyryl-CoA, indicating that the reverse β-oxidation pathway is providing more butyryl-CoA than is degraded. However, the highest 3-PP titers with up to 2.6 mg/L were accomplished when *POX1* was additionally deleted (strain F: CEN.PK2-1C *Δpox1 ura3:: ^{Ppopt}MSAS-^{opt}npgA-^{opt}patG leu2:: Erg10-^{opt}Hbd-^{opt}Crt-^{opt}Ter-natMX*). The results indicated that degradation of endogenously provided butyryl-CoA is partially limiting 3-propylphenol production. 3-methylphenol was still produced by all strains (Figure 5B) probably because endogenous pathways are still providing the MSAS starter unit acetyl-CoA, e.g. via acetyl-CoA synthases (ACS) from glucose (van den Berg *et al.,* 1996).

To conclude,
(i) just blocking the degradation of butyryl-CoA was not sufficient to raise intracellular butyryl-CoA concentrations via yeast endogenous metabolism, and convert it to 3-PP by activated MSAS and 6-MSA decarboxylase;
(ii) overexpression of reverse β-oxidation genes led to an increase in intracellular butyryl-CoA concentrations, which were converted to 3-PP by activated MSAS and 6-MSA decarboxylase although yeast could still degrade butyryl-CoA via endogenous β-oxidation;
(ii) blocking butyryl-CoA degradation in a strain expressing the reverse β-oxidation pathway further increased intracellular butyryl-CoA concentrations and led to the highest 3-PP production by activated MSAS and 6-MSA decarboxylase.

The following examples and drawings illustrate the present invention without, however, limiting the same thereto.

### BRIEF DESCRIPTION OF THE DRAWINGS

**Figure 1****.** *Metabolic pathway for 3-alkylphenol production in S. cerevisiae* mediated by 6-methylsalicylic acid synthase (MSAS) and 6-methylsalicylic acid (6-MSA) decarboxylase utilizing intracellularly provided propionyl-CoA and/or butyryl-CoA.
   In the host *S. cerevisiae* the heterologous polyketide synthase MSAS which must be activated by phosphopantetheinyl transferase (npgA) catalyses the formation of 6-methylsalicylic acid (6-MSA) utilizing malonyl-CoA as extender unit and acetyl-CoA as starter unit that are provided by endogenous pathways. When the bacterial propionyl-CoA synthase (prpE) and/or propionate are present and optionally the propionyl-CoA degrading (methyl) citrate synthases CIT2/3 are deleted, intracellular propionyl-CoA supply is increased and MSAS is also able to utilize propionyl-CoA as starter unit to catalyse the formation of 6-ethylsalicylic acid (6-ESA). The starter unit butyryl-CoA for the formation of 6-propylsalicylic acid (6-PSA) is supplied by the reverse β-oxidation pathway described by Schadeweg and Boles (2016a; 2016b).
   Once 6-MSA, 6-ESA and/or 6-PSA are formed, the fungal 6-MSA decarboxylase (patG) converts the 6-alkylsalicylic acids to their respective 3-alkylphenols (3-methylphenol, 3-ethlphenol and/or 3-propylphenol), which are valuable as tsetse fly attractants.
**Figure 2****.** Blockage of *propionate consumption by deletion of methylcitrate synthases CIT2 and CIT3.*
   Propionate consumption was followed in *S. cerevisiae* wild-type strain CEN.PK2-1C and deletion strains that either had peroxisomal (Δcit2), mitochondrial (Δcit3) or both methylcitrate synthases Δcit2 Δcit3 deleted. Strains were pre-grown in potassium phosphate buffered YPD medium at pH 6.5 at 30°C overnight, then fermentations were started with an initial OD of 4 and cultivated for 144 h at 30°C in 25 mL potassium phosphate buffered YPD medium at pH 6.5 with supplementation of 10 mM propionate. Propionate concentrations were determined in the culture supernatants 0 h, 6 h, 24 h, 48 h, 72 h and 144 h via HPLC analysis. Error bars represent standard deviations of biological duplicates.
**Figure 3****.** *3-ethylphenol (A) and 3-methylphenol production (B) and determination of the factors enabling 3-ethylphenol production without supplementation of external propionate.* 3-alkylphenol production was tracked in CEN.PK2-1C expressing the 3-methylphenol pathway (*^{Ppopt}MSAS, ^{opt}npgA* and *^{opt}patG;* Hitschler and Boles, 2019) with or without additional expression of a propionyl-CoA synthase *^{opt}prpE* to provide the precursor propionyl-CoA that is converted by the activated MSAS and decarboxylase to 3-ethylphenol and with or without *Δcit2Δcit3* double deletion to block degradation of the precursor propionyl-CoA. Strains were pre-grown in potassium phosphate buffered YPD medium at pH 6.5 at 30°C overnight. Fermentations (starting OD = 5) were performed in biological duplicates at 30°C for 144 h in 25 mL potassium phosphate buffered YPD medium at pH 6.5. Culture supernatants were analysed via HPLC for 3-alkylphenol production. Error bars represent standard deviations of biological duplicates.
**Figure 4****.** *3-ethylphenol (A) and 3-methylphenol production (B) and determination of the factors enabling 3-ethylphenol production with supplementation of external propionate.* 3-alkylphenol production was followed in CEN.PK2-1C expressing the 3-methylphenol pathway (*^{Ppopt}MSAS, ^{opt}npgA* and *^{opt}patG;* Hitschler and Boles, 2019) with or without additional expression of a propionyl-CoA synthase *^{opt}prpE* and with propionate to provide the precursor propionyl-CoA that is converted by the activated MSAS and decarboxylase to 3-ethylphenol and with or without *Δcit2Δcit3* double deletion to block degradation of the precursor propionyl-CoA. Strains were pre-grown in potassium phosphate buffered YPD medium at pH 6.5 at 30°C overnight. Fermentations (starting OD = 5) were performed in biological duplicates at 30°C for 144 h in 25 mL potassium phosphate buffered YPD medium at pH 6.5 supplemented with 10 mM propionate. Culture supernatants were analysed via HPLC for 3-alkylphenol production. Error bars represent standard deviations of biological duplicates.
**Figure 5****.** *3-propylphenol (A) and 3-methylphenol production (B) via reverse β-oxidation and* pox1 *deletion.*
   3-alkylphenol production was followed in CEN.PK2-1C expressing the 3-methylphenol pathway (*^{Ppopt}MSAS, ^{opt}npgA* and *^{opt}patG;* Hitschler and Boles, 2019) with or without additional expression of the reverse β-oxidation pathway (*ERG10, ^{opt}Hbd, ^{opt}Crt* and *^{opt}Ter;* Schadeweg and Boles, 2016a, 2016b) for provision of butyryl-CoA that is converted by activated MSAS and decarboxylase to 3-propylphenol and with or without carrying *Δpox1* deletion to block degradation of the precursor butyryl-CoA via β-oxidation. Strains were pre-grown in potassium phosphate buffered YPD medium at pH 6.5 at 30°C overnight. Fermentations (starting OD = 5) were performed in biological duplicates at 30°C for 144 h in 25 mL potassium phosphate buffered YPD medium at pH 6.5. Culture supernatants were analysed via HPLC for 3-alkylphenol production. Error bars represent standard deviations of biological duplicates.

### EXAMPLES

### EXAMPLE 1

### 1. Materials and Methods

In general, materials and methods were used and performed as described in general textbooks about yeast molecular biology and genetics methods (e.g. Guthrie and Fink, 1990; 2002) and as in Hitschler and Boles (2019).

### 1.1 Strains and plasmids

Yeast strains and plasmids used in this study are described in Hitschler and Boles (2019) or are listed in Table 1.

**Table 1. Plasmids and yeast strains used in the present study. Genes from Aspergillus nidulans (An), Aspergillus clavatus (Ac), Clostridium acetobutylicum (Ca), Escherichia coli (Ec), Penicillium patulum (Pp), Saccharomyces cerevisiae (Sc), Salmonella enterica serovar typhimurium (St), Treponema denticola (Td), codon-optimized genes (opt) are indicated by prefixes in superscript. Other abbreviations: hphNT1: hygromycin resistance; Amp^{r}: ampicillin resistance; Cam^{R}: chloramphenicol resistance; Kan^{R}: kanamycin resistance; kanMX: geneticin resistance; natMX: clonat resistance. If not stated otherwise, promoters (p) were taken 1-500 bp upstream and terminators (t) 1-300 bp downstream of respective open reading frames.**

| **Plasmid** | **Plasmid based on** | **Relevant features** | **Reference** |
|---|---|---|---|
| pRS42K | - | *2µ, kanMX, Amp^{r}* | Taxis and Knop, 2006 |
| pRCC-K | - | *2µ, kanMX, Amp^{r}, pROX3-^{opt}Cas9-tCYC1, pSNR52-gRNA* | Generoso *et al.,* 2016 |
| pRS42K_prpE^{783Δ} | pJHV1 | *2µ, kanMX, Amp^{r}, pMET25, tCYC1, pTDH3-^{Stopt}prpE^{G783}-tPGK1* | This work |
| pJHV19 | pRCC-K | *2µ, kanMX, Amp^{r}, pROX3-^{opt}Cas9-tCYC1, pSNR52-gRNA* for *SFA1* | This work |
| pJHV54 | pRCC-K | *2µ, kanMX, Amp^{r}, pROX3-^{opt}Cas9-tCYC1, pSNR52-gRNA* for *CIT3* | This work |
| pJHV62 | - | *ColE1, Amp^{R},ConL3'-pTDH3-^{Tdopt}Ter-tADH1-ConRE'* | This work |
| pJHV65 | - | *ConLS'-pPGK1p-^{Sc}ERG10-tVMA16-ConR1'-ConL1'-pCCW12-^{Caopt}Hbd-tIDP -ConR2'-ConL2'-pENO2-^{Caopt}Crt-tPGK1 -ConR3'-ConL3'-pTDH3-^{Tdopt}Ter-tADH1 -ConRE'-natMX-LEU2 3'Hom-KanR-ColE1-LEU 5'Hom* | This work |
| SiHV110 | - | *ConLS'-gfp-dropout-ConRE'-natMX-LEU2 3'Hom-KanR-ColE1-LEU 5'Hom* | This work |
| pYTK3.41 | - | *ColE1, Cam^{R}, ^{Tdopt}Ter* | This work |
| pYTK3.41 | - | *ColE1, Cam^{R}, ^{Tdopt}Ter* | This work |
| pYTK3.43 | - | *ColE1, Cam^{R}, ^{Caopt}Crt* | This work |
| pYTK3.47 | - | *ColE1, Cam^{R}, ^{Sc}ERG10* | This work |
| pYTK3.49 | - | *ColE1, Cam^{R}, ^{Caopt}Hbd* | This work |
| pYTK_Erg10 | - | *ColE1, Amp^{R},ConLS'-pPGK1-^{Sc}ERG10-tVMA16-ConR1'* | This work |
| pYTK_Hbd | - | *ColE1, Amp^{R},ConL1'-pCCW12-^{Caopt}Hbd-tIDP-ConR2'* | This work |
| pYTK_Crt | - | *ColE1, Amp^{R},ConL2'-pENO2-^{Caopt}Crt-tPGK1 -ConR3'* | This work |
| pYTK01 | - | *ColE1, Cam^{R}, gfp-dropout* | Lee *et al.,* 2015 |
| pYTK05 | - | *ColE1, Cam^{R}, ConL3* | Lee *et al.,* 2015 |
| pYTK09 | - | *ColE1, Cam^{R}, TDH3p* | Lee *et al.,* 2015 |
| pYTK53 | - | *ColE1, Cam^{R}, ADH1t* | Lee *et al.,* 2015 |
| pYTK72 | - | *ColE1, Cam^{R}, ConRE* | Lee *et al.,* 2015 |
| pYTK95 | - | *ColE1, Cam^{R}, Amp^{R}-ColE1* | Lee *et al.,* 2015 |
| pVS06 | - | *CEN6ARS4, kanMX, Amp^{r}, pHXT7⁻¹⁻⁻³⁹²*-*^{Sc}Erg10-tVMA16, pPGK1-^{Caopt}Hbd-tEFM1, pTPI1-^{Caopt}Crt-tYHI9, pPYK1-^{Tdopt}Ter-tIDP1, pADH1-^{Caopt}AdhE2-tRPL3, pTDH3-^{Ecopt}EutE-tRPL41B* | Schadeweg & Boles, 2016b |
| pAB02 | - | *2µ, natMX, Amp^{r}, pROX3-^{opt}Cas9-tCYC1, pSNR52-gRNA* for *POX1* | This work |
| pAB09 | - | *2µ, natMX, Amp^{r}, pROX3-^{opt}Cas9-tCYC1, pSNR52-gRNA* for *CIT2* | This work |
| | | | |
| CEN.PK2-1C | - | *MATa leu2-3,112 ura3-52 trp1-289 his3-Δ1 MAL2-8^{c} SUC2* | Entian and Kötter, 2007 |
| JHY65 | CEN.PK2-1C | *psfa1-sfa1Δ::pTDH3-^{Stopt}prpE-tSFA1* | This work |
| JHY162 (strain A) | CEN.PK2-1C | (strain *ura3::pPGK1-^{Ppopt}MSAS-tCYC1, pHXT7*^{*- 1* -392}-*^{Anopt}npgA*-*tFBA1*,*pFBA1-^{Acopt}patG-tADH1* | Hitschler & Boles, 2019 |
| JHY164 | CEN.PK2-1C | *cit2Δ* | This work |
| JHY174 | JHY164 | *cit2Δ cit3Δ* | This work |
| JHY175 | CEN.PK2-1C | *cit3Δ* | This work |
| JHY179 | JHY65 | *psfa1-sfa1Δ::pTDH3-^{Stopt}prpE-tSFA1 cit3Δ* | This work |
| JHY180 | JHY179 | *psfa1-sfa1Δ::pTDH3-^{Stopt}prpE-tSFA1 cit3Δ cit2Δ* | This work |
| JHY185 (strain C) | JHY180 | *psfa1-sfa1Δ::pTDH3-^{Stopt}prpE-tSFA1 cit3Δ cit2Δ ura3::pPGK1-^{Ppopt}MSAS-tCYC1, pHXT7⁻¹⁻⁻³⁹²-^{Anopt}npgA-tFBA1, pFBA1-^{Acopt}patG-tADH1* | This work |
| JHY194 (strain E) | JHY162 (strain A) | *ura3::pPGK1-^{Ppopt}MSAS-tCYC1, pHXT7*^{*- 1*--*392*}-*^{Anopt}npgA*-*tFBA1*,*pFBA1-^{Acopt}patG-tADH1 leu2::pPGK1-^{Sc}Erg10-tVMA16, pCCW12-^{Caopt}Hbd-tIDP, pENO2-^{Caopt}Crt-tPGK1, pTDH3-^{Tdopt}Ter-tADH1, pTEF-natMX-tTEF* | This work |
| JHY196 | CEN.PK2-1C | *pox1Δ* | This work |
| JHY197 (strain B) | JHY174 | *cit2Δ cit3Δ ura3::pPGK1-^{Ppopt}MSAS-tCYC1, pHXT7⁻¹⁻⁻³⁹²-^{Anopt}npgA-tFBA1, pFBA1-^{Acopt}patG-tADH1* | This work |
| JHY211 (strain G) | JHY196 | *pox1Δ ura3::pPGK1-^{Ppopt}MSAS-tCYC1, pHXT7⁻¹⁻⁻³⁹²-^{Anopt}npgA-tFBA1, pFBA1-^{Acopt}patG-tADH1* | This work |
| JHY212 (strain F) | JHY211 (strain G) | *pox1Δ ura3::pPGK1-^{Ppopt}MSAS-tCYC1, pHXT7⁻¹⁻⁻³⁹²*-*^{Anopt}npgA-tFBA1, pFBA1-^{Acopt}patG-tADH1 leu2::pPGK1-^{Sc}Erg10-tVMA16, pCCW12-^{Caopt}Hbd-tIDP, pENO2-^{Caopt}Crt-tPGK1, pTDH3-^{Tdopt}Ter-tADH1, pTEF-natMX-tTEF* | This work |
| JHY218 (strain D) | JHY65 | *sfa1p-sfa1Δ::TDH3p-^{Stopt}prpE-SFA1t ura3::pPGK1-^{Ppopt}MSAS-tCYC1, pHXTT ^{1- -392}-^{Anopt}npgA-tFBA1, pFBA1-^{Acopt}patG-tADH1* | This work |

### 1.2 Plasmid and strain construction

The DNA sequences of *patG, MSAS, npgA* and *prpE* were codon-optimized with the JCat tool (Grote *et al.,* 2005), then referred to as *^{opt}patG, ^{Ppopt}MSAS, ^{opt}npgA* and *^{opt}prpE* and ordered as described in Hitschler and Boles (2019). Open reading frames, promoters and terminators were amplified by PCR from genomic DNA of CEN.PK 2-1C or from plasmids with 35 bp homologous overlaps. Primers and genes used in this study are described in Hitschler and Boles (2019), Schadeweg and Boles (2016a; 2016b) or are listed in Table 2 and 3. Plasmid assembly in yeast via homologous recombination or in *E. coli* via Gibson assembly (Gibson *et al.,* 2009) were conducted as described previously (Hitschler and Boles 2019). Yeast was transformed with DNA fragments according to Gietz and Schiestl (2007). Assembled plasmids were recovered by yeast DNA preparations or transformed directly in *E. coli* for propagation and amplification. Genomic integrations into the *ura3* locus of CEN.PK2-1C were performed with the CRISPR/Cas9 system (Generoso *et al.,* 2016) as described in Hitschler and Boles (2019). The CRISPR/Cas9 system was also utilized for deletions. For this purpose, CRISPR/Cas9 plasmids carrying the guide RNA (gRNA) for the specific deletion were constructed utilizing overlapping primers with the gRNA sequence as overhang and pRCC-K as template and the two PCR fragments were assembled via Gibson. The primers used as donor DNA consisted of 40 bp upstream and 40 bp downstream sequences of the open reading frame and were annealed to double stranded DNA as described previously (Reifenrath and Boles, 2018). For genomic integration of the butyryl-CoA pathway genes (Schadeweg and Boles) an integration vector was assembled with the Golden Gate system (Lee *et al.,* 2015).

The Golden Gate system utilizes part plasmids consisting of a sequence of interest flanked by *Bsa*I-generated overhangs and conferring chloramphenicol resistance in *E. coli.* Most part plasmids were obtained from Lee *et al.* (2015), only pYTK3.41, pYTK3.43, pYTK3.47 and pYTK3.49 were assembled in this work. For assembly of the part plasmids, *^{opt}Ter, ^{opt}Crt, ERG10* and *^{opt}Hbd* were amplified by PCR, respectively, with pVS6 (Schadeweg & Boles, 2016b) as DNA-template and primers creating overhangs as part plasmid type 3. Part plasmids were assembled with the respective PCR product and pYTK01 as backbone with *Esp3*I as described previously (Lee *et al.,* 2015) incubating the reaction mixture for 10 min at 37°C, using 15 cycles of digestion and ligation (37°C 2 min, 16°C 5 min) and heat inactivating the enzymes at 60°C for 10 min and 80°C for 10 min and transformed into *E. coli.* For single gene expression in yeast, part plasmids pYTK3.41, pYTK05, pYTK09, pYTK053, pYTK72 and pYTK95 were assembled to the cassette plasmid pJHV62 with *Bsa*I-HF as described previously (Lee *et al.,* 2015) incubating the reaction mixture as described for the part plasmid assembly and transformed into *E. coli.* The other cassette plasmids pYTK_Erg10, pYTK_Hbd, pYTK_Crt and SiHV110 were assembled similarly as described above utilizing part plasmids from Lee *et al.* (2015) and part plasmid pYTK3.47, pYTK3.49, and pYTK3.43 for the reverse β-oxidation genes, respectively. Next, the cassette plasmids pYTK_Erg10, pYTK_Hbd, pYTK_Crt, pJHV62 and SiHV110 were assembled with *Esp3*I to the integration vector pJHV65 incubating the reaction mixture for 10 min at 37°C, using 25 cycles of digestion and ligation (37°C 1.5 min, 16°C 3 min), 37°C for 5 min and heat inactivating the enzymes at 50°C for 5 min and 80°C for10 min and transformed into *E. coli.* For genomic integration in the *LEU2* locus pJHV65 was carrying 500 bp homologous sequences to the upstream and downstream region of *LEU2* for homologous recombination in yeast and a *natMX* cassette for selection and pJHV65 was digested with *Not*I. After yeast transformation cells were streaked out on selective YPD medium.

**Table 2. Primers for plasmid or strain construction used in the present study**

| **Primer name** [SEQ ID NO.] | **5'-3' sequence** | **Application** |
|---|---|---|
| **Deletion of *CIT2*** | | |
| ABP047_ProCIT2_fw [SEQ ID NO. 17] | CATTTATCCGGTGGTCATCG | amplification of *CIT2*, forward |
| ABP048_terCIT2_ rev [SEQ ID NO. 18] | GCTAGCCAAGGCAGTAAGG | amplification of *CIT2*, reverse |
| ABP049_CIT2_rev [SEQ ID NO. 19] | GCTTTCCAAGGCAGTTACAG | sequencing of *CIT2*, reverse |
| ABP054_CIT2_Del40_Oligo [SEQ ID NO. 20] | CTCAAAACTTTTTGTTTTAATAATAC TAGTAACAAGAAAATTGGATTACATC CTACTTTTACACCCCTCTGCATATTT TT | Donor-DNA for deletion of *CIT2* binding in *CIT2p* with overhang to *CIT2t*, forward |
| ABP055_CIT2_Del40_Oligo_Comp [SEQ ID NO. 21] | AAAAATATGCAGAGGGGTGTAAAAGT AGGATGTAATCCAATTTTCTTGTTAC TAGTATTATTAAAACAAAAAGTTTTG AG | Donor-DNA for deletion of *CIT2* binding in *CIT2t* with overhang to *CIT2p*, reverse |

| **Cloning of pJHV54** | | |
|---|---|---|
| MGP126_CrCASseq. fw [SEQ ID NO. 22] | GGGAAACGCCTGGTATC | sequencing of gRNA, forward |
| WGP243_S-Cas9-1_Rv [SEQ ID NO. 23] | TCTTCTTGAAGTAGTCTTCC | amplification of pRCC, reverse |
| WGP245_S-Cas9-3_Fw [SEQ ID NO. 24] | GGCTATTGTTGACTTGTTG | amplification of pRCC, forward |
| JHP233_gRNA_CIT3_f [SEQ ID NO. 25] | cttctgcatatatgtgcgcccgTTTTA GAGCTAGAAATAGCAAGTTAAAATAA GG | amplification of pRCC with gRNA for *CIT3,* forward |
| JHP234_gRNA_CIT3_r [SEQ ID NO. 26] | cggcgcacatatatgcagaagGATCA TTTATCTTTCACTGCGGAGA | amplification of pRCC with gRNA for *CIT3*, reverse |

| **Deletion of *CIT3*** | | |
|---|---|---|
| JHP237_CIT3p_f [SEQ ID NO. 27] | CCATGGTAGCGGTTCTAAAG | amplification of *CIT3*, forward |
| JHP238_CIT3t_r [SEQ ID NO. 28] | TTTGTAAACGGCCCGAGG | amplification of *CIT3*, reverse |
| JHP239_CIT3seq_f [SEQ ID NO. 29] | GGGATTAGCGGGTCCTTTG | seqencing of *CIT3,* forward |
| JHP240_CIT3_del40_Oligo [SEQ ID NO. 30] | AGAATTTATACATAGACGCCGCTAAA TAATTGAATACAAACGCAGTTCCAAT TTACAAGAATGCTTCGTTTGCTATTA CAA | Donor-DNA for deletion of *CIT3* binding in *CIT3p* with overhang to *CIT3t*, forward |
| JHP241_CIT3_del40 _Oligo_comp [SEQ ID NO. 31] | TTGTAATAGCAAACGAAGCATTCTTG TAAATTGGAACTGCGTTTGTATTCAA TTATTTAGCGGCGTCTATGTATAAAT TCT | Donor-DNA for deletion of *CIT3* binding in *CIT3t* with overhang to *CIT3p,* reverse |

| **Cloning of pJHV19** | | |
|---|---|---|
| JHP108_CC_sfa1#2_r [SEQ ID NO. 32] | cacagtcccgcagccaaatacGATCA TTTATCTTTCACTGCGGAG | amplification of pRCC with gRNA for *SFA1*, reverse |
| JHP107_CC_sfa1#2_f [SEQ ID NO. 33] | gtatttggctgcgggactgtgTTTTA GAGCTAGAAATAGCAAGTTAAAATAA GG | amplification of pRCC with gRNA for *SFA1*, reverse |

| **Genomic integration of *TDH3p-^{Stopt}prpE-sfa1t* into *sfa1* locus** | | |
|---|---|---|
| JHP018_ovTDH3p_r [SEQ ID NO. 34] | ttgatagatctttggtagaattcaga gaaagacatTTTGTTTGTTTATGTGT GTTTATTC | amplification of *TDH3p* with overhang to *prpE* and pJHV1, reverse |
| JHP019_ovPGK1t_f [SEQ ID NO. 35] | aacaaatcagacaagctatcgaagaa taaccgcggATTGAATTGAATTGAAA TCGATAG | amplification of *PGK1t* with overhang to *prpE* and pJHV1, forward |
| JHP058_prpE_ovTD H3p [SEQ ID NO. 36] | tttaaaacacCAAGAACTTAGTTTCG AATAAACACACATA | amplification of *prpE* with overhang to *TDH3p* and *PGK1t*, forward |
| JHP059_prpE_ovPG K1t [SEQ ID NO. 37] | gagaaaagaaAAAAATTGATCTATCG ATTTCAATTCAATTC | amplification of *prpE* with overhang to *TDH3p* and *PGK1t*, reverse |
| JHP060_ovTDH3p_S acI_f [SEQ ID NO. 38] | tttaatttgcggccggtacccaattc gcccgagctcACAGTTTATTCCTGGC ATCCACTA | amplification of *TDH3p* with overhang to *prpE*, forward |
| JHP061_ovPGK1t_B amHI_r [SEQ ID NO. 39] | agcgcgcaattaaccctcactaaagg gaacaaggatccAAATAATATCCTTC TCGAAAGC | amplification of *PGK1t* with overhang to *prpE*, reverse |
| JHP075_prpE_d->c_1r [SEQ ID NO. 40] | cgaagatagtgtccatagaagtAGCC AAAGCAACAGCGTAAC | correct deletion at 783 bp in *prpE*, reverse |
| JHP076_prpE_d->c 2f [SEQ ID NO. 41] | agacgttggtggttacgctgTTGCTT TGGCTACTTCTATG | correct deletion at 783 bp in *prpE*, forward |
| JHP079_TDH3p_prp E_ovsfa1up_f [SEQ ID NO. 42] | ataatacttacggggctagttttatt ttctaccacaaataaaaataaagcat cttttaaACAGTTTATTCCTGGCATC CACTA | amplification of *prpE* and *TDH3p* with overhang to *SFA1* upstream region, forward |
| JHP080_prpE_ovsfa1 t_r [SEQ ID NO. 43] | ttgattttcaaagtattccagaaaat ttgagtcatgcttacttagtttaatt aagtactcTTATTCTTCGATAGCTTG TCTGATT | amplification of *prpE* and *TDH3p* with overhang to *SFA1t*, reverse |
| vsp328_pSFA1_fw [SEQ ID NO. 44] | GAATAAGTCCTGGTTCCAGGCAAAAC C | primer binding upstream of *SFA1* locus to check integration, forward |
| MGP146_SFA1down -rev [SEQ ID NO. 45] | GTTAGGAACAGGCGAGGTC | primer binding downstream of *SFA1* locus to check integration, reverse |
| JHP021_prpEseq1 [SEQ ID NO. 46] | CGCTGTTGACAGATGGAGAG | sequencing of *prpE* |
| JHP022_prpEseq2 [SEQ ID NO. 47] | CCCAGACTGTGGTGTTTG | sequencing of *prpE* |
| JHP083_sfa1_seq1_f [SEQ ID NO. 48] | GTGACAACCGAAAGTCAG | sequencing primer binding upstream of *SFA1* locus, forward |
| JHP084_sfa1_seq2_r [SEQ ID NO. 49] | TTTCTTCAGGTCTAACTGATTG | sequencing primer binding downstream of *SFA1* locus, reverse |

| **Deletion of *POX1*** | | |
|---|---|---|
| ABP017*_POX1_Del40_Oligo [SEQ ID NO. 50] | TCACAGAAAAAAAGAAAATATAATAA ATTAGTATTGCGATGTAGAGGTTTCC TGTTTTCCTTCGAACCCTCTGTTTTG CG | Donor-DNA for deletion of *POX1* binding in *POX1p* with overhang to *POX1t*, forward |
| ABP025_POX1_Del40_Oligo_comp [SEQ ID NO. 51] | CGCAAAACAGAGGGTTCGAAGGAAAA CAGGAAACCTCTACATCGCAATACTA ATTTATTATATTTTCTTTTTTTCTGT GA | Donor-DNA for deletion of *POX1* binding in *POX1t* with overhang to *POX1p*, reverse |

| **Assembly via part plasmids and sequencing of pJHV62 and pJHV65** | | |
|---|---|---|
| GDP253 Rv TER_ACP1 GG as 3 [SEQ ID NO. 52] | CGTCTCAGGTCGGTCTCAGGATTTAA ATTCTGTCGAATCTTTCAACTTC | amplification of *Ter* with overhangs for Golden Gate part 3, reverse |
| GDP261 Fw tdTER GG as 3 [SEQ ID NO. 53] | CGTCTCGTCGGTCTCATATGATTGTT AAGCCAATGGTTAGAAACAAC | amplification of *Ter* with overhangs for Golden Gate part 3, forward |
| GDP255 Rv Crt_mtMDH GG as 3 [SEQ ID NO. 54] | CGTCTCAGGTCGGTCTCAGGATTTAT CTGTTCTTGAAACCTTCAATC | amplification of *Crt* with overhangs for Golden Gate part 3, reverse |
| GDP263 Fw Crt GG as 3 [SEQ ID NO. 55] | CGTCTCGTCGGTCTCATATGGAATTG AACAACGTCATC | amplification of *Crt* with overhangs for Golden Gate part 3, forward |
| GDP259 Rv ERG10_mtNC GG as 3 [SEQ ID NO. 56] | CGTCTCAGGTCGGTCTCAGGATTTAA ATCTTTTCAATGACAATAGAGGAAGC | amplification of *ERG10* with overhangs for Golden Gate part 3, reverse |
| GDP262 Fw ERG10 GG as 3 [SEQ ID NO. 57] | CGTCTCGTCGGTCTCATATGTCTCAA AACGTTTACATTG | amplification of *ERG10* with overhangs for Golden Gate part 3, forward |
| GDP265 Rv Hbd GG as 3 part 1 [SEQ ID NO. 58] | CTGGGTTGAAGAAGTGCATACCAATA ACCTTATCTGGCCTCTTAGTAGCAGA AG | amplification of *Hbd* part 1 without *Bsa*I cutsite and with overhangs for Golden Gate part 3, reverse |
| GDP266 Fw Hbd GG as 3 part 2 [SEQ ID NO. 59] | TCTGCTACTAAGAGGCCAGATAAGGT TATTGGTATGCACTTCTTCAAC | amplification of *Hbd* part 2 without *Bsa*I cutsite and with overhangs for Golden Gate part 3, forward |
| GDP267 Rv Hbd GG as 3 part 2 [SEQ ID NO. 60] | CGTCTCAGGTCGGTCTCAGGATTTAC TTAGAGTAATCGTAGAAACCC | amplification of *Hbd* part 2 without *Bsa*I cutsite and with overhangs for Golden Gate part 3, reverse |
| GDP285 Fw Hbd GG as 3 part 1 [SEQ ID NO. 61] | CTGATTCTGTGGATAACCGTAGTCGG TCTCATATGAAGAAGGTTTGTGTTAT TG | amplification of *Hbd* part 1 without *Bsa*I cutsite and with overhangs for Golden Gate part 3, forward |
| hdp073 [SEQ ID NO. 62] | GGTTGCATCACTCCATTG | sequencing primer binding in *TDH3p*, reverse |
| Hdp446 [SEQ ID NO. 63] | TCCTTTACGCTAAAATAATAGTTTAT TT | sequencing primer binding in *PGK1t*, forward |
| JTP302 [SEQ ID NO. 64] | AAGGCATTAAAAGAGGAGCG | sequencing primer binding in *PGK1t*, reverse |
| SZ069seq1EcPPC_ for [SEQ ID NO. 65] | GTTATCCCCTGATTCTGTG | primer binding in SiHV110 backone, forward |
| vsp84_Hbd_ovpPGK1_fw [SEQ ID NO. 66] | TCTACTTTTTACAACAAATATAAAAC AATGAAGAAGGTTTGTGTTATTGG | sequencing primer binding in *Hbd*, forward |
| vsp156 _seq3_tVMA16 [SEQ ID NO. 67] | CATACACATGTATCTCAGATATCTC | sequencing primer binding in *VMA16t*, reverse |
| Vsp157_seq4_ERG10 [SEQ ID NO. 68] | TTTCGTTGTCGAACTTACC | sequencing primer binding in *ERG10*, reverse |
| Vsp160_seq7_hbd [SEQ ID NO. 69] | TATTGCTATTGGTAAGGATCC | sequencing primer binding in *Hbd*, forward |
| Vsp162_seq9_crt [SEQ ID NO. 70] | CACCGAAACCTGGGG | sequencing primer binding in *Crt*, reverse |
| vsp313_seq55_tdTer [SEQ ID NO. 71] | CCGTCTTGAAGCCATTCGG | sequencing primer binding in *Ter*, forward |
| vsp314_seq56_tdTer [SEQ ID NO. 72] | TTACAGACACGACTTCTTGGC | sequencing primer binding in *Ter*, forward |
| vsp315_seq57_pTDH 3 [SEQ ID NO. 73] | CAACTACAGAGAACAGGGGC | sequencing primer binding in *TDH3p*, forward |

**Table 3. Genes used in the study with their source organism and sequence. Sequences codon-optimized (opt) for S. cerevisiae are indicated by prefixes in superscript.**

| **Gene** | **Sequence** | **Source organism** |
|---|---|---|
| | [SEQ ID NO.] | |
| ^{Ppopt}MSAS | ATGCACTCTGCTGCTACTTCTACTTACCCATCTGGTAAGACTTCTCCAGCTCCAG TTGGTACTCCAGGTACTGAATACTCTGAATACGAATTCTCTAACGACGTTGCTGT TGTTGGTATGGCTTGTAGAGTTGCTGGTGGTAACCACAACCCAGAATTGTTGTGG CAATCTTTGTTGTCTCAAAAGTCTGCTATGGGTGAAATCCCACCAATGAGATGGG AACCATACTACAGAAGAGACGCTAGAAACGAAAAGTTCTTGAAGAACACTACTTC TAGAGGTTACTTCTTGGACAGATTGGAAGACTTCGACTGTCAATTCTTCGGTATC TCTCCAAAGGAAGCTGAACAAATGGACCCACAACAAAGAGTTTCTTTGGAAGTTG CTTCTGAAGCTTTGGAAGACGCTGGTATCCCAGCTAAGTCTTTGTCTGGTTCTGA CACTGCTGTTTTCTGGGGTGTTAACTCTGACGACTACTCTAAGTTGGTTTTGGAA GACTTGCCAAACGTTGAAGCTTGGATGGGTATCGGTACTGCTTACTGTGGTGTTC CAAACAGAATCTCTTACCACTTGAACTTGATGGGTCCATCTACTGCTGTTGACGC TGCTTGTGCTTCTTCTTTGGTTGCTATCCACCACGGTGTTCAAGCTATCAGATTG GGTGAATCTAAGGTTGCTATCGTTGGTGGTGTTAACGCTTTGTGTGGTCCAGGTT TGACTAGAGTTTTGGACAAGGCTGGTGCTATCTCTTCTGACGGTTCTTGTAAGTC TTTCGACGACGACGCTCACGGTTACGCTAGAGGTGAAGGTGCTGGTGCTTTGGTT TTGAAGTCTTTGCACAGAGCTTTGTTGGACCACGACAACGTTTTGGCTGTTATCA AGGGTTCTGCTGTTTGTCAAGACGGTAAGACTAACGGTATCATGGCTCCAAACTC TGTTGCTCAACAATTGGCTGCTAACAACGCTTTGTCTGCTGCTAACATCGACCCA CACACTGTTAGATACGTTGAAGCTCACGCTACTTCTACTCCATTGGGTGACCCAA CTGAAATCTCTGCTATCGCTTCTGTTTACGGTGCTGACAGACCAGCTGACGACCC ATGTTACATCGGTTCTATCAAGCCAAACATCGGTCACTTGGAAGCTGGTGCTGGT GTTATGGGTTTCATCAAGGCTGTTTTGGCTATCCAAAAGGGTGTTTTGCCACCAC AAGCTAACTTGACTAAGTTGAACTCTAGAATCGACTGGAAGACTGCTGGTGTTAA GGTTGTTCAAGAAGCTACTCCATGGCCAGAATCTGACCCAATCAGAAGAGCTGGT GTTTGTTCTTACGGTTACGGTGGTACTGTTTCTCACGCTGTTATCGAAGAATTCT CTCCAATCTTGCAACCAGACCCATTGGGTAACGGTGCTGTTTCTGGTCCAGGTTT GTTGTTGTTGTCTGGTCCACAAGAAAAGAGATTGGCTTTGCAAGCTAAGACTTTG AGAGACTGGATGACTGCTGAAGGTAAGGACCACAACTTGTCTGACATCTTGACTA CTTTGGCTACTAGAAGAGACCACCACGACTACAGAGCTGCTTTGGTTGTTGACGA CTACAGAGACGCTGAACAAGTTTTGCAATCTTTGGCTAACGGTGTTGACCACACT TTCACTACTCAATCTAGAGTTTTGGGTTCTGACATCTCTAAGGACGTTGTTTGGG TTTTCTCTGGTCACGGTGCTCAATGGCCAGACATGGGTAAGCAATTGATCCACAA CCCAGTTTTCTTCGCTGCTATCCAACCATTGGACGAATTGATCCAAGCTGAAATC | *Penicillium patulum* |
| | GGTTTGTCTCCAATCGAATTGTTGAGAACTGGTGACTTCGAATCTTCTGACAGAG TTCAAATCTTGACTTACGTTATGCAAATCGGTTTGTCTGCTTTGTTGCAATCTAA CGGTATCACTCCACAAGCTGTTATCGGTCACTCTGTTGGTGAAATCGCTGCTTCC GTTGTTGCTGGTGCTTTGTCTCCAGCTGAAGGTGCTTTGATCGTTACTAGAAGAG CTTTGTTGTACAGACAAGTTATGGGTAAGGGTGGTATGATCTTGGTTAACTTGCC ATCTGCTGAAACTGAAGAAATCTTGGGTTCTAGATCTGACTTGGTTGTTGCTATC GACTCTTCTCCATCTTCTTGTGTTGTTGCTGGTGACAAGGAATTGGTTGCTGAAA CTGCTGAAGCTTTGAAGGCTAGAGGTGTTAAGACTTTCACTGTTAAGTCTGACAT CGCTTTCCACTCTCCAACTTTGAACGGTTTGGTTGACCCATTGAGAGACGTTTTG GCTGAAACTTTGTCTCCAGTTTCTCCAAACGTTAAGTTGTACTCTACTGCTTTGG CTGACCCAAGAGGTCAAGACTTGAGAGACGTTGAATACTGGGCTGGTAACATGGT TAACAGAGTTAGATTGACTTCTGCTGTTAAGGCTGCTGTTGAAGACGGTTACAGA TTGTTCTTGGAAGTTTCTACTCACCCAGTTGTTTCTCACTCTATCAACGAAACTT TGATGGACGCTGGTATGGAAGACTTCGCTGTTATCCCAACTTTGTTGAGAAAGAA GCCAACTGAAAAGCACATCTTGCACTCTATCGCTCAATTGCACTGTAGAGGTGCT GAAGTTAACTGGGCTGCTCAAATGCCAGGTAGATGGGCTACTGGTGTTCCAACTA CTACTTGGATGCACAAGCCAATCTGGAGAAAGATCGAAACTGCTCCATTGCACAC TGGTTTGACTCACGACGTTGAAAAGCACACTTTGTTGGGTCAAAGAATCCCAGTT CCAGGTACTGACACTTACGTTTACACTACTAGATTGGACAACGACACTAAGCCAT TCCCAGGTTCTCACCCATTGCACGGTACTGAAATCGTTCCAGCTGCTGGTTTGAT CAACACTTTCTTGAAGGGTACTGGTGGTCAAATGTTGCAAAACGTTGTTTTGAGA GTTCCAGTTGCTATCAACGCTCCAAGATCTGTTCAAGTTGTTGTTCAACAAGACC AAGTTAAGGTTGTTTCTAGATTGATCCCATCTGAACCATCTCAATTGGACGACGA CGCTTCTTGGGTTACTCACACTACTGCTTACTGGGACAGAAAGGTTGCTGGTTCT GAAGACAGAATCGACTTCGCTGCTGTTAAGTCTAGATTGGTTACTAAGTTGGCTG ACAACTTCTCTATCGACTACTTGGACAAGGTTGGTGTTTCTGCTATGGGTTTCCC ATGGGCTGTTACTGAACACTACAGAAACGACAAGGAAATGTTGGCTAGAGTTGAC GTTAACCCAGCTATCTCTGGTGACGCTCCATTGCCATGGGACTCTTCTTCTTGGG CTCCAGTTTTGGACGCTGCTACTTCTGTTGGTTCTACTATCTTCCCAACTCCAGC TTTGAGAATGCCAGCTCAAATCGAAAGAGTTGAAGTTTTCACTTCTCAAGACCCA CCAAAGATCTCTTGGTTGTACGTTCAAGAAGCTTCTGACTCTGTTCCAACTTCTC ACGTTTCTGTTGTTTCTGAAGCTGGTGAAGTTTTGGCTAAGTTCACTGCTATGAG ATTCTCTGAAATCGAAGGTACTCCAGGTGTTTCTGGTTCTATGGAATCTTTGGTT CACCAAATCGCTTGGCCACCAGCTACTCCAGCTGAAGAACCATTGTCTATCGAAA CTGTTATCTTGGTTTCTCCAGACGCTACTACTAGAGCTTTGTACGCTGCTTCTTT GCCAACTAGAGTTAACTCTTTCCAATTCTCTTCTACTCAAGAATTCTTCTCTAAC GCTTCTTCTTTGCCATTGGAAAAGGGTACTGTTGTTACTTACATCCCAGGTGAAG TTGCTTCTTTGGCTGAAGTTCCAGCTGCTTCTGAATCTTTCACTTGGAACTTGTT GGAATTGATCAAGTTCACTGTTAACGGTTCTTTGCCAATCAAGGTTTTCACTTTG ACTGCTAACATCGGTGAAGGTCAAACTCCAACTGCTTTGGCTCAATCTCCATTGT ACGGTTTGGCTAGAGTTATCGCTTCTGAACACCCAGACTTGGGTACTTTGATCGA CGTTGAAGAACCAGTTATCCCATTGTCTACTATGAGATACATCCAAGGTGCTGAC ATCATCAGAATCAACGACGGTATCGCTAGAACTTCTAGATTCAGATCTTTGCCAA GAAACAAGTTGTTGCCAGCTTCTGAAGGTCCAAGATTGTTGCCAAGACCAGAAGG TACTTACTTGATCACTGGTGGTTTGGGTGTTTTGGGTTTGGAAGTTGCTGACTTC TTGGTTGAAAAGGGTGCTAGAAGATTGTTGTTGATCTCTAGAAGAGCTTTGCCAC CAAGAAGAACTTGGGACCAAGTTTCTGAAGACTTGCAACCAACTATCGCTAAGAT CAGATTGTTGGAATCTAGAGGTGCTTCTGTTCACGTTTTGCCATTGGACATCACT AAGCCAGACGCTGTTGAACAATTGACTACTGCTTTGGACAGATTGTCTTTGCCAT CTGTTCAAGGTGTTGTTCACGCTGCTGGTGTTTTGGACAACGAATTGGTTATGCA AACTACTAGAGACGCTTTCAACAGAGTTTTGGCTCCAAAGATCGCTGGTGCTTTG GCTTTGCACGAAGTTTTCCCACCAAAGTCTGTTGACTTCTTCGTTATGTTCTCTT CTTGTGGTAACTTGGTTGGTTTCACTGGTCAAGCTTCTTACGGTTCTGGTAACGC TTTCTTGGACACTTTGGCTACTCACAGAGCTAGATTGGGTGACGCTGCTGTTTCT TTCCAATGGACTTCTTGGAGAGGTTTGGGTATGGGTGCTTCTACTGACTTCATCA ACGCTGAATTGGAATCTAAGGGTATCACTGACGTTACTAGAGACGAAGCTTTCGC TGCTTGGCAACACTTGGCTAAGTACGACATGGACCACGGTGTTGTTTTGAGATCT AGAGCTTTCGAAGACGGTGAACCAATCCCAGTTTCTATCTTGAACGACATCGCTG TTAGAAGAGTTGGTACTGTTTCTAACACTTCTCCAGCTGCTGCTGGTTCTTCTGA CGCTGTTCCAACTTCTGGTCCAGAATTGAAGGCTTACTTGGACGAAAAGATCAGA GGTTGTGTTGCTAAGGTTTTGCAAATGACTGCTGAAGACGTTGACTCTAAGGCTG CTTTGGCTGACTTGGGTGTTGACTCTGTTATGACTGTTACTTTGAGAAGACAATT GCAACTTACTTTGAAGATCGCTGTTCCACCAACTTTGACTTGGTCTCACCCAACT GTTTCTCACTTGGCTGTTTGGTTCGCTGAAAAGTTGGCTAAGTAA [SEQ ID NO. 9] | |
| *^{opt}npgA* | ATGGTTCAAGACACTTCTTCTGCTTCTACTTCTCCAATCTTGACTAGATGGTACA TCGACACTAGACCATTGACTGCTTCTACTGCTGCTTTGCCATTGTTGGAAACTTT GCAACCAGCTGACCAAATCTCTGTTCAAAAGTACTACCACTTGAAGGACAAGCAC | *Aspergillus nidulans* |
| | ATGTCTTTGGCTTCTAACTTGTTGAAGTACTTGTTCGTTCACAGAAACTGTAGAA TCCCATGGTCTTCTATCGTTATCTCTAGAACTCCAGACCCACACAGAAGACCATG TTACATCCCACCATCTGGTTCTCAAGAAGACTCTTTCAAGGACGGTTACACTGGT ATCAACGTTGAATTTAACGTTTCTCACCAAGCTTCTATGGTTGCTATCGCTGGTA CTGCTTTCACTCCAAACTCTGGTGGTGACTCTAAGTTGAAGCCAGAAGTTGGTAT CGACATCACTTGTGTTAACGAAAGACAAGGTAGAAACGGTGAAGAAAGATCTTTG GAATCTTTGAGACAATACATCGACATCTTCTCTGAAGTTTCTCTACTGCTGAAA TGGCTAACATCAGAAGATTGGACGGTGTTTCTTCTTCTTCTTTGTCTGCTGACAG ATTGGTTGACTACGGTTACAGATTGTTCTACACTTACTGGGCTTTGAAGGAAGCT TACATCAAGATGACTGGTGAAGCTTTGTTGGCTCCATGGTTGAGAGAATTGGAAT TCTCTAACGTTGTTGCTCCAGCTGCTGTTGCTGAATCTGGTGACTCTGCTGGTGA CTTCGGTGAACCATACACTGGTGTTAGAACTACTTTGTACAAGAACTTGGTTGAA GACGTTAGAATCGAAGTTGCTGCTTTGGGTGGTGACTACTTGTTCGCTACTGCTG CTAGAGGTGGTGGTATCGGTGCTTCTTCTAGACCAGGTGGTGGTCCAGACGGTTC TGGTATCAGATCTCAAGACCCATGGAGACCATTCAAGAAGTTGGACATCGAAAGA GACATCCAACCATGTGCTACTGGTGTTTGTAACTGTTTGTCTTAA [SEQ ID NO. 10] | |
| *^{opt}patG* | ATGGCTAAGATCGACGTTCACCACCACTTCTACCCACAAGCTATGAGAGAAGCTT TGGAAAGAGCTGGTGGTGACCCATCTGGTTGGTACATCCCACCATGGACTTTGGA CTTGGACAAGGAAATCTCTAGAGTTTTGAAGGTTCAAACTACTATCTTGTCTGTT ACTGCTCCAGGTCCAGGTATCGAAACTGACCCAGGTAAGGCTGCTGCTTTGGCTA GATTGTGTAACGAAGAAGCTGCTGCTATCAGAGACGCTCACCCATTGCAATACGG TTTCTTCGCTTCTGTTCCATCTTTGTTCGACACTGCTGCTGTTTTGGCTGAAATC GAACACGCTTTCACTAACTTGCACGCTGACGGTGTTACTTTGTACACTAGATACG GTGCTGGTCACTCTTACTTGGGTGACGAAAGATTCAGACCAGTTTGGGCTGAATT GTCTAAGAGAAGAGCTGTTGTTTTCATCCACCCAACTCACGCTGTTGACACTCAA TTGATCAACTCTTGGATGCCACAACCAATGTTCGACTACCCACACGAAACTGGTA GAACTGCTATGGACTTGTTGACTAGAGGTGTTATCAGAGACTACCCAGGTTGTAA GATCATCTTGTCTCACGCTGGTGGTACTTTGCCATACTTGATCCACAGAGCTGCT ACTATGTTGCCATTCATGCCAAGAAACTTGGGTATGTCTAGAGAAGAAATCGTTG AAGCTGCTAGAACTTTGTACTTCGACACTGCTATCTCTGCTAACCCAGTTACTTT GAAGGCTTTGTTGGAATTCGCTAAGCCAGGTCACGTTTTGTTCGGTTCTGACTTC CCAAACGCTCCAAGAGGTGCTATCACTCACTTCACTTCTTTCTTGGAAGGTTACG ACAACATGTCTGAAGAAACTAGAAGATTGGTTGAAAGAGAAGCTGCTTTGGAATT GTTCCCAAGATTGAGAGGTCAATCTACTAGAGCTTGTTTGTAA [SEQ ID NO. 11] | *Aspergillus clavatus* |
| *^{opt}prpE* | ATGTCTTTCTCTGAATTCTACCAAAGATCTATCAACGAACCAGAAGCTTTCTGGG CTGAACAAGCTAGAAGAATCGACTGGAGACAACCATTCACTCAAACTTTGGACCA CTCTAGACCACCATTCGCTAGATGGTTCTGTGGTGGTACTACTAACTTGTGTCAC AACGCTGTTGACAGATGGAGAGACAAGCAACCAGAAGCTTTGGCTTTGATCGCTG TTTCTTCTGAAACTGACGAAGAAAGAACTTTCACTTTCTCTCAATTGCACGACGA AGTTAACATCGTTGCTGCTATGTTGTTGTCTTTGGGTGTTCAAAGAGGTGACAGA GTTTTGGTTTACATGCCAATGATCGCTGAAGCTCAAATCACTTTGTTGGCTTGTG CTAGAATCGGTGCTATCCACTCTGTTGTTTTCGGTGGTTTCGCTTCTCACTCTGT TGCTGCTAGAATCGACGACGCTAGACCAGCTTTGATCGTTTCTGCTGACGCTGGT GCTAGAGGTGGTAAGATCTTGCCATACAAGAAGTTGTTGGACGACGCTATCGCTC AAGCTCAACACCAACCAAAGCACGTTTTGTTGGTTGACAGAGGTTTGGCTAAGAT GGCTTGGGTTGACGGTAGAGACTTGGACTTCGCTACTTTGAGACAACAACACTTG GGTGCTTCTGTTCCAGTTGCTTGGTTGGAATCTAACGAAACTTCTTGTATCTTGT ACACTTCTGGTACTACTGGTAAGCCAAAGGGTGTTCAAAGAGACGTTGGTGGTTA CGCTGTTGCTTTGGCTACTTCTATGGACACTATCTTCGGTGGTAAGGCTGGTGGT GTTTTCTTCTGTGCTTCTGACATCGGTTGGGTTGTTGGTCACTCTTACATCGTTT ACGCTCCATTGTTGGCTGGTATGGCTACTATCGTTTACGAAGGTTTGCCAACTTA CCCAGACTGTGGTGTTTGGTGGAAGATCGTTGAAAAGTACCAAGTTAACAGAATG TTCTCTGCTCCAACTGCTATCAGAGTTTTGAAGAAGTTCCCAACTGCTCAAATCA GAAACCACGACTTGTCTTCTTTGGAAGCTTTGTACTTGGCTGGTGAACCATTGGA CGAACCAACTGCTTCTTGGGTTACTGAAACTTTGGGTGTTCCAGTTATCGACAAC TACTGGCAAACTGAATCTGGTTGGCCAATCATGGCTTTGGCTAGAGCTTTGGACG ACAGACCATCTAGATTGGGTTCTCCAGGTGTTCCAATGTACGGTTACAACGTTCA ATTGTTGAACGAAGTTACTGGTGAACCATGTGGTATCAACGAAAAGGGTATGTTG GTTATCGAAGGTCCATTGCCACCAGGTTGTATCCAAACTATCTGGGGTGACGACG CTAGATTCGTTAAGACTTACTGGTCTTTGTTCAACAGACAAGTTTACGCTACTTT CGACTGGGGTATCAGAGACGCTGAAGGTTACTACTTCATCTTGGGTAGAACTGAC GACGTTATCAACATCGCTGGTCACAGATTGGGTACTAGAGAAATCGAAGAATCTA TCTCTTCTTACCCAAACGTTGCTGAAGTTGCTGTTGTTGGTATCAAGGACGCTTT GAAGGGTCAAGTTGCTGTTGCTTTCGTTATCCCAAAGCAATCTGACACTTTGGCT GACAGAGAAGCTGCTAGAGACGAAGAAAACGCTATCATGGCTTTGGTTGACAACC | *Salmonella typhimurium* |
| | AAATCGGTCACTTCGGTAGACCAGCTCACGTTTGGTTCGTTTCTCAATTGCCAAA GACTAGATCTGGTAAGATGTTGAGAAGAACTATCCAAGCTATCTGTGAAGGTAGA GACCCAGGTGACTTGACTACTATCGACGACCCAGCTTCTTTGCAACAAATCAGAC AAGCTATCGAAGAATAA [SEQ ID NO. 12] | |
| *ERG10* | ATGTCTCAAAACGTTTACATTGTTTCTACTGCTAGAACCCCAATTGGTTCTTTCC AAGGTTCTTTGTCCTCCAAGACCGCTGTTGAATTGGGTGCTGTTGCTTTGAAGGG TGCTTTGGCTAAGGTTCCAGAATTGGATGCTTCCAAGGATTTCGACGAAATTATT TTCGGTAACGTTTTGTCTGCTAACTTGGGTCAAGCTCCAGCTAGACAAGTTGCTT TGGCTGCTGGTTTGTCTAACCACATCGTTGCTTCTACCGTTAACAAGGTCTGTGC TTCCGCTATGAAGGCTATCATTTTGGGTGCTCAATCCATCAAGTGTGGTAACGCT GATGTTGTCGTTGCTGGTGGTTGTGAATCTATGACTAACGCTCCATACTACATGC CAGCTGCTAGAGCTGGTGCTAAGTTCGGTCAAACTGTTTTGGTTGATGGTGTCGA AAGAGATGGTTTGAACGATGCTTACGATGGTTTGGCTATGGGTGTTCACGCTGAA AAGTGTGCTAGAGATTGGGATATTACTAGAGAACAACAAGACAACTTCGCTATCG AATCCTACCAAAAGTCTCAAAAGTCTCAAAAGGAAGGTAAGTTCGACAACGAAAT TGTTCCAGTTACCATTAAGGGTTTCAGAGGTAAGCCAGATACTCAAGTCACCAAG GACGAAGAACCAGCTAGATTGCACGTTGAAAAGTTGAGATCTGCTAGAACTGTTT TCCAAAAGGAAAACGGTACTGTTACTGCTGCTAACGCTTCTCCAATCAACGATGG TGCTGCTGCTGTCATCTTGGTTTCCGAAAAGGTTTTGAAGGAAAAGAACTTGAAG CCATTGGCTATTATCAAGGGTTGGGGTGAAGCTGCTCACCAACCAGCTGATTTCA CCTGGGCTCCATCTTTGGCTGTTCCAAAGGCTTTGAAGCACGCTGGTATCGAAGA CATCAACTCTGTTGATTACTTCGAATTCAACGAAGCTTTCTCTGTTGTCGGTTTG GTTAACACTAAGATTTTGAAGTTGGACCCATCTAAGGTTAACGTTTACGGTGGTG CTGTTGCTTTGGGTCACCCATTGGGTTGTTCTGGTGCTAGAGTTGTTGTTACCTT GTTGTCCATCTTGCAACAAGAAGGTGGTAAGATCGGTGTTGCTGCTATTTGTAAC GGTGGTGGTGGTGCTTCCTCTATTGTCATTGAAAAGATTTAA [SEQ ID NO. 13] | *Saccharomyces cerevisiae* |
| *^{opt}Hbd* | ATGAAGAAGGTTTGTGTTATTGGTGCTGGTACTATGGGTTCTGGTATTGCTCAAG CTTTCGCTGCTAAGGGTTTCGAAGTTGTTTTGAGAGATATTAAGGATGAATTCGT TGATAGAGGTTTGGATTTCATCAACAAGAACTTGTCTAAGTTGGTTAAGAAGGGT AAGATTGAAGAAGCTACTAAGGTTGAAATCTTGACTAGAATTTCCGGTACCGTTG ACTTGAACATGGCTGCTGATTGTGATTTGGTTATTGAAGCTGCTGTTGAAAGAAT GGATATTAAGAAGCAAATTTTCGCTGACTTGGACAACATTTGTAAGCCAGAAACC ATTTTGGCTTCTAACACCTCTTCTTTGTCTATTACCGAAGTTGCTTCTGCTACTA AGAGGCCAGATAAGGTTATTGGTATGCACTTCTTCAACCCAGCTCCAGTTATGAA GTTGGTTGAAGTTATTAGAGGTATTGCTACCTCTCAAGAAACTTTCGATGCTGTT AAGGAAACCTCTATTGCTATTGGTAAGGATCCAGTTGAAGTTGCTGAAGCTCCAG GTTTCGTTGTTAACAGAATTTTGATTCCAATGATTAACGAAGCTGTTGGTATTTT GGCTGAAGGTATTGCTTCTGTTGAAGACATTGATAAGGCTATGAAGTTGGGTGCT AACCACCCAATGGGTCCATTGGAATTGGGTGATTTCATTGGTTTGGATATTTGTT TGGCTATTATGGATGTTTTGTACTCTGAAACTGGTGATTCTAAGTACAGACCACA CACCTTGTTGAAGAAGTACGTTAGAGCTGGTTGGTTGGGTAGAAAGTCTGGTAAG GGTTTCTACGATTACTCTAAGTAA [SEQ ID NO. 14] | *Clostridium acetobutylicum* |
| *^{opt}Crt* | ATGGAATTGAACAACGTCATCTTGGAAAAGGAAGGTAAGGTTGCTGTTGTTACCA TTAACAGACCAAAGGCTTTGAACGCTTTGAACTCTGATACCTTGAAGGAAATGGA TTACGTTATTGGTGAAATTGAAAACGATTCTGAAGTTTTGGCTGTTATTTTGACT GGTGCTGGTGAAAAGTCTTTCGTTGCTGGTGCTGATATTTCTGAAATGAAGGAAA TGAACACCATTGAAGGTAGAAAGTTCGGTATTTTGGGTAACAAGGTTTTCAGAAG ATTGGAATTGTTGGAAAAGCCAGTTATTGCTGCTGTTAACGGTTTCGCTTTGGGT GGTGGTTGTGAAATTGCTATGTCTTGTGATATTAGAATTGCTTCTTCTAACGCTA GATTCGGTCAACCAGAAGTTGGTTTGGGTATTACCCCAGGTTTCGGTGGTACCCA AAGATTGTCTAGATTGGTTGGTATGGGTATGGCTAAGCAATTGATTTTCACTGCT CAAAACATTAAGGCTGATGAAGCTTTGAGAATCGGTTTGGTTAACAAGGTTGTTG AACCATCTGAATTGATGAACACCGCTAAGGAAATTGCTAACAAGATTGTTTCTAA CGCTCCAGTTGCTGTTAAGTTGTCTAAGCAAGCTATTAACAGAGGTATGCAATGT GATATTGATACTGCTTTGGCTTTCGAATCTGAAGCTTTCGGTGAATGTTTCTCTA CCGAAGATCAAAAGGATGCTATGACCGCTTTCATTGAAAAGAGAAAGATTGAAGG TTTCAAGAACAGATAA [SEQ ID NO. 15] | *Clostridium acetobutylicum* |
| *^{opt}Ter* | ATGATTGTTAAGCCAATGGTTAGAAACAACATTTGTTTGAACGCTCACCCACAAG GTTGTAAGAAGGGTGTTGAAGATCAAATTGAATACACCAAGAAGAGAATTACCGC TGAAGTTAAGGCTGGTGCTAAGGCTCCAAAGAACGTTTTGGTTTTGGGTTGTTCT AACGGTTACGGTTTGGCTTCTAGAATTACCGCTGCTTTCGGTTACGGTGCTGCTA | *Treponema denticola* |
| | CTATCGGTGTTTCCTTCGAAAAGGCTGGTTCTGAAACCAAGTACGGTACCCCAGG TTGGTACAACAACTTGGCTTTCGACGAAGCTGCTAAGAGAGAAGGTTTGTACTCC GTTACTATTGACGGTGACGCTTTCTCCGATGAAATCAAGGCTCAAGTTATCGAAG AAGCTAAGAAGAAGGGTATTAAGTTCGATTTGATTGTTTACTCTTTGGCTTCTCC AGTTAGAACCGATCCAGACACCGGTATTATGCACAAGTCCGTCTTGAAGCCATTC GGTAAGACCTTCACCGGTAAGACCGTCGATCCATTCACCGGTGAATTGAAGGAAA TCTCCGCTGAACCAGCTAACGATGAAGAAGCTGCTGCTACCGTTAAGGTTATGGG TGGTGAAGACTGGGAAAGATGGATTAAGCAATTGTCTAAGGAAGGTTTGTTGGAA GAAGGTTGTATTACCTTGGCTTACTCCTACATCGGTCCAGAAGCTACTCAAGCTT TGTACAGAAAGGGTACCATTGGTAAGGCTAAGGAACACTTGGAAGCTACTGCTCA CAGATTGAACAAGGAAAACCCATCTATTAGAGCTTTCGTTTCTGTTAACAAGGGT TTGGTTACCAGAGCTTCTGCTGTTATCCCAGTTATTCCATTGTACTTGGCTTCCT TGTTCAAGGTTATGAAGGAAAAGGGTAACCACGAAGGTTGTATCGAACAAATTAC CAGATTGTACGCTGAAAGATTGTACAGAAAGGACGGTACCATCCCAGTCGATGAA GAAAACAGAATCAGAATCGACGACTGGGAATTGGAAGAAGACGTTCAAAAGGCTG TTTCTGCTTTGATGGAAAAGGTTACCGGTGAAAACGCTGAATCTTTGACCGACTT GGCTGGTTACAGACACGACTTCTTGGCTTCTAACGGTTTCGATGTTGAAGGTATC AACTACGAAGCTGAAGTTGAAAGATTCGACAGAATTTAA [SEQ ID NO. 16] | |

### 1.3 Cell cultivation

Fermentations for propionate consumption or 3-alkylphenol production were performed as described previously (Hitschler and Boles, 2019). Precultures were cultivated in 150 mL YPD supplemented with corresponding antibiotics in 500 mL Erlenmeyer shake flasks at 180 rpm 30°C. To prevent possible toxic effects of the weak acid propionate, the medium was always buffered with 100 m potassium phosphate buffer (KPᵢ) at pH 6.5. Precultures were harvested in exponential phase and for main culture 25 mL YPD buffered with KPᵢ at pH 6.5 and with respective antibiotics in 100 mL Erlenmeyer shake flasks were inoculated to an optical density (OD_{600 nm}) of 5 in high OD-fermentations and shaken at 180 rpm 30°C for 144 h. When propionate consumption or biotransformation to 3-ethylphenol was followed, 10 mM propionate was added to main cultures. Cultures were incubated in a 30°C shaking waterbath (Memmert, Germany) under a hood or in a 30°C container equipped with a shaker to avoid 3-alkylphenol inhalation. Samples for cell density determination and HPLC analysis were taken every 0, 6, 24, 48, 72 and 144 h.

### 1.4 Growth and metabolite analysis

Cell growth was followed in the spectrophotometer Ultrospec 2100 pro (GE Healthcare, USA) at an optical density of 600 nm. Culture supernatants for HPLC analysis of 3-alkylphenol formation were obtained as described previously (Hitschler and Boles, 2019) centrifuging at 10,000 rpm for 2 min, 400 µL of supernatants were treated with 100 µL acetonitrile, centrifuged again and stored at -20°C until analysis. Samples were analysed in an HPLC (Dionex) using an Agilent Zorbax SB-C8 column (4.6 x 150 mm, 3.5 µm) at 40°C. 3-methylphenol and 3-ethylphenol were separated by the following gradient of solvent A (0.1% (v/v) formic acid in ddH₂O) and solvent B (0.1% (v/v) formic acid in acetonitrile) at a flow rate of 1 mL/min: 5 min 15% B, 20 min linear gradient to 40% B, 1 min linear gradient to 100% B, 4 min 100% B, 1 min linear gradient to 15% B, 4 min 15% B. For 3-propylphenol the gradient stayed at 40% B for 5 min before it switched to 100% B to prolong the separation before the washing step. The 3-alkylphenols were detected at 270 nm in an UV detector (Dionex UltiMate 3000 Variable Wavelength Detector). For quantification and calibration, 3-ethylphenol and 3-propylphenol standards were prepared in ddH₂O from 3-ethylphenol purchased from Sigma-Aldrich (210-627-3) and 3-propylphenol purchased from Alfa Aesar (621-27-2).

For the analysis of propionate 50 µL 50% (w/v) sulfosalicylic acid was added to 450 µL culture supernatant. Samples were analysed in the HPLC equipped with the ion exchange column HyperREZ XP Carbohydrate H+ (7.7 × 300 mm, 8 µm) and a refractive index detector. The metabolites were separated with 5 mM sulfuric acid as liquid phase at a flow rate of 0.6 mL/min and 65°C. For quantification, propionate standards of different concentrations were prepared in ddH₂O from propionic acid purchased from Carl Roth (6026.2).

### REFERENCES

Baader S, Podsiadly PE, Cole-Hamilton DJ, Goossen LJ. Synthesis of tsetse fly attractants from a cashew nut shell extract by isomerising metathesis. Green Chem. 2014;16:4885. doi: 10.1039/c4gc01269k
Bursell E, Gough AJE, Beevor PS, Cork A, Hall DR, Vale GA. Identification of components of cattle urine attractive to tsetse flies, Glossina spp. (Diptera: Glossinidae). Cambridge Univ. Press 1988; 78: 281-291. doi:10.1017/s0007485300013043
Carbone A, Zinovyev A, Képès F. Codon adaptation index as a measure of dominating codon bias. Bioinformatics 2003; 19(16):2005-2015. doi: 10.1093/bioinformatics/btg272
Coghlan A, Wolfe KH. Relationship of codon bias to mRNA concentration and protein length in Saccharomyces cerevisiae. Yeast 2000;16:1131-1145. doi: 10.1002/1097-0061(20000915)16:12<1131::AID-YEA609>3.0.CO;2-F
Dimroth P, Ringelmann E, Lynen F. 6-methylsalicylic acid synthetase from Penicillium patulum. Eur. J. Biochem. 1976; 68:591-596. doi: 10.1111/j.1432-1033.1976.tb10847.x
Dmochowska A, Dignard D, Maleszka R, Thomas DY. Structure and transcriptional control of the Saccharomyces cerevisiae POX1 gene encoding acyl-coenzyme A oxidase. Gene 1990; 88:247-252. doi: 10.1016/0378-1119(90)90038-S
Entian KD, Kötter P. Yeast genetic strain and plasmid collections. Method Microbiol. 2007; 36:629-666. doi: 10.1016/S0580-9517(06)36025-4
   Generoso WC, Gottardi M, Oreb M, Boles E. Simplified CRISPR-Cas genome editing for Saccharomyces cerevisiae. J. Microbiol. Methods 2016; 127: 203-205. doi: 10.1016/j.mimet.2016.06.020
Gibson DG, Young L, Chuang RY, Venter JC, Hutchison III CA, Smith HO. Enzymatic assembly of DNA molecules up to several hundred kilobases. Nat. Methods 2009; 6:343-345. doi: 10.1038/nmeth.1318
Gietz RD, Schiestl RH. 2007. Quick and easy yeast transformation using the LiAc/ss carrier DNA/PEG method. Nat. Protoc. 2007; 2:35-37. doi: 10.1038/nprot.2007.14
Graybill ER, Rouhier MF, Kirby CE, Hawes JW. Functional comparison of citrate synthase isoforms from S. cerevisiae. Arch Biochem Biophys. 2007 Sep 1;465(1):26-37. Epub 2007 May 21. doi: 10.1016/j.abb.2007.04.039
Grote A, Hiller K, Scheer M, Münch R, Nortemann B, Hempel DC, Jahn D. JCat : a novel tool to adapt codon usage of a target gene to its potential expression host. Nucleic Acids Res. 2005; 33:526-531. doi: 10.1093/nar/gki376.
Guthrie and Fink. Guide to Yeast Genetics and Molecular and Cell Biology, Parts A, B, C, Methods in Enzymology 194, 350, 351; Elsevier Academic Press 1990/2002
Hitschler J, Boles E. De novo production of aromatic m-cresol in Saccharomyces cerevisiae mediated by heterologous polyketide synthases combined with a 6-methylsalicylic acid decarboxylase. Metab. Eng. Commun. 2019; 9: e00093. doi: 10.1016/j.mec.2019.e00093
Krink-Koutsoubelis N, Loechner AC, Lechner A, Link H, Denby CM, Vögeli B, Erb TJ, Yuzawa S, Jakociunas T, Katz L, Jensen MK, Sourjik V, Keasling JD. Engineered production of short-chain acyl-coenzyme A esters in Saccharomyces cerevisiae. ACS Synth. Biol. 2018; 7(4):1105-1115. doi: 10.1021/acssynbio.7b00466
Lee M, DeLoache WC, Cervantes B, Dueber JE. A highly characterized yeast toolkit for modular, multipart assembly. ACS Synth. Biol. 2015; 4(9):975-986. doi: 10.1021/sb500366v
Light RJ, Vogel G. 6-Methylsalicylic acid (2,6-cresotic acid) decarboxylase. Methods Enzymol. 1975;43:530-40. doi: 10.1016/0076-6879(75)43115-9 Get
Luttik MA, Kötter P, Salomons FA, van der Klei IJ, van Dijken JP, Pronk JT. The Saccharomyces cerevisiae ICL2 gene encodes a mitochondrial 2-methylisocitrate lyase involved in propionyl-coenzyme A metabolism. J Bacteriol. 2000 Dec;182(24):7007-13. doi: 10.1128/jb.182.24.7007-7013.2000
Mutka SC, Bondi SM, Carney JR, Da Silva NA, Kealey JT. Metabolic pathway engineering for complex polyketide biosynthesis in Saccharomyces cerevisiae. FEMS Yeast Res. 2006 Jan;6(1):40-7. doi: 10.1111/j.1567-1356.2005.00001.x
Parascandolo JS, Havemann J, Potter HK, Huang F, Riva E, Connolly J, Wilkening I, Song L, Leadlay PF, Tosin M. Insights into 6-Methylsalicylic Acid Bio-assembly by Using Chemical Probes. Angew Chem Weinheim Bergstr Ger. 2016 Mar 1;128(10):3524-3528. Epub 2016 Feb 2. doi: 10.1002/anie.201509038
Pronk JT, van der Linden-Beuman A, Verduyn C, Scheffers WA, van Dijken JP. Propionate metabolism in Saccharomyces cerevisiae: implications for the metabolon hypothesis. Microbiology. 1994 Apr;140 (Pt 4):717-22. doi: 10.1099/00221287-140-4-717
Reifenrath M, Boles E. Engineering of hydroxymandelate synthases and the aromatic amino acid pathway enables de novo biosynthesis of mandelic and 4-hydroxymandelic acid with Saccharomyces cerevisiae. Metab. Eng. 2018; 45:246-254. doi: 10.1016/j.ymben.2018.01.001
Richardson MT, Pohl NL. Tolerance and specificity of recombinant 6-methylsalicylic acid synthase. Metab. Eng. 1999; 1:180-187. doi: 10.1006/mben.1999.0113
Schadeweg V, Boles E. Increasing n-butanol production with Saccharomyces cerevisiae by optimizing acetyl-CoA synthesis, NADH levels and trans-2-enoyl-CoA reductase expression. Biotechnol. Biofuels. 2016a; 9: 1-11. doi: 10.1186/s13068-016-0673-0
Schadeweg V, Boles E. n-Butanol production in Saccharomyces cerevisiae is limited by the availability of coenzyme A and cytosolic acetyl-CoA. Biotechnol. Biofuels. 2016b; 9: 44. doi: 10.1186/s13068-016-0456-7
Sharp PM, Li WH. The codon adaptation index - a measure of directional synonymous codon usage bias, and its potential applications. Nucleic Acids Res. 1987; 15(3):1281-1295. doi: 10.1093/nar/15.3.1281
Shockey JM, Fulda MS, Browse J. Arabidopsis contains a large superfamily of acyl-activating enzymes. Phylogenetic and biochemical analysis reveals a new class of acyl-coenzyme A synthetases. Mol. Biol. Evol. 2003; 132(2):1065-1076. doi: 10.1104/pp.103.020552
Snini SP, Tadrist S, Laffitte J, Jamin EL, Oswald IP, Puel O. The gene PatG involved in the biosynthesis pathway of patulin, a food-borne mycotoxin, encodes a 6-methylsalicylic acid decarboxylase. Int J Food Microbiol. 2014;171:77-83. doi: 10.1016/j.ijfoodmicro.2013.11.020. Epub 2013 Nov 27.
Taxis C, Knop M. System of centromeric, episomal, and integrative vectors based on drug resistance markers for Saccharomyces cerevisiae. Biotechniques 2006; 40(1):73-77. doi: 10.2144/000112040
Turner JE, Greville K, Murphy EC, Hooks MA. Characterization of Arabidopsis fluoroacetate-resistant mutants reveals the principle mechanisms of acetate activation for entry into the glyoxylate cycle. J. Biol. Chem. 2005; 280(4):2780-2787. doi: 10.1074/jbc.M407291200
Van den Berg MA, de Jong-Gubbels P, Kortland CJ, van Dijken JP, Pronk JT, Steensma HY. The two acetyl-coenzyme A synthetases of Saccharomyces cerevisiae differ with respect to kinetic properties and transcriptional regulation. J. Biol. Chem. 1996; 271: 28953-28959. doi: 10.1074/jbc.271.46.28953
Wattanachaisaereekul S, Lantz AE, Nielsen ML, Andrésson OS, Nielsen J. Optimization of heterologous production of the polyketide 6-MSA in Saccharomyces cerevisiae. Biotechnol Bioeng. 2007 Jul 1;97(4):893-900.
Wiedemann B, Boles E. Codon-optimized bacterial genes improve L-arabinose fermentation in recombinant Saccharomyces cerevisiae. Appl. Environ. Microbiol. 2008; 74(7):2043-2050. doi: 10.1128/AEM.02395-07

## Claims

1. A host cell containing
(i) a heterologous nucleic acid molecule coding for a 6-methylsalicylic acid synthase (MSAS),
(ii) a heterologous nucleic acid molecule coding for a phosphopantetheinyl transferase (PPTase) which is able to transfer a phosphopantethein group to MSAS,
(iii) a heterologous nucleic acid molecule coding for a 6-methylsalicylic acid (6-MSA) decarboxylase, and
(iv) source for increased intracellular synthesis of propionyl-CoA and/or butyryl-CoA, and,
optionally, (v) a block in the degradation pathway of propionyl-CoA and/or butyryl-CoA
wherein the host cell is a fungus cell.

2. The host cell of claim 1, wherein the fungus cell is a yeast cell, such as *Saccharomyces* species, *Kluyveromyces* sp., *Ogataea* sp., *Pichia* sp. *Rhodotorula* sp. or *Yarrowia* sp., preferably *S. cerevisiae.*

3. The host cell according to claim 1 or 2, wherein the nucleic acid molecule coding for MSAS (i) is the *MSAS* gene of *Penicillium patulum.*

4. The host cell according to any one of claims 1 to 3, wherein the nucleic acid molecule coding for a phosphopantetheinyl transferase (PPTase) (ii) is the *npgA* gene from *Aspergillus nidulans.*

5. The host cell according to any one of the preceding claims, wherein the nucleic acid molecule coding for 6-MSA decarboxylase (ii) is the 6-MSA decarboxylase gene *patG* of *Aspergillus clavatus.*

6. The host cell according to any one of the preceding claims, wherein the source for increased intracellular synthesis of propionyl-CoA (iv) is provided by culturing the cell in a culture medium containing propionate and/or propionic acid, preferably with the host cell containing a heterologous nucleic acid molecule coding for a propionyl-CoA synthase, and/or wherein the source for increased intracellular synthesis of butyryl-CoA (iv) is provided by expressing nucleic acid molecules coding for a reverse β-oxidation pathway, comprising enzymes with thiolase activity, 3-hydroxybutyryl-CoA dehydrogenase activity, crotonase activity and trans-2-enoyl-CoA reductase activity, in the cell.

7. The host cell according to any one of the preceding claims, wherein the heterologous nucleic acid molecules are each linked to a promoter, such as such as p*PGK1,* p*HXT7*^{-1- -392}, p*FBA1,* p*PDC1,* p*PYK1,* p*TPI1,* p*ADH1, pTDH3,* p*CCW12* and p*ENO2* promoters, or other strong promoters,
and/or wherein the host cell contains the heterologous nucleic acid molecules, preferably linked to promoters, integrated into the genome.

8. The host cell according to any one of the preceding claims, wherein at least two of the nucleic acid molecules are codon-optimised for use in a host cell.

9. The host cell according to any one of the preceding claims, wherein
the nucleic acid molecule coding for MSAS (i) comprises a nucleic acid sequence coding for an amino acid sequence of SEQ ID NO. 1 or an amino acid sequence which is at least 90% or 95% identical to the amino acid sequence of SEQ ID NO. 1,
the nucleic acid molecule coding for PPTase (ii) comprises a nucleic acid sequence coding for an amino acid sequence of SEQ ID NO. 2 or an amino acid sequence which is at least 90% or 95% identical to the amino acid sequence of SEQ ID NO. 2,
the nucleic acid molecule coding for 6-MSA decarboxylase (iii) comprises a nucleic acid sequence coding for an amino acid sequence of SEQ ID NO. 3 or an amino acid sequence which is at least 90% or 95% identical to the amino acid sequence of SEQ ID NO. 3,
the nucleic acid molecule coding for propionyl-CoA synthase comprises a nucleic acid sequence coding for an amino acid sequence of SEQ ID NO. 4 or an amino acid sequence which is at least 90% or 95% identical to the amino acid sequence of SEQ ID NO. 4, and/or
the nucleic acid molecules coding for a reverse β-oxidation pathway comprises nucleic acid sequences coding for amino acid sequences of SEQ ID NOs. 5-8 or amino acid sequences which are at least 90% or 95% identical to the amino acid sequences of SEQ ID NOs. 5-8.

10. The host cell according to any one of the preceding claims, wherein the block in the degradation pathway of propionyl-CoA (v) is provided by mutation(s) in or, preferably deletion(s) of the gene(s) coding for 2-methylcitrate synthase(s), preferably *CIT3* and more preferably *CIT3* and *CIT2,*
and/or wherein the block in the degradation pathway of butyryl-CoA (v) is provided by mutation(s) in or, preferably deletion of the *POX1* gene coding for fatty acyl-Coenzyme A oxidase.

11. Use of the host cell according to any one of claims 1 to 10 for the production of 3-alkyl phenols, preferably, 3-ethylphenol (3-EP) and/or 3-propylphenol (3-PP), optionally together with 3-methyl phenol (3-MP).

12. A method for the production of 3-alkylphenols, preferably of 3-ethylphenol (3-EP), optionally together with 3-methylphenol (3-MP),
comprising the steps of
providing a host cell according to any one of claims 1 to 10,
culturing the host cell by adding carbon source(s), preferably sugar(s) to the culture medium,
or culturing the host cell by adding carbon source(s) and propionate to the culture medium,
optionally, obtaining the 3-alkylphenols.

13. A method for the production of 3-alkyl phenols, preferably 3-propylphenol (3-PP), optionally together with 3-methylphenol (3-MP),
comprising the steps of
providing a host cell according to any one of claims 1 to 10,
culturing the host cell by adding carbon source(s), preferably sugar(s) to the culture medium,
or culturing the host cell by adding carbon source(s) and butyrate to the culture medium,
optionally, obtaining the 3-alkylphenols.

14. A method for the production of 3-alkyl phenols, preferably of 3-ethylphenol (3-EP) and 3-propylphenol (3-PP), optionally together with 3-methylphenol (3-MP)
comprising the steps of
providing a host cell according to any one of claims 1 to 10,
culturing the host cell by adding carbon source(s), preferably sugar(s) to the culture medium,
or culturing the host cell by adding carbon source(s) and propionate and, optionally, butyrate to the culture medium,
optionally, obtaining the 3-alkylphenols.

15. A composition comprising 3-alkyl phenols, preferably 3-ethylphenol (3-EP) and/or 3-propylphenol (3-PP), optionally in combination with 3-methylphenol (3-MP),
preferably obtained by a method of any one of claims 12 to 14.

16. Use of at least one of the following:
(a) the host cell according to any one of claims 1 to 10,
(b) the host cell extracts and/or the host cell culture and/or the host cell culture supernatant obtained in a method of any one of claims 12 to 14,
(c) the composition of claim 15,
as tsetse fly attractant,
such as in a flytrap.

17. A tsetse fly attractant composition, comprising
(a) the host cell according to any one of claims 1 to 10,
(b) the composition of claim 15, and/or
(c) the host cell extracts and/or the host cell culture and/or the host cell culture supernatant obtained in a method of any one of claims 12 to 14,
(d) optionally, excipient(s) and/or carrier.


SEQUENCE LISTING

<110>  Goethe-Universität Frankfurt am Main

<120>  Biotechnological production of alkylphenols and their uses

<130>  U30945EP

<160>  73

<170>  PatentIn version 3.5

<210>  1
<211>  1774
<212>  PRT
<213>  Penicillium patulum

<400>  1

Met His Ser Ala Ala Thr Ser Thr Tyr Pro Ser Gly Lys Thr Ser Pro
1               5                   10                  15

Ala Pro Val Gly Thr Pro Gly Thr Glu Tyr Ser Glu Tyr Glu Phe Ser
            20                  25                  30

Asn Asp Val Ala Val Val Gly Met Ala Cys Arg Val Ala Gly Gly Asn
        35                  40                  45

His Asn Pro Glu Leu Leu Trp Gln Ser Leu Leu Ser Gln Lys Ser Ala
    50                  55                  60

Met Gly Glu Ile Pro Pro Met Arg Trp Glu Pro Tyr Tyr Arg Arg Asp
65                  70                  75                  80

Ala Arg Asn Glu Lys Phe Leu Lys Asn Thr Thr Ser Arg Gly Tyr Phe
                85                  90                  95

Leu Asp Arg Leu Glu Asp Phe Asp Cys Gln Phe Phe Gly Ile Ser Pro
            100                 105                 110

Lys Glu Ala Glu Gln Met Asp Pro Gln Gln Arg Val Ser Leu Glu Val
        115                 120                 125

Ala Ser Glu Ala Leu Glu Asp Ala Gly Ile Pro Ala Lys Ser Leu Ser
    130                 135                 140

Gly Ser Asp Thr Ala Val Phe Trp Gly Val Asn Ser Asp Asp Tyr Ser
145                 150                 155                 160

Lys Leu Val Leu Glu Asp Leu Pro Asn Val Glu Ala Trp Met Gly Ile
                165                 170                 175

Gly Thr Ala Tyr Cys Gly Val Pro Asn Arg Ile Ser Tyr His Leu Asn
        180                 185                 190

Leu Met Gly Pro Ser Thr Ala Val Asp Ala Ala Cys Ala Ser Ser Leu
    195                 200                 205

Val Ala Ile His His Gly Val Gln Ala Ile Arg Leu Gly Glu Ser Lys
    210                 215                 220

Val Ala Ile Val Gly Gly Val Asn Ala Leu Cys Gly Pro Gly Leu Thr
225                 230                 235                 240

Arg Val Leu Asp Lys Ala Gly Ala Ile Ser Ser Asp Gly Ser Cys Lys
                245                 250                 255

Ser Phe Asp Asp Asp Ala His Gly Tyr Ala Arg Gly Glu Gly Ala Gly
            260                 265                 270

Ala Leu Val Leu Lys Ser Leu His Arg Ala Leu Leu Asp His Asp Asn
        275                 280                 285

Val Leu Ala Val Ile Lys Gly Ser Ala Val Cys Gln Asp Gly Lys Thr
    290                 295                 300

Asn Gly Ile Met Ala Pro Asn Ser Val Ala Gln Gln Leu Ala Ala Asn
305                 310                 315                 320

Asn Ala Leu Ser Ala Ala Asn Ile Asp Pro His Thr Val Arg Tyr Val
                325                 330                 335

Glu Ala His Ala Thr Ser Thr Pro Leu Gly Asp Pro Thr Glu Ile Ser
            340                 345                 350

Ala Ile Ala Ser Val Tyr Gly Ala Asp Arg Pro Ala Asp Asp Pro Cys
        355                 360                 365

Tyr Ile Gly Ser Ile Lys Pro Asn Ile Gly His Leu Glu Ala Gly Ala
    370                 375                 380

Gly Val Met Gly Phe Ile Lys Ala Val Leu Ala Ile Gln Lys Gly Val
385                 390                 395                 400

Leu Pro Pro Gln Ala Asn Leu Thr Lys Leu Asn Ser Arg Ile Asp Trp
                405                 410                 415

Lys Thr Ala Gly Val Lys Val Val Gln Glu Ala Thr Pro Trp Pro Glu
            420                 425                 430

Ser Asp Pro Ile Arg Arg Ala Gly Val Cys Ser Tyr Gly Tyr Gly Gly
        435                 440                 445

Thr Val Ser His Ala Val Ile Glu Glu Phe Ser Pro Ile Leu Gln Pro
    450                 455                 460

Asp Pro Leu Gly Asn Gly Ala Val Ser Gly Pro Gly Leu Leu Leu Leu
465                 470                 475                 480

Ser Gly Pro Gln Glu Lys Arg Leu Ala Leu Gln Ala Lys Thr Leu Arg
                485                 490                 495

Asp Trp Met Thr Ala Glu Gly Lys Asp His Asn Leu Ser Asp Ile Leu
            500                 505                 510

Thr Thr Leu Ala Thr Arg Arg Asp His His Asp Tyr Arg Ala Ala Leu
        515                 520                 525

Val Val Asp Asp Tyr Arg Asp Ala Glu Gln Val Leu Gln Ser Leu Ala
    530                 535                 540

Asn Gly Val Asp His Thr Phe Thr Thr Gln Ser Arg Val Leu Gly Ser
545                 550                 555                 560

Asp Ile Ser Lys Asp Val Val Trp Val Phe Ser Gly His Gly Ala Gln
                565                 570                 575

Trp Pro Asp Met Gly Lys Gln Leu Ile His Asn Pro Val Phe Phe Ala
            580                 585                 590

Ala Ile Gln Pro Leu Asp Glu Leu Ile Gln Ala Glu Ile Gly Leu Ser
        595                 600                 605

Pro Ile Glu Leu Leu Arg Thr Gly Asp Phe Glu Ser Ser Asp Arg Val
    610                 615                 620

Gln Ile Leu Thr Tyr Val Met Gln Ile Gly Leu Ser Ala Leu Leu Gln
625                 630                 635                 640

Ser Asn Gly Ile Thr Pro Gln Ala Val Ile Gly His Ser Val Gly Glu
                645                 650                 655

Ile Ala Ala Ser Val Val Ala Gly Ala Leu Ser Pro Ala Glu Gly Ala
            660                 665                 670

Leu Ile Val Thr Arg Arg Ala Leu Leu Tyr Arg Gln Val Met Gly Lys
        675                 680                 685

Gly Gly Met Ile Leu Val Asn Leu Pro Ser Ala Glu Thr Glu Glu Ile
    690                 695                 700

Leu Gly Ser Arg Ser Asp Leu Val Val Ala Ile Asp Ser Ser Pro Ser
705                 710                 715                 720

Ser Cys Val Val Ala Gly Asp Lys Glu Leu Val Ala Glu Thr Ala Glu
                725                 730                 735

Ala Leu Lys Ala Arg Gly Val Lys Thr Phe Thr Val Lys Ser Asp Ile
            740                 745                 750

Ala Phe His Ser Pro Thr Leu Asn Gly Leu Val Asp Pro Leu Arg Asp
        755                 760                 765

Val Leu Ala Glu Thr Leu Ser Pro Val Ser Pro Asn Val Lys Leu Tyr
    770                 775                 780

Ser Thr Ala Leu Ala Asp Pro Arg Gly Gln Asp Leu Arg Asp Val Glu
785                 790                 795                 800

Tyr Trp Ala Gly Asn Met Val Asn Arg Val Arg Leu Thr Ser Ala Val
                805                 810                 815

Lys Ala Ala Val Glu Asp Gly Tyr Arg Leu Phe Leu Glu Val Ser Thr
            820                 825                 830

His Pro Val Val Ser His Ser Ile Asn Glu Thr Leu Met Asp Ala Gly
        835                 840                 845

Met Glu Asp Phe Ala Val Ile Pro Thr Leu Leu Arg Lys Lys Pro Thr
    850                 855                 860

Glu Lys His Ile Leu His Ser Ile Ala Gln Leu His Cys Arg Gly Ala
865                 870                 875                 880

Glu Val Asn Trp Ala Ala Gln Met Pro Gly Arg Trp Ala Thr Gly Val
                885                 890                 895

Pro Thr Thr Thr Trp Met His Lys Pro Ile Trp Arg Lys Ile Glu Thr
            900                 905                 910

Ala Pro Leu His Thr Gly Leu Thr His Asp Val Glu Lys His Thr Leu
        915                 920                 925

Leu Gly Gln Arg Ile Pro Val Pro Gly Thr Asp Thr Tyr Val Tyr Thr

930                 935                 940

Thr Arg Leu Asp Asn Asp Thr Lys Pro Phe Pro Gly Ser His Pro Leu
945                 950                 955                 960

His Gly Thr Glu Ile Val Pro Ala Ala Gly Leu Ile Asn Thr Phe Leu
                965                 970                 975

Lys Gly Thr Gly Gly Gln Met Leu Gln Asn Val Val Leu Arg Val Pro
            980                 985                 990

Val Ala Ile Asn Ala Pro Arg Ser  Val Gln Val Val Val  Gln Gln Asp
        995                 1000                 1005

Gln Val  Lys Val Val Ser Arg  Leu Ile Pro Ser Glu  Pro Ser Gln
    1010                 1015                 1020

Leu Asp  Asp Asp Ala Ser Trp  Val Thr His Thr Thr  Ala Tyr Trp
    1025                 1030                 1035

Asp Arg  Lys Val Ala Gly Ser  Glu Asp Arg Ile Asp  Phe Ala Ala
    1040                 1045                 1050

Val Lys  Ser Arg Leu Val Thr  Lys Leu Ala Asp Asn  Phe Ser Ile
    1055                 1060                 1065

Asp Tyr  Leu Asp Lys Val Gly  Val Ser Ala Met Gly  Phe Pro Trp
    1070                 1075                 1080

Ala Val  Thr Glu His Tyr Arg  Asn Asp Lys Glu Met  Leu Ala Arg
    1085                 1090                 1095

Val Asp  Val Asn Pro Ala Ile  Ser Gly Asp Ala Pro  Leu Pro Trp
    1100                 1105                 1110

Asp Ser  Ser Ser Trp Ala Pro  Val Leu Asp Ala Ala  Thr Ser Val
    1115                 1120                 1125

Gly Ser  Thr Ile Phe Pro Thr  Pro Ala Leu Arg Met  Pro Ala Gln
    1130                 1135                 1140

Ile Glu  Arg Val Glu Val Phe  Thr Ser Gln Asp Pro  Pro Lys Ile
    1145                 1150                 1155

Ser Trp  Leu Tyr Val Gln Glu  Ala Ser Asp Ser Val  Pro Thr Ser
    1160                 1165                 1170

His Val Ser Val Val Ser Glu Ala Gly Glu Val Leu Ala Lys Phe
    1175                1180                1185

Thr Ala Met Arg Phe Ser Glu Ile Glu Gly Thr Pro Gly Val Ser
    1190                1195                1200

Gly Ser Met Glu Ser Leu Val His Gln Ile Ala Trp Pro Pro Ala
    1205                1210                1215

Thr Pro Ala Glu Glu Pro Leu Ser Ile Glu Thr Val Ile Leu Val
    1220                1225                1230

Ser Pro Asp Ala Thr Thr Arg Ala Leu Tyr Ala Ala Ser Leu Pro
    1235                1240                1245

Thr Arg Val Asn Ser Phe Gln Phe Ser Ser Thr Gln Glu Phe Phe
    1250                1255                1260

Ser Asn Ala Ser Ser Leu Pro Leu Glu Lys Gly Thr Val Val Thr
    1265                1270                1275

Tyr Ile Pro Gly Glu Val Ala Ser Leu Ala Glu Val Pro Ala Ala
    1280                1285                1290

Ser Glu Ser Phe Thr Trp Asn Leu Leu Glu Leu Ile Lys Phe Thr
    1295                1300                1305

Val Asn Gly Ser Leu Pro Ile Lys Val Phe Thr Leu Thr Ala Asn
    1310                1315                1320

Ile Gly Glu Gly Gln Thr Pro Thr Ala Leu Ala Gln Ser Pro Leu
    1325                1330                1335

Tyr Gly Leu Ala Arg Val Ile Ala Ser Glu His Pro Asp Leu Gly
    1340                1345                1350

Thr Leu Ile Asp Val Glu Glu Pro Val Ile Pro Leu Ser Thr Met
    1355                1360                1365

Arg Tyr Ile Gln Gly Ala Asp Ile Ile Arg Ile Asn Asp Gly Ile
    1370                1375                1380

Ala Arg Thr Ser Arg Phe Arg Ser Leu Pro Arg Asn Lys Leu Leu
    1385                1390                1395

Pro Ala Ser Glu Gly Pro Arg Leu Leu Pro Arg Pro Glu Gly Thr
    1400                1405                1410

Tyr Leu Ile Thr Gly Gly Leu Gly Val Leu Gly Leu Glu Val Ala
    1415                1420                1425

Asp Phe Leu Val Glu Lys Gly Ala Arg Arg Leu Leu Leu Ile Ser
    1430                1435                1440

Arg Arg Ala Leu Pro Pro Arg Arg Thr Trp Asp Gln Val Ser Glu
    1445                1450                1455

Asp Leu Gln Pro Thr Ile Ala Lys Ile Arg Leu Leu Glu Ser Arg
    1460                1465                1470

Gly Ala Ser Val His Val Leu Pro Leu Asp Ile Thr Lys Pro Asp
    1475                1480                1485

Ala Val Glu Gln Leu Thr Thr Ala Leu Asp Arg Leu Ser Leu Pro
    1490                1495                1500

Ser Val Gln Gly Val Val His Ala Ala Gly Val Leu Asp Asn Glu
    1505                1510                1515

Leu Val Met Gln Thr Thr Arg Asp Ala Phe Asn Arg Val Leu Ala
    1520                1525                1530

Pro Lys Ile Ala Gly Ala Leu Ala Leu His Glu Val Phe Pro Pro
    1535                1540                1545

Lys Ser Val Asp Phe Phe Val Met Phe Ser Ser Cys Gly Asn Leu
    1550                1555                1560

Val Gly Phe Thr Gly Gln Ala Ser Tyr Gly Ser Gly Asn Ala Phe
    1565                1570                1575

Leu Asp Thr Leu Ala Thr His Arg Ala Arg Leu Gly Asp Ala Ala
    1580                1585                1590

Val Ser Phe Gln Trp Thr Ser Trp Arg Gly Leu Gly Met Gly Ala
    1595                1600                1605

Ser Thr Asp Phe Ile Asn Ala Glu Leu Glu Ser Lys Gly Ile Thr
    1610                1615                1620

Asp Val Thr Arg Asp Glu Ala Phe Ala Ala Trp Gln His Leu Ala
    1625                1630                1635

Lys Tyr Asp Met Asp His Gly Val Val Leu Arg Ser Arg Ala Phe
    1640                1645                1650

Glu Asp Gly Glu Pro Ile Pro Val Ser Ile Leu Asn Asp Ile Ala
    1655                1660                1665

Val Arg Arg Val Gly Thr Val Ser Asn Thr Ser Pro Ala Ala Ala
    1670                1675                1680

Gly Ser Ser Asp Ala Val Pro Thr Ser Gly Pro Glu Leu Lys Ala
    1685                1690                1695

Tyr Leu Asp Glu Lys Ile Arg Gly Cys Val Ala Lys Val Leu Gln
    1700                1705                1710

Met Thr Ala Glu Asp Val Asp Ser Lys Ala Ala Leu Ala Asp Leu
    1715                1720                1725

Gly Val Asp Ser Val Met Thr Val Thr Leu Arg Arg Gln Leu Gln
    1730                1735                1740

Leu Thr Leu Lys Ile Ala Val Pro Pro Thr Leu Thr Trp Ser His
    1745                1750                1755

Pro Thr Val Ser His Leu Ala Val Trp Phe Ala Glu Lys Leu Ala
    1760                1765                1770

Lys

<210> 2
<211> 344
<212> PRT
<213> Aspergillus nidulans

<400> 2

Met Val Gln Asp Thr Ser Ser Ala Ser Thr Ser Pro Ile Leu Thr Arg
1               5                   10                  15

Trp Tyr Ile Asp Thr Arg Pro Leu Thr Ala Ser Thr Ala Ala Leu Pro
            20                  25                  30

Leu Leu Glu Thr Leu Gln Pro Ala Asp Gln Ile Ser Val Gln Lys Tyr
        35                  40                  45

Tyr His Leu Lys Asp Lys His Met Ser Leu Ala Ser Asn Leu Leu Lys
    50                  55                  60

Tyr Leu Phe Val His Arg Asn Cys Arg Ile Pro Trp Ser Ser Ile Val
65                  70                  75                  80

Ile Ser Arg Thr Pro Asp Pro His Arg Arg Pro Cys Tyr Ile Pro Pro
                85                  90                  95

Ser Gly Ser Gln Glu Asp Ser Phe Lys Asp Gly Tyr Thr Gly Ile Asn
            100                 105                 110

Val Glu Phe Asn Val Ser His Gln Ala Ser Met Val Ala Ile Ala Gly
        115                 120                 125

Thr Ala Phe Thr Pro Asn Ser Gly Gly Asp Ser Lys Leu Lys Pro Glu
    130                 135                 140

Val Gly Ile Asp Ile Thr Cys Val Asn Glu Arg Gln Gly Arg Asn Gly
145                 150                 155                 160

Glu Glu Arg Ser Leu Glu Ser Leu Arg Gln Tyr Ile Asp Ile Phe Ser
                165                 170                 175

Glu Val Phe Ser Thr Ala Glu Met Ala Asn Ile Arg Arg Leu Asp Gly
            180                 185                 190

Val Ser Ser Ser Ser Leu Ser Ala Asp Arg Leu Val Asp Tyr Gly Tyr
        195                 200                 205

Arg Leu Phe Tyr Thr Tyr Trp Ala Leu Lys Glu Ala Tyr Ile Lys Met
    210                 215                 220

Thr Gly Glu Ala Leu Leu Ala Pro Trp Leu Arg Glu Leu Glu Phe Ser
225                 230                 235                 240

Asn Val Val Ala Pro Ala Ala Val Ala Glu Ser Gly Asp Ser Ala Gly
                245                 250                 255

Asp Phe Gly Glu Pro Tyr Thr Gly Val Arg Thr Thr Leu Tyr Lys Asn
            260                 265                 270

Leu Val Glu Asp Val Arg Ile Glu Val Ala Ala Leu Gly Gly Asp Tyr
        275                 280                 285

Leu Phe Ala Thr Ala Ala Arg Gly Gly Gly Ile Gly Ala Ser Ser Arg
    290                 295                 300

Pro Gly Gly Gly Pro Asp Gly Ser Gly Ile Arg Ser Gln Asp Pro Trp
305                 310                 315                 320

Arg Pro Phe Lys Lys Leu Asp Ile Glu Arg Asp Ile Gln Pro Cys Ala

325                 330                 335

Thr Gly Val Cys Asn Cys Leu Ser
            340

<210>  3
<211>  325
<212>  PRT
<213>  Aspergillus clavatus

<400>  3

Met Ala Lys Ile Asp Val His His His Phe Tyr Pro Gln Ala Met Arg
1               5                   10                  15

Glu Ala Leu Glu Arg Ala Gly Gly Asp Pro Ser Gly Trp Tyr Ile Pro
            20                  25                  30

Pro Trp Thr Leu Asp Leu Asp Lys Glu Ile Ser Arg Val Leu Lys Val
        35                  40                  45

Gln Thr Thr Ile Leu Ser Val Thr Ala Pro Gly Pro Gly Ile Glu Thr
    50                  55                  60

Asp Pro Gly Lys Ala Ala Ala Leu Ala Arg Leu Cys Asn Glu Glu Ala
65                  70                  75                  80

Ala Ala Ile Arg Asp Ala His Pro Leu Gln Tyr Gly Phe Phe Ala Ser
                85                  90                  95

Val Pro Ser Leu Phe Asp Thr Ala Ala Val Leu Ala Glu Ile Glu His
            100                 105                 110

Ala Phe Thr Asn Leu His Ala Asp Gly Val Thr Leu Tyr Thr Arg Tyr
        115                 120                 125

Gly Ala Gly His Ser Tyr Leu Gly Asp Glu Arg Phe Arg Pro Val Trp
    130                 135                 140

Ala Glu Leu Ser Lys Arg Arg Ala Val Val Phe Ile His Pro Thr His
145                 150                 155                 160

Ala Val Asp Thr Gln Leu Ile Asn Ser Trp Met Pro Gln Pro Met Phe
                165                 170                 175

Asp Tyr Pro His Glu Thr Gly Arg Thr Ala Met Asp Leu Leu Thr Arg
            180                 185                 190

Gly Val Ile Arg Asp Tyr Pro Gly Cys Lys Ile Ile Leu Ser His Ala

195                 200                 205

Gly Gly Thr Leu Pro Tyr Leu Ile His Arg Ala Ala Thr Met Leu Pro
    210                 215                 220

Phe Met Pro Arg Asn Leu Gly Met Ser Arg Glu Glu Ile Val Glu Ala
225                 230                 235                 240

Ala Arg Thr Leu Tyr Phe Asp Thr Ala Ile Ser Ala Asn Pro Val Thr
                245                 250                 255

Leu Lys Ala Leu Leu Glu Phe Ala Lys Pro Gly His Val Leu Phe Gly
            260                 265                 270

Ser Asp Phe Pro Asn Ala Pro Arg Gly Ala Ile Thr His Phe Thr Ser
        275                 280                 285

Phe Leu Glu Gly Tyr Asp Asn Met Ser Glu Glu Thr Arg Arg Leu Val
    290                 295                 300

Glu Arg Glu Ala Ala Leu Glu Leu Phe Pro Arg Leu Arg Gly Gln Ser
305                 310                 315                 320

Thr Arg Ala Cys Leu
                325

<210>  4
<211>  628
<212>  PRT
<213>  Salmonella typhimurium

<400>  4

Met Ser Phe Ser Glu Phe Tyr Gln Arg Ser Ile Asn Glu Pro Glu Ala
1               5                   10                  15

Phe Trp Ala Glu Gln Ala Arg Arg Ile Asp Trp Arg Gln Pro Phe Thr
            20                  25                  30

Gln Thr Leu Asp His Ser Arg Pro Pro Phe Ala Arg Trp Phe Cys Gly
        35                  40                  45

Gly Thr Thr Asn Leu Cys His Asn Ala Val Asp Arg Trp Arg Asp Lys
    50                  55                  60

Gln Pro Glu Ala Leu Ala Leu Ile Ala Val Ser Ser Glu Thr Asp Glu
65                  70                  75                  80

Glu Arg Thr Phe Thr Phe Ser Gln Leu His Asp Glu Val Asn Ile Val

85                  90                  95

Ala Ala Met Leu Leu Ser Leu Gly Val Gln Arg Gly Asp Arg Val Leu
            100                 105                 110

Val Tyr Met Pro Met Ile Ala Glu Ala Gln Ile Thr Leu Leu Ala Cys
        115                 120                 125

Ala Arg Ile Gly Ala Ile His Ser Val Val Phe Gly Gly Phe Ala Ser
    130                 135                 140

His Ser Val Ala Ala Arg Ile Asp Asp Ala Arg Pro Ala Leu Ile Val
145                 150                 155                 160

Ser Ala Asp Ala Gly Ala Arg Gly Gly Lys Ile Leu Pro Tyr Lys Lys
                165                 170                 175

Leu Leu Asp Asp Ala Ile Ala Gln Ala Gln His Gln Pro Lys His Val
            180                 185                 190

Leu Leu Val Asp Arg Gly Leu Ala Lys Met Ala Trp Val Asp Gly Arg
        195                 200                 205

Asp Leu Asp Phe Ala Thr Leu Arg Gln Gln His Leu Gly Ala Ser Val
    210                 215                 220

Pro Val Ala Trp Leu Glu Ser Asn Glu Thr Ser Cys Ile Leu Tyr Thr
225                 230                 235                 240

Ser Gly Thr Thr Gly Lys Pro Lys Gly Val Gln Arg Asp Val Gly Gly
                245                 250                 255

Tyr Ala Val Ala Leu Ala Thr Ser Met Asp Thr Ile Phe Gly Gly Lys
            260                 265                 270

Ala Gly Gly Val Phe Phe Cys Ala Ser Asp Ile Gly Trp Val Val Gly
        275                 280                 285

His Ser Tyr Ile Val Tyr Ala Pro Leu Leu Ala Gly Met Ala Thr Ile
    290                 295                 300

Val Tyr Glu Gly Leu Pro Thr Tyr Pro Asp Cys Gly Val Trp Trp Lys
305                 310                 315                 320

Ile Val Glu Lys Tyr Gln Val Asn Arg Met Phe Ser Ala Pro Thr Ala
                325                 330                 335

Ile Arg Val Leu Lys Lys Phe Pro Thr Ala Gln Ile Arg Asn His Asp
            340                 345                 350

Leu Ser Ser Leu Glu Ala Leu Tyr Leu Ala Gly Glu Pro Leu Asp Glu
        355                 360                 365

Pro Thr Ala Ser Trp Val Thr Glu Thr Leu Gly Val Pro Val Ile Asp
    370                 375                 380

Asn Tyr Trp Gln Thr Glu Ser Gly Trp Pro Ile Met Ala Leu Ala Arg
385                 390                 395                 400

Ala Leu Asp Asp Arg Pro Ser Arg Leu Gly Ser Pro Gly Val Pro Met
                405                 410                 415

Tyr Gly Tyr Asn Val Gln Leu Leu Asn Glu Val Thr Gly Glu Pro Cys
            420                 425                 430

Gly Ile Asn Glu Lys Gly Met Leu Val Ile Glu Gly Pro Leu Pro Pro
        435                 440                 445

Gly Cys Ile Gln Thr Ile Trp Gly Asp Asp Ala Arg Phe Val Lys Thr
    450                 455                 460

Tyr Trp Ser Leu Phe Asn Arg Gln Val Tyr Ala Thr Phe Asp Trp Gly
465                 470                 475                 480

Ile Arg Asp Ala Glu Gly Tyr Tyr Phe Ile Leu Gly Arg Thr Asp Asp
                485                 490                 495

Val Ile Asn Ile Ala Gly His Arg Leu Gly Thr Arg Glu Ile Glu Glu
            500                 505                 510

Ser Ile Ser Ser Tyr Pro Asn Val Ala Glu Val Ala Val Val Gly Ile
        515                 520                 525

Lys Asp Ala Leu Lys Gly Gln Val Ala Val Ala Phe Val Ile Pro Lys
    530                 535                 540

Gln Ser Asp Thr Leu Ala Asp Arg Glu Ala Ala Arg Asp Glu Glu Asn
545                 550                 555                 560

Ala Ile Met Ala Leu Val Asp Asn Gln Ile Gly His Phe Gly Arg Pro
                565                 570                 575

Ala His Val Trp Phe Val Ser Gln Leu Pro Lys Thr Arg Ser Gly Lys
            580                 585                 590

Met Leu Arg Arg Thr Ile Gln Ala Ile Cys Glu Gly Arg Asp Pro Gly
        595                 600                 605

Asp Leu Thr Thr Ile Asp Asp Pro Ala Ser Leu Gln Gln Ile Arg Gln
    610                 615                 620

Ala Ile Glu Glu
625

<210>  5
<211>  398
<212>  PRT
<213>  Saccharomyces cerevisiae

<400>  5

Met Ser Gln Asn Val Tyr Ile Val Ser Thr Ala Arg Thr Pro Ile Gly
1               5                   10                  15

Ser Phe Gln Gly Ser Leu Ser Ser Lys Thr Ala Val Glu Leu Gly Ala
            20                  25                  30

Val Ala Leu Lys Gly Ala Leu Ala Lys Val Pro Glu Leu Asp Ala Ser
        35                  40                  45

Lys Asp Phe Asp Glu Ile Ile Phe Gly Asn Val Leu Ser Ala Asn Leu
    50                  55                  60

Gly Gln Ala Pro Ala Arg Gln Val Ala Leu Ala Ala Gly Leu Ser Asn
65                  70                  75                  80

His Ile Val Ala Ser Thr Val Asn Lys Val Cys Ala Ser Ala Met Lys
                85                  90                  95

Ala Ile Ile Leu Gly Ala Gln Ser Ile Lys Cys Gly Asn Ala Asp Val
            100                 105                 110

Val Val Ala Gly Gly Cys Glu Ser Met Thr Asn Ala Pro Tyr Tyr Met
        115                 120                 125

Pro Ala Ala Arg Ala Gly Ala Lys Phe Gly Gln Thr Val Leu Val Asp
    130                 135                 140

Gly Val Glu Arg Asp Gly Leu Asn Asp Ala Tyr Asp Gly Leu Ala Met
145                 150                 155                 160

Gly Val His Ala Glu Lys Cys Ala Arg Asp Trp Asp Ile Thr Arg Glu
                165                 170                 175

Gln Gln Asp Asn Phe Ala Ile Glu Ser Tyr Gln Lys Ser Gln Lys Ser
            180                 185                 190

Gln Lys Glu Gly Lys Phe Asp Asn Glu Ile Val Pro Val Thr Ile Lys
        195                 200                 205

Gly Phe Arg Gly Lys Pro Asp Thr Gln Val Thr Lys Asp Glu Glu Pro
    210                 215                 220

Ala Arg Leu His Val Glu Lys Leu Arg Ser Ala Arg Thr Val Phe Gln
225                 230                 235                 240

Lys Glu Asn Gly Thr Val Thr Ala Ala Asn Ala Ser Pro Ile Asn Asp
                245                 250                 255

Gly Ala Ala Ala Val Ile Leu Val Ser Glu Lys Val Leu Lys Glu Lys
            260                 265                 270

Asn Leu Lys Pro Leu Ala Ile Ile Lys Gly Trp Gly Glu Ala Ala His
        275                 280                 285

Gln Pro Ala Asp Phe Thr Trp Ala Pro Ser Leu Ala Val Pro Lys Ala
    290                 295                 300

Leu Lys His Ala Gly Ile Glu Asp Ile Asn Ser Val Asp Tyr Phe Glu
305                 310                 315                 320

Phe Asn Glu Ala Phe Ser Val Val Gly Leu Val Asn Thr Lys Ile Leu
                325                 330                 335

Lys Leu Asp Pro Ser Lys Val Asn Val Tyr Gly Gly Ala Val Ala Leu
            340                 345                 350

Gly His Pro Leu Gly Cys Ser Gly Ala Arg Val Val Val Thr Leu Leu
        355                 360                 365

Ser Ile Leu Gln Gln Glu Gly Gly Lys Ile Gly Val Ala Ala Ile Cys
    370                 375                 380

Asn Gly Gly Gly Gly Ala Ser Ser Ile Val Ile Glu Lys Ile
385                 390                 395

<210>  6
<211>  282
<212>  PRT
<213>  Clostridium acetobutylicum

<400>  6

Met Lys Lys Val Cys Val Ile Gly Ala Gly Thr Met Gly Ser Gly Ile
1               5                   10                  15

Ala Gln Ala Phe Ala Ala Lys Gly Phe Glu Val Val Leu Arg Asp Ile
            20                  25                  30

Lys Asp Glu Phe Val Asp Arg Gly Leu Asp Phe Ile Asn Lys Asn Leu
        35                  40                  45

Ser Lys Leu Val Lys Lys Gly Lys Ile Glu Glu Ala Thr Lys Val Glu
    50                  55                  60

Ile Leu Thr Arg Ile Ser Gly Thr Val Asp Leu Asn Met Ala Ala Asp
65                  70                  75                  80

Cys Asp Leu Val Ile Glu Ala Ala Val Glu Arg Met Asp Ile Lys Lys
                85                  90                  95

Gln Ile Phe Ala Asp Leu Asp Asn Ile Cys Lys Pro Glu Thr Ile Leu
            100                 105                 110

Ala Ser Asn Thr Ser Ser Leu Ser Ile Thr Glu Val Ala Ser Ala Thr
        115                 120                 125

Lys Arg Pro Asp Lys Val Ile Gly Met His Phe Phe Asn Pro Ala Pro
    130                 135                 140

Val Met Lys Leu Val Glu Val Ile Arg Gly Ile Ala Thr Ser Gln Glu
145                 150                 155                 160

Thr Phe Asp Ala Val Lys Glu Thr Ser Ile Ala Ile Gly Lys Asp Pro
                165                 170                 175

Val Glu Val Ala Glu Ala Pro Gly Phe Val Val Asn Arg Ile Leu Ile
            180                 185                 190

Pro Met Ile Asn Glu Ala Val Gly Ile Leu Ala Glu Gly Ile Ala Ser
        195                 200                 205

Val Glu Asp Ile Asp Lys Ala Met Lys Leu Gly Ala Asn His Pro Met
    210                 215                 220

Gly Pro Leu Glu Leu Gly Asp Phe Ile Gly Leu Asp Ile Cys Leu Ala
225                 230                 235                 240

Ile Met Asp Val Leu Tyr Ser Glu Thr Gly Asp Ser Lys Tyr Arg Pro
                245                 250                 255

His Thr Leu Leu Lys Lys Tyr Val Arg Ala Gly Trp Leu Gly Arg Lys
        260                 265                 270

Ser Gly Lys Gly Phe Tyr Asp Tyr Ser Lys
        275                 280

<210>  7
<211>  261
<212>  PRT
<213>  Clostridium acetobutylicum

<400>  7

Met Glu Leu Asn Asn Val Ile Leu Glu Lys Glu Gly Lys Val Ala Val
1               5                   10                  15

Val Thr Ile Asn Arg Pro Lys Ala Leu Asn Ala Leu Asn Ser Asp Thr
            20                  25                  30

Leu Lys Glu Met Asp Tyr Val Ile Gly Glu Ile Glu Asn Asp Ser Glu
        35                  40                  45

Val Leu Ala Val Ile Leu Thr Gly Ala Gly Glu Lys Ser Phe Val Ala
    50                  55                  60

Gly Ala Asp Ile Ser Glu Met Lys Glu Met Asn Thr Ile Glu Gly Arg
65                  70                  75                  80

Lys Phe Gly Ile Leu Gly Asn Lys Val Phe Arg Arg Leu Glu Leu Leu
                85                  90                  95

Glu Lys Pro Val Ile Ala Ala Val Asn Gly Phe Ala Leu Gly Gly Gly
            100                 105                 110

Cys Glu Ile Ala Met Ser Cys Asp Ile Arg Ile Ala Ser Ser Asn Ala
        115                 120                 125

Arg Phe Gly Gln Pro Glu Val Gly Leu Gly Ile Thr Pro Gly Phe Gly
    130                 135                 140

Gly Thr Gln Arg Leu Ser Arg Leu Val Gly Met Gly Met Ala Lys Gln
145                 150                 155                 160

Leu Ile Phe Thr Ala Gln Asn Ile Lys Ala Asp Glu Ala Leu Arg Ile
                165                 170                 175

Gly Leu Val Asn Lys Val Val Glu Pro Ser Glu Leu Met Asn Thr Ala
            180                 185                 190

Lys Glu Ile Ala Asn Lys Ile Val Ser Asn Ala Pro Val Ala Val Lys
        195                 200                 205

Leu Ser Lys Gln Ala Ile Asn Arg Gly Met Gln Cys Asp Ile Asp Thr
    210                 215                 220

Ala Leu Ala Phe Glu Ser Glu Ala Phe Gly Glu Cys Phe Ser Thr Glu
225                 230                 235                 240

Asp Gln Lys Asp Ala Met Thr Ala Phe Ile Glu Lys Arg Lys Ile Glu
                245                 250                 255

Gly Phe Lys Asn Arg
            260

<210>  8
<211>  397
<212>  PRT
<213>  Treponema denticola

<400>  8

Met Ile Val Lys Pro Met Val Arg Asn Asn Ile Cys Leu Asn Ala His
1               5                   10                  15

Pro Gln Gly Cys Lys Lys Gly Val Glu Asp Gln Ile Glu Tyr Thr Lys
            20                  25                  30

Lys Arg Ile Thr Ala Glu Val Lys Ala Gly Ala Lys Ala Pro Lys Asn
        35                  40                  45

Val Leu Val Leu Gly Cys Ser Asn Gly Tyr Gly Leu Ala Ser Arg Ile
    50                  55                  60

Thr Ala Ala Phe Gly Tyr Gly Ala Ala Thr Ile Gly Val Ser Phe Glu
65                  70                  75                  80

Lys Ala Gly Ser Glu Thr Lys Tyr Gly Thr Pro Gly Trp Tyr Asn Asn
                85                  90                  95

Leu Ala Phe Asp Glu Ala Ala Lys Arg Glu Gly Leu Tyr Ser Val Thr
            100                 105                 110

Ile Asp Gly Asp Ala Phe Ser Asp Glu Ile Lys Ala Gln Val Ile Glu
        115                 120                 125

Glu Ala Lys Lys Lys Gly Ile Lys Phe Asp Leu Ile Val Tyr Ser Leu
    130                 135                 140

Ala Ser Pro Val Arg Thr Asp Pro Asp Thr Gly Ile Met His Lys Ser
145                 150                 155                 160

Val Leu Lys Pro Phe Gly Lys Thr Phe Thr Gly Lys Thr Val Asp Pro
                165                 170                 175

Phe Thr Gly Glu Leu Lys Glu Ile Ser Ala Glu Pro Ala Asn Asp Glu
            180                 185                 190

Glu Ala Ala Ala Thr Val Lys Val Met Gly Gly Glu Asp Trp Glu Arg
        195                 200                 205

Trp Ile Lys Gln Leu Ser Lys Glu Gly Leu Leu Glu Glu Gly Cys Ile
    210                 215                 220

Thr Leu Ala Tyr Ser Tyr Ile Gly Pro Glu Ala Thr Gln Ala Leu Tyr
225                 230                 235                 240

Arg Lys Gly Thr Ile Gly Lys Ala Lys Glu His Leu Glu Ala Thr Ala
                245                 250                 255

His Arg Leu Asn Lys Glu Asn Pro Ser Ile Arg Ala Phe Val Ser Val
            260                 265                 270

Asn Lys Gly Leu Val Thr Arg Ala Ser Ala Val Ile Pro Val Ile Pro
        275                 280                 285

Leu Tyr Leu Ala Ser Leu Phe Lys Val Met Lys Glu Lys Gly Asn His
    290                 295                 300

Glu Gly Cys Ile Glu Gln Ile Thr Arg Leu Tyr Ala Glu Arg Leu Tyr
305                 310                 315                 320

Arg Lys Asp Gly Thr Ile Pro Val Asp Glu Glu Asn Arg Ile Arg Ile
                325                 330                 335

Asp Asp Trp Glu Leu Glu Glu Asp Val Gln Lys Ala Val Ser Ala Leu
            340                 345                 350

Met Glu Lys Val Thr Gly Glu Asn Ala Glu Ser Leu Thr Asp Leu Ala
        355                 360                 365

Gly Tyr Arg His Asp Phe Leu Ala Ser Asn Gly Phe Asp Val Glu Gly
    370                 375                 380

Ile Asn Tyr Glu Ala Glu Val Glu Arg Phe Asp Arg Ile

385                 390                 395

<210>  9
<211>  5325
<212>  DNA
<213>  Artificial Sequence

<220>
<223>  codon optimized MSAS

<400>  9
atgcactctg ctgctacttc tacttaccca tctggtaaga cttctccagc tccagttggt      60

actccaggta ctgaatactc tgaatacgaa ttctctaacg acgttgctgt tgttggtatg     120

gcttgtagag ttgctggtgg taaccacaac ccagaattgt tgtggcaatc tttgttgtct     180

caaaagtctg ctatgggtga aatcccacca atgagatggg aaccatacta cagaagagac     240

gctagaaacg aaaagttctt gaagaacact acttctagag gttacttctt ggacagattg     300

gaagacttcg actgtcaatt cttcggtatc tctccaaagg aagctgaaca aatggaccca     360

caacaaagag tttctttgga agttgcttct gaagctttgg aagacgctgg tatcccagct     420

aagtctttgt ctggttctga cactgctgtt ttctggggtg ttaactctga cgactactct     480

aagttggttt tggaagactt gccaaacgtt gaagcttgga tgggtatcgg tactgcttac     540

tgtggtgttc caaacagaat ctcttaccac ttgaacttga tgggtccatc tactgctgtt     600

gacgctgctt gtgcttcttc tttggttgct atccaccacg gtgttcaagc tatcagattg     660

ggtgaatcta aggttgctat cgttggtggt gttaacgctt tgtgtggtcc aggtttgact     720

agagttttgg acaaggctgg tgctatctct tctgacggtt cttgtaagtc tttcgacgac     780

gacgctcacg gttacgctag aggtgaaggt gctggtgctt tggttttgaa gtctttgcac     840

agagctttgt tggaccacga caacgttttg gctgttatca agggttctgc tgtttgtcaa     900

gacggtaaga ctaacggtat catggctcca aactctgttg ctcaacaatt ggctgctaac     960

aacgctttgt ctgctgctaa catcgaccca cacactgtta gatacgttga agctcacgct    1020

acttctactc cattgggtga cccaactgaa atctctgcta tcgcttctgt ttacggtgct    1080

gacagaccag ctgacgaccc atgttacatc ggttctatca agccaaacat cggtcacttg    1140

gaagctggtg ctggtgttat gggtttcatc aaggctgttt tggctatcca aaagggtgtt    1200

ttgccaccac aagctaactt gactaagttg aactctagaa tcgactggaa gactgctggt    1260

gttaaggttg ttcaagaagc tactccatgg ccagaatctg acccaatcag aagagctggt    1320

gtttgttctt acggttacgg tggtactgtt tctcacgctg ttatcgaaga attctctcca    1380

atcttgcaac cagacccatt gggtaacggt gctgtttctg gtccaggttt gttgttgttg    1440

tctggtccac aagaaaagag attggctttg caagctaaga ctttgagaga ctggatgact    1500

gctgaaggta aggaccacaa cttgtctgac atcttgacta ctttggctac tagaagagac    1560

caccacgact acagagctgc tttggttgtt gacgactaca gagacgctga acaagttttg 1620

caatctttgg ctaacggtgt tgaccacact ttcactactc aatctagagt tttgggttct 1680

gacatctcta aggacgttgt ttgggttttc tctggtcacg gtgctcaatg gccagacatg 1740

ggtaagcaat tgatccacaa cccagttttc ttcgctgcta tccaaccatt ggacgaattg 1800

atccaagctg aaatcggttt gtctccaatc gaattgttga gaactggtga cttcgaatct 1860

tctgacagag ttcaaatctt gacttacgtt atgcaaatcg gtttgtctgc tttgttgcaa 1920

tctaacggta tcactccaca agctgttatc ggtcactctg ttggtgaaat cgctgcttcc 1980

gttgttgctg gtgctttgtc tccagctgaa ggtgctttga tcgttactag aagagctttg 2040

ttgtacagac aagttatggg taagggtggt atgatcttgg ttaacttgcc atctgctgaa 2100

actgaagaaa tcttgggttc tagatctgac ttggttgttg ctatcgactc ttctccatct 2160

tcttgtgttg ttgctggtga caaggaattg gttgctgaaa ctgctgaagc tttgaaggct 2220

agaggtgtta agactttcac tgttaagtct gacatcgctt tccactctcc aactttgaac 2280

ggtttggttg acccattgag agacgttttg gctgaaactt tgtctccagt ttctccaaac 2340

gttaagttgt actctactgc tttggctgac ccaagaggtc aagacttgag agacgttgaa 2400

tactgggctg gtaacatggt taacagagtt agattgactt ctgctgttaa ggctgctgtt 2460

gaagacggtt acagattgtt cttggaagtt tctactcacc cagttgtttc tcactctatc 2520

aacgaaactt tgatggacgc tggtatggaa gacttcgctg ttatcccaac tttgttgaga 2580

aagaagccaa ctgaaaagca catcttgcac tctatcgctc aattgcactg tagaggtgct 2640

gaagttaact gggctgctca aatgccaggt agatgggcta ctggtgttcc aactactact 2700

tggatgcaca agccaatctg gagaaagatc gaaactgctc cattgcacac tggtttgact 2760

cacgacgttg aaaagcacac tttgttgggt caaagaatcc cagttccagg tactgacact 2820

tacgtttaca ctactagatt ggacaacgac actaagccat tcccaggttc tcacccattg 2880

cacggtactg aaatcgttcc agctgctggt ttgatcaaca ctttcttgaa gggtactggt 2940

ggtcaaatgt tgcaaaacgt tgttttgaga gttccagttg ctatcaacgc tccaagatct 3000

gttcaagttg ttgttcaaca agaccaagtt aaggttgttt ctagattgat cccatctgaa 3060

ccatctcaat tggacgacga cgcttcttgg gttactcaca ctactgctta ctgggacaga 3120

aaggttgctg gttctgaaga cagaatcgac ttcgctgctg ttaagtctag attggttact 3180

aagttggctg acaacttctc tatcgactac ttggacaagg ttggtgtttc tgctatgggt 3240

ttcccatggg ctgttactga acactacaga aacgacaagg aaatgttggc tagagttgac 3300

gttaacccag ctatctctgg tgacgctcca ttgccatggg actcttcttc ttgggctcca 3360

gttttggacg ctgctacttc tgttggttct actatcttcc caactccagc tttgagaatg 3420

ccagctcaaa tcgaaagagt tgaagttttc acttctcaag acccaccaaa gatctcttgg 3480

ttgtacgttc aagaagcttc tgactctgtt ccaacttctc acgtttctgt tgtttctgaa 3540

gctggtgaag ttttggctaa gttcactgct atgagattct ctgaaatcga aggtactcca 3600

ggtgtttctg gttctatgga atctttggtt caccaaatcg cttggccacc agctactcca 3660

gctgaagaac cattgtctat cgaaactgtt atcttggttt ctccagacgc tactactaga 3720

gctttgtacg ctgcttcttt gccaactaga gttaactctt tccaattctc ttctactcaa 3780

gaattcttct ctaacgcttc ttctttgcca ttggaaaagg gtactgttgt tacttacatc 3840

ccaggtgaag ttgcttcttt ggctgaagtt ccagctgctt ctgaatcttt cacttggaac 3900

ttgttggaat tgatcaagtt cactgttaac ggttctttgc caatcaaggt tttcactttg 3960

actgctaaca tcggtgaagg tcaaactcca actgctttgg ctcaatctcc attgtacggt 4020

ttggctagag ttatcgcttc tgaacaccca gacttgggta ctttgatcga cgttgaagaa 4080

ccagttatcc cattgtctac tatgagatac atccaaggtg ctgacatcat cagaatcaac 4140

gacggtatcg ctagaacttc tagattcaga tctttgccaa gaaacaagtt gttgccagct 4200

tctgaaggtc caagattgtt gccaagacca gaaggtactt acttgatcac tggtggtttg 4260

ggtgttttgg gtttggaagt tgctgacttc ttggttgaaa agggtgctag aagattgttg 4320

ttgatctcta gaagagcttt gccaccaaga agaacttggg accaagtttc tgaagacttg 4380

caaccaacta tcgctaagat cagattgttg gaatctagag gtgcttctgt tcacgttttg 4440

ccattggaca tcactaagcc agacgctgtt gaacaattga ctactgcttt ggacagattg 4500

tctttgccat ctgttcaagg tgttgttcac gctgctggtg ttttggacaa cgaattggtt 4560

atgcaaacta ctagagacgc tttcaacaga gttttggctc caaagatcgc tggtgctttg 4620

gctttgcacg aagttttccc accaaagtct gttgacttct tcgttatgtt ctcttcttgt 4680

ggtaacttgg ttggtttcac tggtcaagct tcttacggtt ctggtaacgc tttcttggac 4740

actttggcta ctcacagagc tagattgggt gacgctgctg tttctttcca atggacttct 4800

tggagaggtt tgggtatggg tgcttctact gacttcatca acgctgaatt ggaatctaag 4860

ggtatcactg acgttactag agacgaagct ttcgctgctt ggcaacactt ggctaagtac 4920

gacatggacc acggtgttgt tttgagatct agagctttcg aagacggtga accaatccca 4980

gtttctatct tgaacgacat cgctgttaga agagttggta ctgtttctaa cacttctcca 5040

gctgctgctg gttcttctga cgctgttcca acttctggtc cagaattgaa ggcttacttg 5100

gacgaaaaga tcagaggttg tgttgctaag gttttgcaaa tgactgctga agacgttgac 5160

tctaaggctg ctttggctga cttgggtgtt gactctgtta tgactgttac tttgagaaga 5220

caattgcaac ttactttgaa gatcgctgtt ccaccaactt tgacttggtc tcacccaact 5280

gtttctcact tggctgtttg gttcgctgaa aagttggcta agtaa 5325

<210>  10
<211>  1035
<212>  DNA
<213>  Artificial Sequence

<220>
<223>  Codon optimized PPTase (npgA)

<400>  10
atggttcaag acacttcttc tgcttctact tctccaatct tgactagatg gtacatcgac      60

actagaccat tgactgcttc tactgctgct ttgccattgt tggaaacttt gcaaccagct     120

gaccaaatct ctgttcaaaa gtactaccac ttgaaggaca agcacatgtc tttggcttct     180

aacttgttga agtacttgtt cgttcacaga aactgtagaa tcccatggtc ttctatcgtt     240

atctctagaa ctccagaccc acacagaaga ccatgttaca tcccaccatc tggttctcaa     300

gaagactctt tcaaggacgg ttacactggt atcaacgttg aatttaacgt ttctcaccaa     360

gcttctatgg ttgctatcgc tggtactgct ttcactccaa actctggtgg tgactctaag     420

ttgaagccag aagttggtat cgacatcact tgtgttaacg aaagacaagg tagaaacggt     480

gaagaaagat ctttggaatc tttgagacaa tacatcgaca tcttctctga agttttctct     540

actgctgaaa tggctaacat cagaagattg gacggtgttt cttcttcttc tttgtctgct     600

gacagattgg ttgactacgg ttacagattg ttctacactt actgggcttt gaaggaagct     660

tacatcaaga tgactggtga agctttgttg gctccatggt tgagagaatt ggaattctct     720

aacgttgttg ctccagctgc tgttgctgaa tctggtgact ctgctggtga cttcggtgaa     780

ccatacactg gtgttagaac tactttgtac aagaacttgg ttgaagacgt tagaatcgaa     840

gttgctgctt tgggtggtga ctacttgttc gctactgctg ctagaggtgg tggtatcggt     900

gcttcttcta gaccaggtgg tggtccagac ggttctggta tcagatctca agacccatgg     960

agaccattca agaagttgga catcgaaaga gacatccaac catgtgctac tggtgtttgt    1020

aactgtttgt cttaa                                                     1035


<210>  11
<211>  978
<212>  DNA
<213>  Artificial Sequence

<220>
<223>  Codon optimized 6-MSA decarboxylase (patG)

<400>  11
atggctaaga tcgacgttca ccaccacttc tacccacaag ctatgagaga agctttggaa      60

agagctggtg gtgacccatc tggttggtac atcccaccat ggactttgga cttggacaag     120

gaaatctcta gagttttgaa ggttcaaact actatcttgt ctgttactgc tccaggtcca     180

ggtatcgaaa ctgacccagg taaggctgct gctttggcta gattgtgtaa cgaagaagct 240

gctgctatca gagacgctca cccattgcaa tacggtttct tcgcttctgt tccatctttg 300

ttcgacactg ctgctgtttt ggctgaaatc gaacacgctt tcactaactt gcacgctgac 360

ggtgttactt tgtacactag atacggtgct ggtcactctt acttgggtga cgaaagattc 420

agaccagttt gggctgaatt gtctaagaga agagctgttg ttttcatcca cccaactcac 480

gctgttgaca ctcaattgat caactcttgg atgccacaac caatgttcga ctacccacac 540

gaaactggta gaactgctat ggacttgttg actagaggtg ttatcagaga ctacccaggt 600

tgtaagatca tcttgtctca cgctggtggt actttgccat acttgatcca cagagctgct 660

actatgttgc cattcatgcc aagaaacttg ggtatgtcta gagaagaaat cgttgaagct 720

gctagaactt tgtacttcga cactgctatc tctgctaacc cagttacttt gaaggctttg 780

ttggaattcg ctaagccagg tcacgttttg ttcggttctg acttcccaaa cgctccaaga 840

ggtgctatca ctcacttcac ttctttcttg gaaggttacg acaacatgtc tgaagaaact 900

agaagattgg ttgaaagaga agctgctttg gaattgttcc caagattgag aggtcaatct 960

actagagctt gtttgtaa 978

<210> 12
<211> 1887
<212> DNA
<213> Artificial Sequence

<220>
<223> Codin optimized prpE

<400> 12
atgtctttct ctgaattcta ccaaagatct atcaacgaac cagaagcttt ctgggctgaa 60

caagctagaa gaatcgactg gagacaacca ttcactcaaa ctttggacca ctctagacca 120

ccattcgcta gatggttctg tggtggtact actaacttgt gtcacaacgc tgttgacaga 180

tggagagaca agcaaccaga agctttggct ttgatcgctg tttcttctga aactgacgaa 240

gaaagaactt tcactttctc tcaattgcac gacgaagtta acatcgttgc tgctatgttg 300

ttgtctttgg gtgttcaaag aggtgacaga gttttggttt acatgccaat gatcgctgaa 360

gctcaaatca ctttgttggc ttgtgctaga atcggtgcta tccactctgt tgttttcggt 420

ggtttcgctt ctcactctgt tgctgctaga atcgacgacg ctagaccagc tttgatcgtt 480

tctgctgacg ctggtgctag aggtggtaag atcttgccat acaagaagtt gttggacgac 540

gctatcgctc aagctcaaca ccaaccaaag cacgttttgt tggttgacag aggtttggct 600

aagatggctt gggttgacgg tagagacttg gacttcgcta ctttgagaca acaacacttg 660

ggtgcttctg ttccagttgc ttggttggaa tctaacgaaa cttcttgtat cttgtacact 720

tctggtacta ctggtaagcc aaagggtgtt caaagagacg ttggtggtta cgctgttgct 780

ttggctactt ctatggacac tatcttcggt ggtaaggctg gtggtgtttt cttctgtgct 840

tctgacatcg gttgggttgt tggtcactct tacatcgttt acgctccatt gttggctggt 900

atggctacta tcgtttacga aggtttgcca acttacccag actgtggtgt ttggtggaag 960

atcgttgaaa agtaccaagt taacagaatg ttctctgctc caactgctat cagagttttg 1020

aagaagttcc caactgctca aatcagaaac cacgacttgt cttctttgga agctttgtac 1080

ttggctggtg aaccattgga cgaaccaact gcttcttggg ttactgaaac tttgggtgtt 1140

ccagttatcg acaactactg gcaaactgaa tctggttggc caatcatggc tttggctaga 1200

gctttggacg acagaccatc tagattgggt tctccaggtg ttccaatgta cggttacaac 1260

gttcaattgt tgaacgaagt tactggtgaa ccatgtggta tcaacgaaaa gggtatgttg 1320

gttatcgaag gtccattgcc accaggttgt atccaaacta tctggggtga cgacgctaga 1380

ttcgttaaga cttactggtc tttgttcaac agacaagttt acgctacttt cgactggggt 1440

atcagagacg ctgaaggtta ctacttcatc ttgggtagaa ctgacgacgt tatcaacatc 1500

gctggtcaca gattgggtac tagagaaatc gaagaatcta tctcttctta cccaaacgtt 1560

gctgaagttg ctgttgttgg tatcaaggac gctttgaagg gtcaagttgc tgttgctttc 1620

gttatcccaa agcaatctga cactttggct gacagagaag ctgctagaga cgaagaaaac 1680

gctatcatgg ctttggttga caaccaaatc ggtcacttcg gtagaccagc tcacgtttgg 1740

ttcgtttctc aattgccaaa gactagatct ggtaagatgt tgagaagaac tatccaagct 1800

atctgtgaag gtagagaccc aggtgacttg actactatcg acgacccagc ttctttgcaa 1860

caaatcagac aagctatcga agaataa 1887

<210> 13
<211> 1197
<212> DNA
<213> Saccharomyces cerevisiae

<400> 13
atgtctcaaa acgtttacat tgtttctact gctagaaccc caattggttc tttccaaggt 60

tctttgtcct ccaagaccgc tgttgaattg ggtgctgttg ctttgaaggg tgctttggct 120

aaggttccag aattggatgc ttccaaggat ttcgacgaaa ttattttcgg taacgttttg 180

tctgctaact tgggtcaagc tccagctaga caagttgctt tggctgctgg tttgtctaac 240

cacatcgttg cttctaccgt taacaaggtc tgtgcttccg ctatgaaggc tatcattttg 300

ggtgctcaat ccatcaagtg tggtaacgct gatgttgtcg ttgctggtgg ttgtgaatct 360

atgactaacg ctccatacta catgccagct gctagagctg gtgctaagtt cggtcaaact 420

gttttggttg atggtgtcga aagagatggt ttgaacgatg cttacgatgg tttggctatg 480

ggtgttcacg ctgaaaagtg tgctagagat tgggatatta ctagagaaca acaagacaac 540

ttcgctatcg aatcctacca aaagtctcaa aagtctcaaa aggaaggtaa gttcgacaac 600

gaaattgttc cagttaccat taagggtttc agaggtaagc cagatactca agtcaccaag 660

gacgaagaac cagctagatt gcacgttgaa aagttgagat ctgctagaac tgttttccaa 720

aaggaaaacg gtactgttac tgctgctaac gcttctccaa tcaacgatgg tgctgctgct 780

gtcatcttgg tttccgaaaa ggttttgaag gaaaagaact tgaagccatt ggctattatc 840

aagggttggg gtgaagctgc tcaccaacca gctgatttca cctgggctcc atctttggct 900

gttccaaagg ctttgaagca cgctggtatc gaagacatca actctgttga ttacttcgaa 960

ttcaacgaag ctttctctgt tgtcggtttg gttaacacta agattttgaa gttggaccca 1020

tctaaggtta acgtttacgg tggtgctgtt gctttgggtc acccattggg ttgttctggt 1080

gctagagttg ttgttacctt gttgtccatc ttgcaacaag aaggtggtaa gatcggtgtt 1140

gctgctattt gtaacggtgg tggtggtgct tcctctattg tcattgaaaa gatttaa 1197

<210> 14
<211> 849
<212> DNA
<213> Artificial Sequence

<220>
<223> Codon optimized Hbd

<400> 14
atgaagaagg tttgtgttat tggtgctggt actatgggtt ctggtattgc tcaagctttc 60

gctgctaagg gtttcgaagt tgttttgaga gatattaagg atgaattcgt tgatagaggt 120

ttggatttca tcaacaagaa cttgtctaag ttggttaaga agggtaagat tgaagaagct 180

actaaggttg aaatcttgac tagaatttcc ggtaccgttg acttgaacat ggctgctgat 240

tgtgatttgg ttattgaagc tgctgttgaa agaatggata ttaagaagca aattttcgct 300

gacttggaca acatttgtaa gccagaaacc attttggctt ctaacacctc ttctttgtct 360

attaccgaag ttgcttctgc tactaagagg ccagataagg ttattggtat gcacttcttc 420

aacccagctc cagttatgaa gttggttgaa gttattagag gtattgctac ctctcaagaa 480

actttcgatg ctgttaagga aacctctatt gctattggta aggatccagt tgaagttgct 540

gaagctccag gtttcgttgt taacagaatt ttgattccaa tgattaacga agctgttggt 600

attttggctg aaggtattgc ttctgttgaa gacattgata aggctatgaa gttgggtgct 660

aaccacccaa tgggtccatt ggaattgggt gatttcattg gtttggatat ttgtttggct 720

attatggatg ttttgtactc tgaaactggt gattctaagt acagaccaca caccttgttg 780

aagaagtacg ttagagctgg ttggttgggt agaaagtctg gtaagggttt ctacgattac 840

tctaagtaa 849

<210>  15
<211>  786
<212>  DNA
<213>  Artificial Sequence

<220>
<223>  Codon optimized Crt

<400>  15
atggaattga acaacgtcat cttggaaaag gaaggtaagg ttgctgttgt taccattaac      60

agaccaaagg ctttgaacgc tttgaactct gataccttga aggaaatgga ttacgttatt     120

ggtgaaattg aaaacgattc tgaagttttg gctgttattt tgactggtgc tggtgaaaag     180

tctttcgttg ctggtgctga tatttctgaa atgaaggaaa tgaacaccat tgaaggtaga     240

aagttcggta ttttgggtaa caaggttttc agaagattgg aattgttgga aaagccagtt     300

attgctgctg ttaacggttt cgctttgggt ggtggttgtg aaattgctat gtcttgtgat     360

attagaattg cttcttctaa cgctagattc ggtcaaccag aagttggttt gggtattacc     420

ccaggtttcg gtggtaccca aagattgtct agattggttg gtatgggtat ggctaagcaa     480

ttgattttca ctgctcaaaa cattaaggct gatgaagctt tgagaatcgg tttggttaac     540

aaggttgttg aaccatctga attgatgaac accgctaagg aaattgctaa caagattgtt     600

tctaacgctc cagttgctgt taagttgtct aagcaagcta ttaacagagg tatgcaatgt     660

gatattgata ctgctttggc tttcgaatct gaagctttcg gtgaatgttt ctctaccgaa     720

gatcaaaagg atgctatgac cgctttcatt gaaaagagaa agattgaagg tttcaagaac     780

agataa                                                                786

<210>  16
<211>  1194
<212>  DNA
<213>  Artificial Sequence

<220>
<223>  Codon optimized Ter

<400>  16
atgattgtta agccaatggt tagaaacaac atttgtttga acgctcaccc acaaggttgt      60

aagaagggtg ttgaagatca aattgaatac accaagaaga gaattaccgc tgaagttaag     120

gctggtgcta aggctccaaa gaacgttttg gttttgggtt gttctaacgg ttacggtttg     180

gcttctagaa ttaccgctgc tttcggttac ggtgctgcta ctatcggtgt ttccttcgaa     240

aaggctggtt ctgaaaccaa gtacggtacc ccaggttggt acaacaactt ggctttcgac     300

gaagctgcta agagagaagg tttgtactcc gttactattg acggtgacgc tttctccgat     360

gaaatcaagg ctcaagttat cgaagaagct aagaagaagg gtattaagtt cgatttgatt     420

gtttactctt tggcttctcc agttagaacc gatccagaca ccggtattat gcacaagtcc     480

gtcttgaagc cattcggtaa gaccttcacc ggtaagaccg tcgatccatt caccggtgaa     540

ttgaaggaaa tctccgctga accagctaac gatgaagaag ctgctgctac cgttaaggtt     600

atgggtggtg aagactggga aagatggatt aagcaattgt ctaaggaagg tttgttggaa     660

gaaggttgta ttaccttggc ttactcctac atcggtccag aagctactca agctttgtac     720

agaaagggta ccattggtaa ggctaaggaa cacttggaag ctactgctca cagattgaac     780

aaggaaaacc catctattag agctttcgtt tctgttaaca agggtttggt taccagagct     840

tctgctgtta tcccagttat tccattgtac ttggcttcct tgttcaaggt tatgaaggaa     900

aagggtaacc acgaaggttg tatcgaacaa attaccagat tgtacgctga aagattgtac     960

agaaaggacg gtaccatccc agtcgatgaa gaaaacagaa tcagaatcga cgactgggaa    1020

ttggaagaag acgttcaaaa ggctgtttct gctttgatgg aaaaggttac cggtgaaaac    1080

gctgaatctt tgaccgactt ggctggttac agacacgact tcttggcttc taacggtttc    1140

gatgttgaag gtatcaacta cgaagctgaa gttgaaagat tcgacagaat ttaa          1194

<210>  17
<211>  20
<212>  DNA
<213>  Artificial Sequence

<220>
<223>  Primer

<400>  17
catttatccg gtggtcatcg                                                  20

<210>  18
<211>  19
<212>  DNA
<213>  Artificial Sequence

<220>
<223>  Primer

<400>  18
gctagccaag gcagtaagg                                                   19

<210>  19
<211>  20
<212>  DNA
<213>  Artificial Sequence

<220>
<223>  Primer

<400>  19
gctttccaag gcagttacag                                                  20

<210>  20

<211>  80
<212>  DNA
<213>  Artificial Sequence

<220>
<223>  Primer

<400>  20
ctcaaaactt tttgttttaa taatactagt aacaagaaaa ttggattaca tcctactttt        60

acacccctct gcatattttt                                                    80


<210>  21
<211>  80
<212>  DNA
<213>  Artificial Sequence

<220>
<223>   Primer

<400>  21
aaaaatatgc agaggggtgt aaaagtagga tgtaatccaa ttttcttgtt actagtatta        60

ttaaaacaaa aagttttgag                                                    80


<210>  22
<211>  17
<212>  DNA
<213>  Artificial Sequence

<220>
<223>  Primer

<400>  22
gggaaacgcc tggtatc                                                       17


<210>  23
<211>  20
<212>  DNA
<213>  Artificial Sequence

<220>
<223>  Primer

<400>  23
tcttcttgaa gtagtcttcc                                                    20


<210>  24
<211>  19
<212>  DNA
<213>  Artificial Sequence

<220>
<223>  Primer

<400>  24
ggctattgtt gacttgttg                                                     19

<210>  25
<211>  54
<212>  DNA
<213>  Artificial Sequence

<220>
<223>  Primer

<400>  25
cttctgcata tatgtgcgcc gttttagagc tagaaatagc aagttaaaat aagg          54


<210>  26
<211>  46
<212>  DNA
<213>  Artificial Sequence

<220>
<223>  Primer

<400>  26
cggcgcacat atatgcagaa ggatcattta tctttcactg cggaga                   46


<210>  27
<211>  20
<212>  DNA
<213>  Artificial Sequence

<220>
<223>  Primer

<400>  27
ccatggtagc ggttctaaag                                                20


<210>  28
<211>  18
<212>  DNA
<213>  Artificial Sequence

<220>
<223>  Primer

<400>  28
tttgtaaacg gcccgagg                                                  18


<210>  29
<211>  19
<212>  DNA
<213>  Artificial Sequence

<220>
<223>  Primer

<400>  29
gggattagcg ggtcctttg                                                 19


<210>  30
<211>  81
<212>  DNA

<213>  Artificial Sequence

<220>
<223>  Primer

<400>  30
agaatttata catagacgcc gctaaataat tgaatacaaa cgcagttcca atttacaaga      60

atgcttcgtt tgctattaca a                                                81


<210>  31
<211>  81
<212>  DNA
<213>  Artificial Sequence

<220>
<223>  Primer

<400>  31
ttgtaatagc aaacgaagca ttcttgtaaa ttggaactgc gtttgtattc aattatttag      60

cggcgtctat gtataaattc t                                                81


<210>  32
<211>  45
<212>  DNA
<213>  Artificial Sequence

<220>
<223>  Primer

<400>  32
cacagtcccg cagccaaata cgatcattta tctttcactg cggag                      45


<210>  33
<211>  54
<212>  DNA
<213>  Artificial Sequence

<220>
<223>  Primer

<400>  33
gtatttggct gcgggactgt gttttagagc tagaaatagc aagttaaaat aagg            54


<210>  34
<211>  60
<212>  DNA
<213>  Artificial Sequence

<220>
<223>  Primer

<400>  34
ttgatagatc tttggtagaa ttcagagaaa gacattttgt ttgtttatgt gtgtttattc      60


<210>  35
<211>  59

<212>  DNA
<213>  Artificial Sequence

<220>
<223>  Primer

<400>  35
aacaaatcag acaagctatc gaagaataac cgcggattga attgaattga aatcgatag        59


<210>  36
<211>  40
<212>  DNA
<213>  Artificial Sequence

<220>
<223>  Primer

<400>  36
tttaaaacac caagaactta gtttcgaata aacacacata                            40


<210>  37
<211>  41
<212>  DNA
<213>  Artificial Sequence

<220>
<223>  Primer

<400>  37
gagaaaagaa aaaaattgat ctatcgattt caattcaatt c                          41


<210>  38
<211>  60
<212>  DNA
<213>  Artificial Sequence

<220>
<223>  Primer

<400>  38
tttaatttgc ggccggtacc caattcgccc gagctcacag tttattcctg gcatccacta      60


<210>  39
<211>  60
<212>  DNA
<213>  Artificial Sequence

<220>
<223>  Primer

<400>  39
agcgcgcaat taaccctcac taaagggaac aaggatccaa ataatatcct tctcgaaagc      60


<210>  40
<211>  42
<212>  DNA
<213>  Artificial Sequence

<220>
<223>  Primer

<400>  40
cgaagatagt gtccatagaa gtagccaaag caacagcgta ac                          42


<210>  41
<211>  40
<212>  DNA
<213>  Artificial Sequence

<220>
<223>  Primer

<400>  41
agacgttggt ggttacgctg ttgctttggc tacttctatg                            40


<210>  42
<211>  83
<212>  DNA
<213>  Artificial Sequence

<220>
<223>  Primer

<400>  42
ataatactta cggggctagt tttattttct accacaaata aaaataaagc atcttttaaa      60

cagtttattc ctggcatcca cta                                              83


<210>  43
<211>  85
<212>  DNA
<213>  Artificial Sequence

<220>
<223>  Primer

<400>  43
ttgattttca aagtattcca gaaaatttga gtcatgctta cttagtttaa ttaagtactc      60

ttattcttcg atagcttgtc tgatt                                            85


<210>  44
<211>  27
<212>  DNA
<213>  Artificial Sequence

<220>
<223>  Primer

<400>  44
gaataagtcc tggttccagg caaaacc                                          27


<210>  45
<211>  19
<212>  DNA
<213>  Artificial Sequence

<220>
<223>  Primer

<400>  45
gttaggaaca ggcgaggtc                                                    19


<210>  46
<211>  20
<212>  DNA
<213>  Artificial Sequence

<220>
<223>  Primer

<400>  46
cgctgttgac agatggagag                                                   20


<210>  47
<211>  18
<212>  DNA
<213>  Artificial Sequence

<220>
<223>  Primer

<400>  47
cccagactgt ggtgtttg                                                     18


<210>  48
<211>  18
<212>  DNA
<213>  Artificial Sequence

<220>
<223>  Primer

<400>  48
gtgacaaccg aaagtcag                                                     18


<210>  49
<211>  22
<212>  DNA
<213>  Artificial Sequence

<220>
<223>  Primer

<400>  49
tttcttcagg tctaactgat tg                                                22


<210>  50
<211>  80
<212>  DNA
<213>  Artificial Sequence

<220>
<223>  Primer

<400>  50
tcacagaaaa aaagaaaata taataaatta gtattgcgat gtagaggttt cctgttttcc      60

ttcgaaccct ctgttttgcg                                                  80


<210>  51
<211>  80
<212>  DNA
<213>  Artificial Sequence

<220>
<223>  Primer

<400>  51
cgcaaaacag agggttcgaa ggaaaacagg aaacctctac atcgcaatac taatttatta      60

tattttcttt ttttctgtga                                                  80


<210>  52
<211>  49
<212>  DNA
<213>  Artificial Sequence

<220>
<223>  Primer

<400>  52
cgtctcaggt cggtctcagg atttaaattc tgtcgaatct ttcaacttc                  49


<210>  53
<211>  47
<212>  DNA
<213>  Artificial Sequence

<220>
<223>  Primer

<400>  53
cgtctcgtcg gtctcatatg attgttaagc caatggttag aaacaac                    47


<210>  54
<211>  47
<212>  DNA
<213>  Artificial Sequence

<220>
<223>  Primer

<400>  54
cgtctcaggt cggtctcagg atttatctgt tcttgaaacc ttcaatc                    47


<210>  55
<211>  38
<212>  DNA
<213>  Artificial Sequence

<220>

<223>  Primer

<400>  55
cgtctcgtcg gtctcatatg gaattgaaca acgtcatc                              38


<210>  56
<211>  52
<212>  DNA
<213>  Artificial Sequence

<220>
<223>  Primer

<400>  56
cgtctcaggt cggtctcagg atttaaatct tttcaatgac aatagaggaa gc              52


<210>  57
<211>  39
<212>  DNA
<213>  Artificial Sequence

<220>
<223>  Primer

<400>  57
cgtctcgtcg gtctcatatg tctcaaaacg tttacattg                             39


<210>  58
<211>  54
<212>  DNA
<213>  Artificial Sequence

<220>
<223>  Primer

<400>  58
ctgggttgaa gaagtgcata ccaataacct tatctggcct cttagtagca gaag            54


<210>  59
<211>  48
<212>  DNA
<213>  Artificial Sequence

<220>
<223>  Primer

<400>  59
tctgctacta agaggccaga taaggttatt ggtatgcact tcttcaac                   48


<210>  60
<211>  47
<212>  DNA
<213>  Artificial Sequence

<220>
<223>  Primer

<400>  60

cgtctcaggt cggtctcagg atttacttag agtaatcgta gaaaccc 47

<210> 61
<211> 54
<212> DNA
<213> Artificial Sequence

<220>
<223> Primer

<400> 61
ctgattctgt ggataaccgt agtcggtctc atatgaagaa ggtttgtgtt attg 54

<210> 62
<211> 18
<212> DNA
<213> Artificial Sequence

<220>
<223> Primer

<400> 62
ggttgcatca ctccattg 18

<210> 63
<211> 28
<212> DNA
<213> Artificial Sequence

<220>
<223> Primer

<400> 63
tcctttacgc taaaataata gtttattt 28

<210> 64
<211> 20
<212> DNA
<213> Artificial Sequence

<220>
<223> Primer

<400> 64
aaggcattaa aagaggagcg 20

<210> 65
<211> 19
<212> DNA
<213> Artificial Sequence

<220>
<223> Primer

<400> 65
gttatcccct gattctgtg 19

<210>  66
<211>  50
<212>  DNA
<213>  Artificial Sequence

<220>
<223>  Primer

<400>  66
tctacttttt acaacaaata taaaacaatg aagaaggttt gtgttattgg                50


<210>  67
<211>  25
<212>  DNA
<213>  Artificial Sequence

<220>
<223>  Primer

<400>  67
catacacatg tatctcagat atctc                                           25


<210>  68
<211>  19
<212>  DNA
<213>  Artificial Sequence

<220>
<223>  Primer

<400>  68
tttcgttgtc gaacttacc                                                  19


<210>  69
<211>  21
<212>  DNA
<213>  Artificial Sequence

<220>
<223>  Primer

<400>  69
tattgctatt ggtaaggatc c                                               21


<210>  70
<211>  15
<212>  DNA
<213>  Artificial Sequence

<220>
<223>  Primer

<400>  70
caccgaaacc tgggg                                                      15


<210>  71
<211>  19
<212>  DNA

<213>  Artificial Sequence

<220>
<223>  Primer

<400>  71
ccgtcttgaa gccattcgg                                                    19


<210>  72
<211>  21
<212>  DNA
<213>  Artificial Sequence

<220>
<223>  Primer

<400>  72
ttacagacac gacttcttgg c                                                 21


<210>  73
<211>  20
<212>  DNA
<213>  Artificial Sequence

<220>
<223>  Primer

<400>  73
caactacaga gaacaggggc                                                   20